(19) Europäisches Patentamt
European Patent Office
Office européen des brevets

(11) Publication number: 0 335 723 B1

# (12) EUROPEAN PATENT SPECIFICATION

(45) Date of publication of patent specification: 04.12.91 Bulletin 91/49

(51) Int. Cl.$^5$: C07D 261/12, C07D 261/20, A61K 31/42

(21) Application number: 89303164.1

(22) Date of filing: 30.03.89

## (54) Isoxazole derivatives for use as cerebro-active drugs and central muscle relaxants.

The file contains technical information submitted after the application was filed and not included in this specification

(30) Priority: 30.03.88 JP 77653/88
19.04.88 JP 94654/88
24.05.88 JP 126761/88
30.05.88 JP 132400/88
31.05.88 JP 133433/88
26.07.88 JP 186131/88
14.11.88 JP 287314/88

(43) Date of publication of application: 04.10.89 Bulletin 89/40

(45) Publication of the grant of the patent: 04.12.91 Bulletin 91/49

(84) Designated Contracting States: AT BE CH DE ES FR GB GR IT LI LU NL SE

(56) References cited:
EP-A- 0 273 744
CHEMICAL ABSTRACTS, vol. 104, no. 7, 17th February 1986, page 516, abstract no. 50810a, Columbus, Ohio, US; T. SLAWIK et al.: "New acyl and alkyl derivatives of 1,2-benzoisoxazole"
PATENT ABSTRACTS OF JAPAN, vol. 5, no. 89 (C-58)[761], 10th June 1981; & JP-A-56 34 674
CHEMICAL ABSTRACTS, vol. 88, no. 9, 27th February 1978, page 388, abstract no. 62379f, Columbus, Ohio, US; I. ADACHI et al.: "1,2-Benzisoxazole derivatives"; & JP-A-77 31 070
CHEMICAL ABSTRACTS, vol. 101, no. 1, 2nd July 1984, page 605, abstract no. 7077t, Columbus, Ohio, US; M.R. MINGIARDI et al.: "1,2-Benzisothiazole derivatives potentially active as beta-blockers"

(73) Proprietor: Sankyo Company Limited
5-1 Nihonbashi Honcho 3-chome Chuo-ku
Tokyo (JP)

(72) Inventor: Nagano, Mitsuo, c/o Sankyo Company Ltd.
No. 2-58, 1-chome, Hiromachi
Shinagawa-ku, Tokyo 140 (JP)
Inventor: Sakai, Junichi, c/o Sankyo Company Ltd.
No. 2-58, 1-chome, Hiromachi
Shinagawa-ku, Tokyo 140 (JP)
Inventor: Iwata, Nobuyoshi, c/o Sankyo Company Ltd.
No. 2-58, 1-chome, Hiromachi
Shinagawa-ku, Tokyo 140 (JP)
Inventor: Kobayashi, Kazuo, c/o Sankyo Company Ltd.
No. 2-58, 1-chome, Hiromachi
Shinagawa-ku, Tokyo 140 (JP)
Inventor: Kozuka, Masao, c/o Sankyo Company Ltd.
No. 2-58, 1-chome, Hiromachi
Shinagawa-ku, Tokyo 140 (JP)
Inventor: Kato, Katsunori, c/o Sankyo Company Ltd.
No. 2-58, 1-chome, Hiromachi
Shinagawa-ku, Tokyo 140 (JP)
Inventor: Yoshimi, Kenji, c/o Sankyo Company Ltd.
No. 2-58, 1-chome,Hiromachi
Shinagawa-ku, Tokyo 140 (JP)
Inventor: Kubo, Yoshiko, c/o Sankyo Company Ltd.
No. 2-58, 1-chome, Hiromachi
Shinagawa-ku, Tokyo 140 (JP)

(74) Representative: Gibson, Christian John Robert et al
MARKS & CLERK 57/60 Lincoln's Inn Fields
London WC2A 3LS (GB)

EP 0 335 723 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a series of new isoxazole derivatives, which are regarded as derivatives of isoxazolones, and which may be used for the treatment of cerebral circulatory problems and for use as centrally acting muscle relaxants. The invention also provides methods of using these compounds and processes for preparing them.

Cerebrovascular disorders, including stroke infarction, are among the main causes of death in the world today. Moreover, although the patient may survive in such cases, cognition impairment, which may be one of the sequelae of the disease, is a substantial social problem at present. For this reason, the development of therapeutic agents for the treatment of such disorders (commonly referred to as "cerebro-active drugs") is required.

Moreover, rigidity and/or spasticity are often observed as the sequelae of cerebrovascular disorders, such as cerebral stroke infarction, or as cerebrotraumatic sequelae, and make rehabilitation difficult. Centrally-acting muscle relaxants are available which can relieve such spasrigido hemiplegia, and act on the central nervous system. Examples of existing centrally-acting muscle relaxants include eperisone hydrochloride and afloqualone.

We have now discoverd a series of new compounds which can be used as cerebro-active drugs and as centrally-acting muscle relaxants. Such compounds are needed which can treat cerebral circulatory problems and alleviate myotony and spasrigido hemiplegia, preferably without giving rise to accompanying drowsiness.

Certain isoxazolone derivatives are known which have the required effect. For example, Japanese Patent Application Kokai (i.e. as laid open to public inspection) Sho. No. 56-34674, discloses such compounds having anti-inflammatory, analgesic and anti-pyretic activities, European Patent Publication No. 273744 discloses that the same compounds have a centrally acting muscle relaxant activity, and a European Patent Application filed on 23 March 1989 in the name of the present Applicants and entitled "USE OF ISOXAZOLINONES AS CEREBRO-ACTIVE DRUGS" discloses that the same compounds are useful for the treatment of cerebral circulatory problems.

Additionally, certain compounds closely related to those of the present invention are disclosed in European Patent Application No. -A-0334674.

Acta Pol. Pharma 41(6) (1984) 625-31, summarised in English in CA 104 (1986) 50810a, describes certain acyl and alkyl derivatives of 1,2-benzoisoxazole, certain of these having structures similar to the compounds of the present invention. Activity of the compounds is not discussed.

We have now discovered a series of isoxazole derivatives, which are capable of acting as cerebro-active drugs and as centrally acting muscle relaxants.

The present invention accordingly provides compounds of formula (I) :

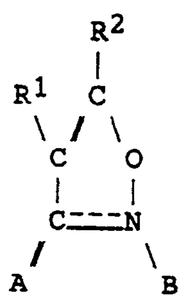

(I)

in which : either

(a) the dotted line (- - -) represents a single bond ;

A = represents a oxygen atom ; and

B represents a group of formula (II) :

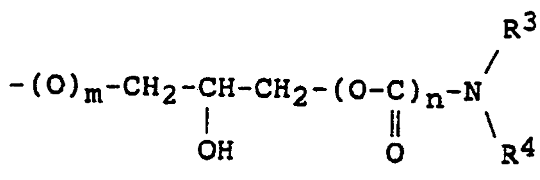

(II)

in which m is 0 and n is 0 or 1 ;
(b) the dotted line (- - -) represents a double bond ;

A = represents said group of formula (II) in which m is 1 and n is 0 or 1 ; and
B is absent ; and
$R^1$ represents a hydrogen atom, a halogen atom, a $C_1$-$C_4$ alkyl group, a $C_2$-$C_4$ alkenyl group, a $C_2$-$C_4$ alkynyl group, a benzyl group, a benzyl group having at least one of substituents (a), defined below, a phenyl group or a substituted phenyl group having at least one of substituents (a), defined below ;
$R^2$ represents a hydrogen atom, a halogen atom, a $C_1$-$C_4$ alkyl group, a phenyl group, a substituted phenyl group having at least one of substituents (a), defined below, or a heterocyclic group having 5 or 6 ring atoms, of which from 1 to 3 are nitrogen and/or oxygen and/or sulphur hetero-atoms, said heterocyclic group being unsubstituted or having at least one of substituents (a), defined below ; or
$R^1$ and $R^2$, together with the carbon atoms to which they are attached, represent a hydrocarbon ring fused to the isoxazole ring and having, in total, from 5 to 7 ring carbon atoms, said hydrocarbon ring being unsubstituted or having at least one of substituents (b), defined below ;
$R^3$ and $R^4$ are the same or different and each represents a hydrogen atom, a $C_1$-$C_4$ alkyl group, a benzyl group, a substituted benzyl group having at least one of substituents (a), defined below, a phenyl group or a substituted phenyl group having at least one of substituents (a), defined below ; or
$R^3$, $R^4$ and the nitrogen atom to which they are attached together represent an alicyclic amino group having a total of 5 or 6 ring atoms, of which one is said nitrogen atom and 0 or 1 is an additional hetero-atom nitrogen and/or oxygen and/or sulphur hetero-atom, said alicyclic amino group being unsubstituted or, where there is an additional nitrogen hetero-atom, having at least one substituent on that additional nitrogen hetero-atom selected from substituents (b), defined below ;

substituents (a) :

$C_1$-$C_3$ alkyl groups, $C_1$-$C_3$ alkoxy groups, hydroxy groups, halogen atoms, nitro groups, amino groups and $C_2$-$C_4$ aliphatic carboxylic acyl groups ;

substituents (b) :

$C_1$-$C_3$ alkyl groups, $C_1$-$C_3$ alkoxy groups, hydroxy groups, halogen atoms, nitro groups, amino groups, $C_2$-$C_4$ aliphatic carboxylic acylamino groups, benzyl groups, substituted benzyl groups having at least one of substituents (a), defined above, phenyl groups and substituted phenyl groups having at least one of substituents (a), defined above ;

provided that

(i) where B represents said group of formula (II) and n is 0, then $R^1$ does not represent a hydrogen or halogen atom ;
(ii) where A represents said group of formula (II) and n is 0, $R^1$ and $R^2$ together represent a benzene ring fused to the isoxazole ring and $R^4$ represents an alkyl group, then $R^3$ does not represent a hydrogen atom; and
(iii) when (- - -) represents a single bond, $R^1$ and $R^2$, together with the carbon atoms to which they are attached form a $C_{6-7}$ hydrocarbon ring, then n = 1 ;

and pharmaceutically acceptable salts thereof ; but

excluding :

the compound 5-chloro-2-(3-piperidino-2-hydroxy) propylbenzisoxazolin-3-one.

The present invention also provides the use for the manufacture of a medicament for treating cerebrovascular disorders (e.g. in a mammal, which may be human, suffering from or prone to cerebrovascular disorders) of at least one cerebro-active drug, wherein the cerebro-active drug is a compound of formula (I) or a pharmaceutically acceptable salt thereof.

The present invention also provides the use for the manufacture of a medicament for effecting centrally-acting muscle relaxant activity (e.g. in a mammal, which may be human), of a compound of formula (I) or a pharmaceutically acceptable salt thereof.

The invention also provides a pharmaceutical composition for the treatment of cerebrovascular disorders or as a centrally-acting muscle relaxant, which composition comprises an effective amount of an active compound, wherein the active compound is a compound of formula (I) or a pharmaceutically acceptable salt thereof.

The invention also provides processes for preparing the compounds of the present invention, which processes are described in more detail hereafter.

Compounds similar to those of formula (I), but in which B represents said group of formula (II) in which n is 0, and $R^1$ represents a hydrogen or halogen atom are disclosed in the aforementioned Japanese Patent Application Kokai Sho. No. 56-34674, although their cerebro-active and centrally-acting muscle relaxant properties are not disclosed therein. The present invention, therefore, also provides these compounds for use as cerebro-active drugs and centrally-acting muscle relaxants.

Compounds similar to those of formula (I), but in which A represents said group of formula (II) in which n is 0, $R^1$ and $R^2$ together represent a benzene ring fused to the isoxazole ring, $R^4$ represents an alkyl group, and $R^3$ represents a hydrogen atom are disclosed in the aforementioned Japanese Patent Application Kokai Sho. No. 52-31070, although their cerebro-active and centrally-acting muscle relaxant properties are not disclosed therein. The present invention, therefore, also provides these compounds for use as cerebro-active drugs and centrally-acting muscle relaxants.

In the compounds of the present invention, where $R^1$ represents a $C_1$-$C_4$ alkyl group, this may be a straight or branched chain group and examples include the methyl, ethyl, propyl, isopropyl, butyl, isobutyl, sec-butyl and t-butyl groups, of which the methyl, ethyl, isopropyl and isobutyl groups are preferred.

Where $R^1$ represents a halogen atom, this may be a fluorine, chlorine, bromine or iodine atom, of which the fluorine, chlorine and bromine atoms are preferred, the chlorine atom being most preferred.

Where $R^1$ represents a $C_2$-$C_4$ alkenyl group, this may be a straight or branched chain group and examples include the vinyl, allyl, 1-propenyl, 1-butenyl, 2-butenyl, 3-butenyl, 3-methylallyl and 2-methylallyl groups, of which the vinyl, allyl, 2-butenyl, 3-methylallyl and 2-methylallyl groups are preferred.

Where $R^1$ represents a $C_2$-$C_4$ alkynyl group, this may be a straight or branched chain group and examples include the ethynyl, 1-propynyl, 2-propynyl, 1-butynyl, 2-butynyl and 3-butynyl groups, of which the ethynyl and 2-propynyl groups are most preferred.

Where $R^1$ represents a benzyl group, this may be unsubstituted or it may have one or more substituents. If it is substituted, it may have from 1 to 5 substituents, more preferably from 1 to 3 substituents, and most preferably 1 or 2 substituents, which may be selected from substituents (a), defined above and exemplified below. Examples of such optionally substituted benzyl groups include the benzyl group itself, and the 2-chlorobenzyl, 3-chlorobenzyl, 4-chlorobenzyl, 2-fluorobenzyl, 3-fluorobenzyl, 4-fluorobenzyl, 2-bromobenzyl, 3-bromobenzyl, 4-bromobenzyl, 2-nitrobenzyl, 3-nitrobenzyl, 4-nitrobenzyl, 2-methoxybenzyl, 3-methoxybenzyl, 4-methoxybenzyl, 2-propoxybenzyl, 3-propoxybenzyl, 4-propoxybenzyl, 2-hydroxybenzyl, 3-hydroxybenzyl, 4-hydroxybenzyl, 2-aminobenzyl, 3-aminobenzyl, 4-aminobenzyl, 2-propylbenzyl, 3-propylbenzyl, 4-propylbenzyl, 2-methylbenzyl, 3-methylbenzyl, 4-methylbenzyl, 2-acetylbenzyl, 3-acetylbenzyl, 4-acetylbenzyl, 2-butyrylbenzyl, 3-butyrylbenzyl, 4-butyrylbenzyl, 2,4-dichlorobenzyl, 2,4-difluorobenzyl, 2-chloro-4-fluorobenzyl, 2-chloro-4-butylbenzyl, 3,5-dichlorobenzyl, 3,5-difluorobenzyl, 3-chloro-5-fluorobenzyl and 3-chloro-5-butylbenzyl groups, but the benzyl group itself is preferred.

Where $R^1$ represents a phenyl group, this may be unsubstituted or it may have one or more substituents. If it is substituted, it may have from 1 to 5 substituents, more preferably from 1 to 3 substituents, and most preferably 1 or 2 substituents, which may be selected from substituents (a), defined above and exemplified below. Examples of such optionally substituted phenyl groups include the phenyl group itself, and the 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 2-nitrophenyl, 3-nitrophenyl, 4-nitrophenyl, 2-methoxyphenyl, 3-methoxyphenyl, 4-methoxyphenyl, 2-propoxyphenyl, 3-propoxyphenyl, 4-propoxyphenyl, 2-hydroxyphenyl, 3-hydroxyphenyl, 4-hydroxyphenyl, 2-aminophenyl, 3-aminophenyl, 4-aminophenyl, 2-propylphenyl, 3-propylphenyl, 4-propylphenyl, 2-methylphenyl, 3-methylphenyl, 4-methylphenyl, 2-acetylphenyl, 3-acetylphenyl, 4-acetylphenyl, 2-butyrylphenyl, 3-butyrylphenyl, 4-butyrylphenyl, 2,4-dichlorophenyl, 2,4-difluorophenyl, 2-chloro-4-fluorophenyl, 2-chloro-4-butylphenyl, 3,5-dichlorophenyl, 3,5-difluorophenyl, 3-chloro-5-fluorophenyl and 3-chloro-5-butylphenyl groups, but the phenyl group itself is preferred.

In general, preferred groups and atoms which may be represented by $R^1$ include the hydrogen and chlorine atoms, and the methyl, ethyl, isopropyl, isobutyl, allyl, 3-methylallyl, 2-propynyl, phenyl and benzyl groups.

Where $R^2$ represents a halogen atom, this may be as exemplified above in relation to the halogen atoms which may be represented by $R^1$, and preferred halogen atoms are also as exemplified in relation to $R^1$.

Where $R^2$ represents an alkyl group, this may be as exemplified above in relation to the alkyl groups which may be represented by $R^1$. Preferred alkyl groups are the methyl, ethyl, propyl, isopropyl, butyl, isobutyl, sec-butyl and t-butyl groups, of which the methyl group is most preferred.

Where $R^2$ represents a phenyl group, this may be as exemplified above in relation to the optionally substituted phenyl groups which may be represented by $R^1$. Preferred optionally substituted phenyl groups for $R^2$

are the phenyl group itself, and the 4-chlorophenyl, 3-chlorophenyl, 4-nitrophenyl, 4-methoxyphenyl, 3-methoxyphenyl, 2-methoxyphenyl, 4-hydroxyphenyl, 4-fluorophenyl and 2,4-dichlorophenyl groups.

Where $R^2$ represents a heterocyclic group having 5 or 6 ring atoms, from 1 to 3 of these are nitrogen and/or oxygen and/or sulphur hetero-atoms, and said heterocyclic group is unsubstituted or has at least one of substituents (a), defined above and exemplified below. In general, the number of such substituents may be from 1 to 5, although the preferred number is from 1 to 3, more preferably 1 or 2. The heterocyclic group may be aromatic or non-aromatic in character, although it is preferably aromatic. Examples of such heterocyclic groups include the thienyl, furyl, pyranyl, pyrrolyl, imidazolyl, pyrazolyl, thiazolyl, isothiazolyl, oxazolyl, isoxazolyl, pyridyl, pyrazinyl, pyrimidinyl, pyridazinyl, furazanyl, pyrrolidinyl, pyrrolinyl, imidazolidinyl, imidazolinyl, pyrazolidinyl, pyrazolinyl, piperidyl, piperazinyl, morpholinyl and thiomorpholinyl groups. Examples of such substituted heterocyclic groups include the 6-chloro-3-pyridyl, 6-(trifluoromethyl)-3-pyridyl, 5-chloro-2-pyridyl, 5-(trifluoromethyl)-2-furyl and 5-(trifluoromethyl)-2-thienyl and 5-chloro-2-thienyl groups. The preferred groups are the thiazolyl, furyl, thienyl and pyridyl groups, of which the thienyl and pyridyl groups, still more preferably the 2-thienyl and 3-pyridyl groups, are most preferred, and these may be unsubstituted or substituted as defined above.

Where $R^1$ and $R^2$, together with the carbon atoms to which they are attached, represent a hydrocarbon ring fused to the isoxazole ring and having, in total, from 5 to 7 ring carbon atoms, the hydrocarbon ring may be unsubstituted or may have at least one of substituents (b), defined above and exemplified below. Such a hydrocarbon group may be wholly (i.e. aromatically) or partially unsaturated, but is preferably aromatic in character. Examples of 5 to 7 membered ring systems that may be formed by $R^1$ and $R^2$, together with the carbon atoms to which they are attached, include the benzene, cyclohexene, cyclohexadiene, cyclopentene, cyclopentadiene, cycloheptene, cycloheptadiene and cycloheptatriene ring systems, of which the multiply unsaturated groups are preferred, the benzene ring being most preferred. Such ring systems may be unsubstituted or may have at least one of substituents (a), defined above and exemplified below. The maximum number of substituents is limited only by the number of substitutable positions on the hydrocarbon ring, although, in the case of "bulky" substituents, steric constraints may also apply. Preferred substituted and unsubstituted ring systems are (including the isoxazole ring to which they are attached): the 1,2-benzisoxazole, 5-chloro-1,2-benzisoxazole, 5-amino-1,2-benzisoxazole, 5-acetamido-1,2-benzisoxazole, 5-methoxy-1,2-benzisoxazole, 6-chloro-1,2-benzisoxazole, 4-chloro-1,2-benzisoxazole, 7-chloro-1,2-benzisoxazole, 5-methyl-1,2-benzisoxazole, 5,6-dichloro-1,2-benzisoxazole, 4,5,6,7-tetrachloro-1,2-benzisoxazole, 5-propyl-1,2-benzisoxazole, 5-ethoxy-1,2-benzisoxazole, 5-phenyl-1,2-benzisoxazole, 5-benzyl-1,2-benzisoxazole, 5-(4-chlorophenyl)-1,2-benzisoxazole and 5-(4-chlorobenzyl)-1,2-benzisoxazole groups. Of these, we prefer the 1,2-benzisoxazole, 5-chloro-1,2-benzisoxazole, 5-amino-1,2-benzisoxazole, 5-acetamido-1,2-benzisoxazole, 5-methoxy-1,2-benzisoxazole and 6-chloro-1,2-benzisoxazole groups.

Where $R^3$ and/or $R^4$ represents a $C_1$-$C_4$ alkyl group, a benzyl group, a benzyl group having at least one of substituents (a), defined above and exemplified below, a phenyl group or a phenyl group having at least one of substituents (a), defined above and exemplified below, then examples of such groups are as exemplified above in relation to the same groups which may be represented by $R^1$. Preferred groups and atoms which may be represented by $R^3$ and/or $R^4$ include the hydrogen atom, and the methyl, ethyl, propyl, isopropyl, butyl, isobutyl, sec-butyl, t-butyl, phenyl and benzyl groups, the hydrogen atom being most preferred. The two groups $R^3$ and $R^4$ may be the same or different; where they are different, the most preferred groups are those in which one of $R^3$ and $R^4$ represents a hydrogen atom and the other represents one of the other groups defined above, more preferably an alkyl group.

Hence, preferred groups represented by $-NR^3R^4$ include the amino, methylamino, ethylamino, propylamino, isopropylamino, butylamino, isobutylamino, sec-butylamino, t-butylamino, dimethylamino, diethylamino, phenylamino and benzylamino groups.

Alternatively, $R^3$, $R^4$ and the nitrogen atom to which they are attached may together represent an alicyclic amino group having a total of 5 or 6 ring atoms, of which one is said nitrogen atom and 0 or 1 is an additional nitrogen and/or oxygen and/or sulphur hetero-atom, said alicyclic amino group being unsubstituted or, where there is an additional nitrogen hetero-atom, having at least one substituent on that additional nitrogen hetero-atom selected from the group consisting of substituents (b), defined above and exemplified below. Examples of such groups include the 1-pyrrolyl, 1-imidazolyl, 1-pyrazolyl, 3-thiazolyl, 2-isothiazolyl, 3-oxazolyl, 2-isoxazolyl, 1-pyridyl, 1-pyrazinyl, 1-pyrimidinyl, 1-pyridazinyl, 2-furazanyl, 1-pyrrolidinyl, 1-pyrrolinyl, 1-imidazolidinyl, 1-imidazolinyl, 1-pyrazolidinyl, 1-pyrazolinyl, piperidino, 1-piperazinyl, morpholino and thiomorpholino groups, which may be substituted or unsubstituted, and, if substituted, the substituents are selected from the group consisting of substituents (b), defined above and exemplified below. Of these, the morpholino, 1-piperazinyl, 4-methyl-1-piperazinyl, 1-pyrrolidinyl and piperidino groups are most preferred.

Examples of groups and atoms included in substituents (a) include :

$C_1$-$C_3$ alkyl groups, such as the methyl, ethyl, propyl and isopropyl groups ;
$C_1$-$C_3$ alkoxy groups, such as the methoxy, ethoxy, propoxy and isopropoxy groups ;
the hydroxy, nitro and amino groups ;
halogen atoms, such as the fluorine, chlorine, bromine and iodine atoms ; and
$C_2$-$C_4$ aliphatic carboxylic acylamino groups, especially the $C_2$-$C_4$ alkanoylamino groups, such as the acetamido, propionamido, butyramido, isobutyramido, acryloylamino, propioloylamino, methacryloylamino, crotonoylamino and isocrotonoylamino groups.

Examples of groups and atoms included in substituents (b) include those exemplified above in relation to substituents (a) and optionally substituted phenyl and benzyl groups, such as those exemplified above in relation to the same groups which may be represented by $R^1$.

Preferred classes of compounds of the present invention are the following compounds :

(a) compounds of formula (Ia) :

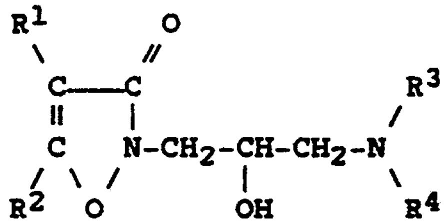

(Ia)

in which :
$R^1$ represents a $C_1$-$C_4$ alkyl group, a $C_2$-$C_4$ alkenyl group, a $C_2$-$C_4$ alkynyl group, a benzyl group, a substituted benzyl group, a phenyl group or a substituted phenyl group, the substituents being selected from substituents (a), defined above ;
$R^2$ represents hydrogen atom, a $C_1$-$C_4$ alkyl group, a phenyl group, a substituted phenyl group, or a heterocyclic group as defined above ;
$R^3$ and $R^4$ are the same or different and each represents a hydrogen atom, a $C_1$-$C_4$ alkyl group, a benzyl group, a substituted benzyl group, a phenyl group or a substituted phenyl group, or $R^3$ and $R^4$ may be taken together with the nitrogen atom to which they are attached to form an alicyclic amino group, as defined above ;

(b) compounds of formula (Ib) :

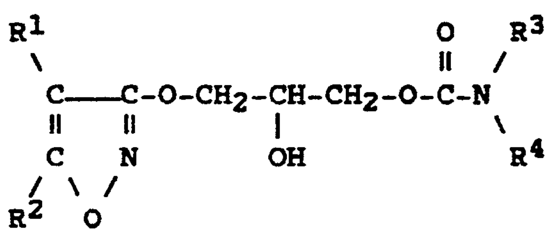

(Ib)

in which :
$R^1$ represents a hydrogen atom, a halogen atom, a $C_1$-$C_4$ alkyl group, a $C_2$-$C_4$ alkenyl group, a $C_2$-$C_4$ alkynyl group, a benzyl group, a substituted benzyl group having at least one of substituents (a), defined above, a phenyl group or a substituted phenyl group having at least one of substituents (a), defined above ;
$R^2$ represents a hydrogen atom, a $C_1$-$C_4$ alkyl group, a phenyl group, a substituted phenyl group having at least one of substituents (a), defined above, or a heterocyclic group as defined above ; or
$R^1$ and $R^2$, together with the carbon atoms to which they are attached, represent a hydrocarbon ring fused to the isoxazole ring and as defined above ;
$R^3$ and $R^4$ are the same or different and each represents a hydrogen atom, a $C_1$-$C_4$ alkyl group, a benzyl group, a substituted benzyl group having at least one of substituents (a), defined above, a phenyl group or a substituted phenyl group having at least one of substituents (a), defined above ; or
$R^3$, $R^4$ and the nitrogen atom to which they are attached together represent an alicyclic amino group as defined above ;

(c) compounds of formula (Ic) :

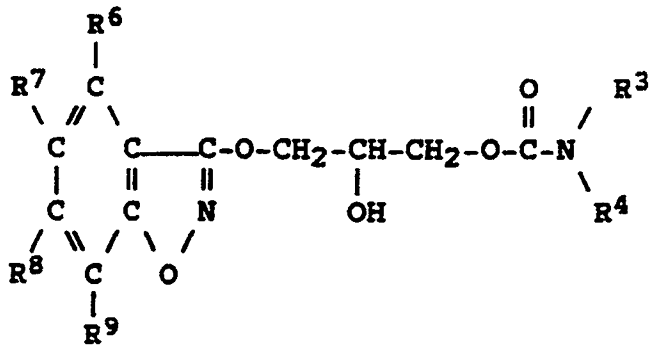

(Ic)

in which :

$R^3$ and $R^4$ are as defined above in class (b) ; and one of $R^6$, $R^7$, $R^8$ and $R^9$ represents a hydrogen atom, a halogen atom, a $C_1$-$C_4$ alkyl group or a $C_1$-$C_4$ alkoxy group, and the others all represent hydrogen atoms;

(d) compounds of formula (Id) :

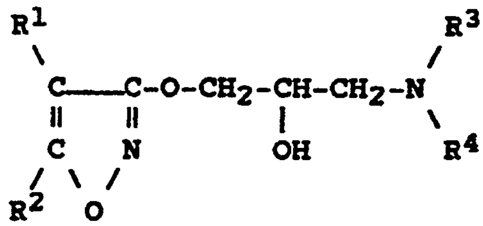

(Id)

in which :

$R^1$, $R^2$, $R^3$ and $R^4$ are as defined above in class (b), provided that, where $R^1$ and $R^2$ together represent a benzene ring fused to the isoxazole ring and $R^4$ represents an alkyl group, then $R^3$ does not represent a hydrogen atom ;

(e) compounds of formula (Ie) :

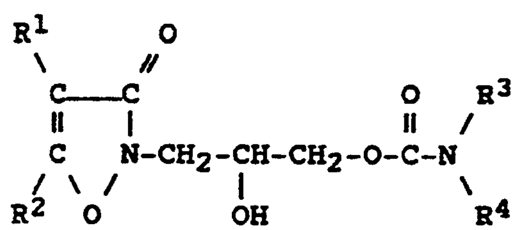

(Ie)

in which :

$R^1$, $R^2$, $R^3$ and $R^4$ are as defined above in class (b) ;

(f) compounds of formula (If) :

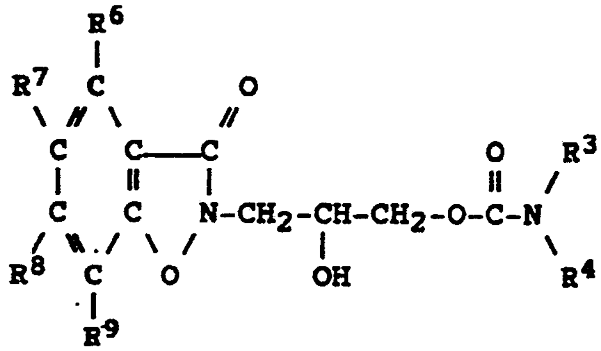

(If)

in which :

$R^3$ and $R^4$ are as defined above in class (b) ; and $R^6$, $R^7$, $R^8$ and $R^9$ are as defined above in class (c) ; and pharmaceutically acceptable salts thereof.

Especially preferred classes of compounds of the present invention are those compounds of formulae (I), (Ia), (Ib), (Ic), (Id), (Ie) and (If), shown above, in which :

(A) $R^1$ represents a hydrogen atom, a halogen atom or a $C_1$-$C_4$ alkyl group ;

(B) $R^2$ represents a phenyl group or a phenyl group having at least one of substituents (c), defined below;

<u>substituents (c) :</u>

$C_1$-$C_3$ alkoxy groups, a hydroxy groups and halogen atoms ;

(C) $R^1$ and $R^2$, together with the carbon atoms to which they are attached, represent a 6-membered hydrocarbon ring fused to the isoxazole ring and being unsubstituted or having at least one halogen atoms ;

(D) $R^3$ and $R^4$ are the same or different and each represents a hydrogen atom, a $C_1$-$C_4$ alkyl group, a benzyl group or a phenyl group ;

(E) $R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

<u>substituents (d) :</u>

$C_1$-$C_3$ alkyl groups, benzyl groups and phenyl groups ; and the most preferred compounds of the present invention are those compounds of formulae (I), (Ia), (Ib), (Ic), (Id) and (Ie), shown above, in which :

(F) $R^1$ represents a hydrogen atom, a chlorine atom or a $C_1$ or $C_2$ alkyl group ;

(G) $R^2$ represents a phenyl group or a phenyl group having at least one of substituents (c'), defined below;

<u>substituents (c') :</u>

methoxy groups and halogen atoms ;

(H) $R^1$ and $R^2$, together with the carbon atoms to which they are attached, represent a benzene ring fused to the isoxazole ring and being unsubstituted or having one halogen atoms ;

(I) $R^3$ and $R^4$ are the same or different and each represents a hydrogen atom, a methyl group or an ethyl group ;

(J) $R^3$, $R^4$ and the nitrogen atom to which they are attached together represent a morpholino group, a 1-piperazinyl group, a 4-methyl-1-piperazinyl group, a 1-pyrrolidinyl group or a piperidino group ;

and pharmaceutically acceptable salts thereof.

The compounds of the present invention necessarily contain several basic nitrogen atoms and can, therefore, form acid addition salts and such salts also form part of the present invention. There is no limitation upon the nature of such salts, provided that, where they are to be used for therapeutic purposes, they are pharmaceutically acceptable, which, as is well-known in the art, means that they do not have reduced activity (or unacceptably reduced activity) or increased toxicity (or unacceptably increased toxicity) compared with the free compound of formula (I). Where, however, they are to be used for non-therapeutic purposes, e.g. as intermediates in the preparation of other compounds, even this limitation does not apply. Examples of suitable salts

include : salts with an inorganic acid, such as a hydrogen halide (e.g. hydrofluoric acid, hydrochloric acid, hydrobromic acid or hydroiodic acid), or nitric acid, perchloric acid, sulphuric acid or phosphoric acid ; and salts with an organic acid, such as a lower alkylsulphonic acid (e.g. methanesulphonic acid, trifluoromethanesulphonic acid or ethanesulphonic acid), an arylsulphonic acid (e.g. benzenesulphonic acid or p-toluenesulphonic acid), or a carboxylic acid (e.g. fumaric acid, lactic acid, citric acid, tartaric acid, succinic acid, oxalic acid or maleic acid).

The compounds of the present invention can exist in the form of various optical isomers and diastereomers because of the existence of asymmetric carbon atoms in the molecule. The optical isomers can be resolved using conventional techniques of optical resolution to give optically active compounds. The present invention covers both the individual isomers and mixtures (e.g. racemic mixtures) thereof, whether as obtained by their synthesis reaction or by mixing. If individual isomers are required, these may be prepared from mixtures, by conventional means, or they may be prepared by stereospecific synthesis techniques, as are well known in the art.

Examples of specific compounds of the invention are given in the following formulae (I-1) to (I-4), in which the substituents are as defined in the corresponding one of Tables 1 to 4, respectively [i.e. Table 1 relates to formula (I-1), Table 2 relates to formula (I-2) and so on]. In the Tables, the following abbreviations are used :

| | |
|---|---|
| Ac | acetyl |
| All | allyl |
| Bu | butyl |
| iBu | isobutyl |
| sBu | sec-butyl |
| tBu | t-butyl |
| Bz | benzyl |
| Et | ethyl |
| Me | methyl |
| Mor | morpholino |
| Ph | phenyl |
| Pip | piperidyl |
| Piz | piperazinyl |
| Pr | propyl |
| iPr | isopropyl |
| Pyr | pyridyl |
| Pyrd | pyrrolidinyl |
| Thi | thienyl |

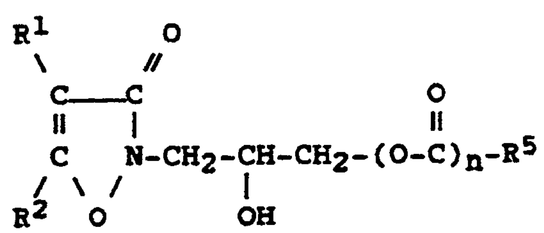

(I-1)

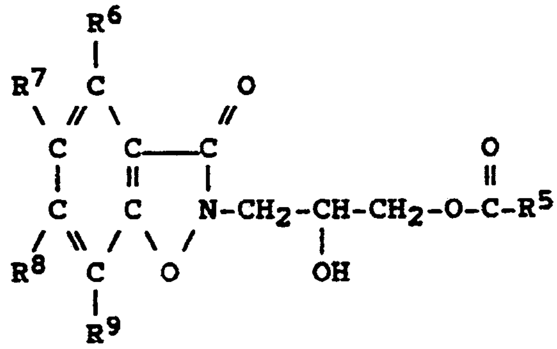

(I-2)

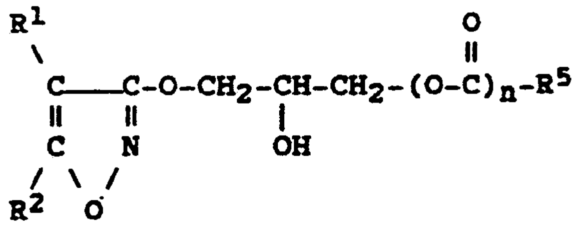

(I-3)

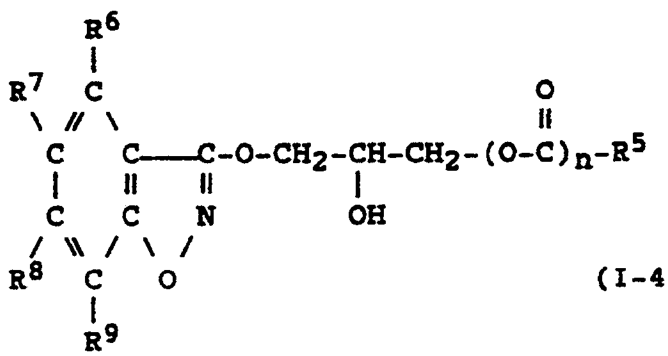

(I-4)

Table 1

| Cpd No. | $R^1$ | $R^2$ | $R^5$ | n |
|---|---|---|---|---|
| 1-1 | Me | Ph | $-NH_2$ | 0 |
| 1-2 | Me | Ph | -NHMe | 0 |
| 1-3 | Me | Ph | -NHEt | 0 |
| 1-4 | Me | Ph | -NHPr | 0 |
| 1-5 | Me | Ph | -NHiPr | 0 |
| 1-6 | Me | Ph | -NHBu | 0 |
| 1-7 | Me | Ph | -NHiBu | 0 |
| 1-8 | Me | Ph | -NHsBu | 0 |
| 1-9 | Me | Ph | -NHtBu | 0 |
| 1-10 | Me | Ph | -NHPh | 0 |
| 1-11 | Me | Ph | -NHBz | 0 |
| 1-12 | Me | Ph | 1-Pyrd | 0 |
| 1-13 | Me | Ph | Mor | 0 |
| 1-14 | Me | Ph | 1-Piz | 0 |
| 1-15 | Me | Ph | 4-Me-1-Piz | 0 |
| 1-16 | Et | Ph | Mor | 0 |
| 1-17 | iPr | Ph | Mor | 0 |
| 1-18 | iBu | Ph | Mor | 0 |
| 1-19 | All | Ph | Mor | 0 |
| 1-20 | $MeCH=CHCH_2-$ | Ph | Mor | 0 |
| 1-21 | $HC{\equiv}C-CH_2-$ | Ph | Mor | 0 |
| 1-22 | Ph | Ph | Mor | 0 |
| 1-23 | Bz | Ph | Mor | 0 |
| 1-24 | Me | Me | Mor | 0 |
| 1-25 | Me | Et | Mor | 0 |
| 1-26 | Me | Pr | Mor | 0 |
| 1-27 | Me | iPr | Mor | 0 |
| 1-28 | Me | Bu | Mor | 0 |

Table 1 (cont)

| Cpd No. | $R^1$ | $R^2$ | $R^5$ | n |
|---|---|---|---|---|
| 1-29 | Me | iBu | Mor | 0 |
| 1-30 | Me | sBu | Mor | 0 |
| 1-31 | Me | tBu | Mor | 0 |
| 1-32 | Me | H | Mor | 0 |
| 1-33 | Me | 4-CℓPh | Mor | 0 |
| 1-34 | Me | 3-CℓPh | Mor | 0 |
| 1-35 | Me | 2-CℓPh | Mor | 0 |
| 1-36 | Me | 4-$NO_2$Ph | Mor | 0 |
| 1-37 | Me | 4-MeOPh | Mor | 0 |
| 1-38 | Me | 3-MeOPh | Mor | 0 |
| 1-39 | Me | 2-MeOPh | Mor | 0 |
| 1-40 | Me | 2-HOPh | Mor | 0 |
| 1-41 | Me | 2,4-diCℓPh | Mor | 0 |
| 1-42 | Me | 4-FPh | Mor | 0 |
| 1-43 | Me | 2-Thi | Mor | 0 |
| 1-44 | Me | 3-Pyr | Mor | 0 |
| 1-45 | H | Ph | $-NH_2$ | 1 |
| 1-46 | H | Ph | -NHMe | 1 |
| 1-47 | H | Ph | -NHEt | 1 |
| 1-48 | H | Ph | -NHPr | 1 |
| 1-49 | H | Ph | -NHiPr | 1 |
| 1-50 | H | Ph | -NHBu | 1 |
| 1-51 | H | Ph | -NHiBu | 1 |
| 1-52 | H | Ph | -NHsBu | 1 |
| 1-53 | H | Ph | -NHtBu | 1 |
| 1-54 | H | Ph | -NHPh | 1 |
| 1-55 | H | Ph | -NHBz | 1 |
| 1-56 | H | Ph | $-NMe_2$ | 1 |

## Table 1 (cont)

| Cpd No. | $R^1$ | $R^2$ | $R^5$ | n |
|---|---|---|---|---|
| 1-57 | H | Ph | 1-Pyrd | 1 |
| 1-58 | H | Ph | Mor | 1 |
| 1-59 | H | Ph | 1-Piz | 1 |
| 1-60 | H | Ph | 4-Me-1-Piz | 1 |
| 1-61 | H | Ph | 1-Pip | 1 |
| 1-62 | Cl | Ph | $-NH_2$ | 1 |
| 1-63 | Cl | Ph | -NHMe | 1 |
| 1-64 | Cl | Ph | -NHEt | 1 |
| 1-65 | Cl | Ph | -NHPr | 1 |
| 1-66 | Cl | Ph | -NHiPr | 1 |
| 1-67 | Cl | Ph | -NHBu | 1 |
| 1-68 | Cl | Ph | -NHiBu | 1 |
| 1-69 | Cl | Ph | -NHsBu | 1 |
| 1-70 | Cl | Ph | -NHtBu | 1 |
| 1-71 | Cl | Ph | -NHPh | 1 |
| 1-72 | Cl | Ph | -NHBz | 1 |
| 1-73 | Cl | Ph | $-NMe_2$ | 1 |
| 1-74 | Cl | Ph | 1-Pyrd | 1 |
| 1-75 | Cl | Ph | Mor | 1 |
| 1-76 | Cl | Ph | 1-Piz | 1 |
| 1-77 | Cl | Ph | 4-Me-1-Piz | 1 |
| 1-78 | Cl | Ph | 1-Pip | 1 |
| 1-79 | Me | Ph | $-NH_2$ | 1 |
| 1-80 | Et | Ph | $-NH_2$ | 1 |
| 1-81 | iPr | Ph | $-NH_2$ | 1 |
| 1-82 | iBu | Ph | $-NH_2$ | 1 |
| 1-83 | All | Ph | $-NH_2$ | 1 |
| 1-84 | $MeCH{=}CHCH_2-$ | Ph | $-NH_2$ | 1 |

Table 1 (cont)

| Cpd No. | $R^1$ | $R^2$ | $R^5$ | n |
|---|---|---|---|---|
| 1-85 | $HC\equiv C-CH_2-$ | Ph | $-NH_2$ | 1 |
| 1-86 | Ph | Ph | $-NH_2$ | 1 |
| 1-87 | Bz | Ph | $-NH_2$ | 1 |
| 1-88 | H | H | $-NH_2$ | 1 |
| 1-89 | H | Me | $-NH_2$ | 1 |
| 1-90 | H | Et | $-NH_2$ | 1 |
| 1-91 | H | Pr | $-NH_2$ | 1 |
| 1-92 | H | iPr | $-NH_2$ | 1 |
| 1-93 | H | Bu | $-NH_2$ | 1 |
| 1-94 | H | iBu | $-NH_2$ | 1 |
| 1-95 | H | sBu | $-NH_2$ | 1 |
| 1-96 | H | tBu | $-NH_2$ | 1 |
| 1-97 | Cl | Me | $-NH_2$ | 1 |
| 1-98 | H | 4-ClPh | $-NH_2$ | 1 |
| 1-99 | H | 2-ClPh | $-NH_2$ | 1 |
| 1-100 | H | 3-ClPh | $-NH_2$ | 1 |
| 1-101 | H | $4-NO_2Ph$ | $-NH_2$ | 1 |
| 1-102 | H | 4-MeOPh | $-NH_2$ | 1 |
| 1-103 | H | 3-MeOPh | $-NH_2$ | 1 |
| 1-104 | H | 2-MeOPh | $-NH_2$ | 1 |
| 1-105 | H | 4-HOPh | $-NH_2$ | 1 |
| 1-106 | H | 4-FPh | $-NH_2$ | 1 |
| 1-107 | H | 2,4-diClPh | $-NH_2$ | 1 |
| 1-108 | H | 2-Thi | $-NH_2$ | 1 |
| 1-109 | H | 3-Pyr | $-NH_2$ | 1 |

Table 2

| Cpd. No. | $R^6$ | $R^7$ | $R^8$ | $R^9$ | $R^5$ |
|---|---|---|---|---|---|
| 2-1 | H | H | H | H | $-NH_2$ |
| 2-2 | H | H | H | H | -NHMe |
| 2-3 | H | H | H | H | -NHEt |
| 2-4 | H | H | H | H | -NHPr |
| 2-5 | H | H | H | H | -NHiPr |
| 2-6 | H | H | H | H | -NHBu |
| 2-7 | H | H | H | H | -NHiBu |
| 2-8 | H | H | H | H | -NHsBu |
| 2-9 | H | H | H | H | -NHtBu |
| 2-10 | H | H | H | H | -NHPh |
| 2-11 | H | H | H | H | -NHBz |
| 2-12 | H | H | H | H | $-NMe_2$ |
| 2-13 | H | H | H | H | 1-Pyrd |
| 2-14 | H | H | H | H | Mor |
| 2-15 | H | H | H | H | 1-Piz |
| 2-16 | H | H | H | H | 4-Me-1-Piz |
| 2-17 | H | H | H | H | 1-Pip |
| 2-18 | H | Cl | H | H | -NHMe |
| 2-19 | H | Cl | H | H | -NHEt |
| 2-20 | H | Cl | H | H | -NHPr |
| 2-21 | H | Cl | H | H | -NHiPr |
| 2-22 | H | Cl | H | H | -NHBu |
| 2-23 | H | Cl | H | H | -NHiBu |
| 2-24 | H | Cl | H | H | -NHsBu |
| 2-25 | H | Cl | H | H | -NHtBu |
| 2-26 | H | Cl | H | H | -NHPh |
| 2-27 | H | Cl | H | H | -NHBz |
| 2-28 | H | Cl | H | H | $-NMe_2$ |

Table 2 (cont)

| Cpd. No. | $R^6$ | $R^7$ | $R^8$ | $R^9$ | $R^5$ |
|---|---|---|---|---|---|
| 2-29 | H | Cl | H | H | 1-Pyrd |
| 2-30 | H | Cl | H | H | Mor |
| 2-31 | H | Cl | H | H | 1-Piz |
| 2-32 | H | Cl | H | H | 4-Me-1-Piz |
| 2-33 | H | Cl | H | H | 1-Pip |
| 2-34 | H | Cl | H | H | $-NH_2$ |
| 2-35 | H | H | H | Me | $-NH_2$ |
| 2-36 | H | $-NH_2$ | H | H | $-NH_2$ |
| 2-37 | H | -NHAc | H | H | $-NH_2$ |
| 2-38 | H | -OMe | H | H | $-NH_2$ |

Table 3

| Cpd No. | $R^1$ | $R^2$ | $R^5$ | n |
|---|---|---|---|---|
| 3-1 | H | Ph | $-NH_2$ | 1 |
| 3-2 | H | Ph | -NHMe | 1 |
| 3-3 | H | Ph | -NHEt | 1 |
| 3-4 | H | Ph | -NHPr | 1 |
| 3-5 | H | Ph | -NHiPr | 1 |
| 3-6 | H | Ph | -NHBu | 1 |
| 3-7 | H | Ph | -NHiBu | 1 |
| 3-8 | H | Ph | -NHsBu | 1 |
| 3-9 | H | Ph | -NHtBu | 1 |
| 3-10 | H | Ph | -NHPh | 1 |
| 3-11 | H | Ph | -NHBz | 1 |
| 3-12 | H | Ph | $-NMe_2$ | 1 |
| 3-13 | H | Ph | 1-Pyrd | 1 |
| 3-14 | H | Ph | Mor | 1 |
| 3-15 | H | Ph | 1-Piz | 1 |
| 3-16 | H | Ph | 4-Me-1-Piz | 1 |
| 3-17 | H | Ph | 1-Pip | 1 |
| 3-18 | Cl | Ph | $-NH_2$ | 1 |
| 3-19 | Cl | Ph | -NHMe | 1 |
| 3-20 | Cl | Ph | -NHEt | 1 |
| 3-21 | Cl | Ph | -NHPr | 1 |
| 3-22 | Cl | Ph | -NHiPr | 1 |
| 3-23 | Cl | Ph | -NHBu | 1 |
| 3-24 | Cl | Ph | -NHiBu | 1 |
| 3-25 | Cl | Ph | -NHsBu | 1 |
| 3-26 | Cl | Ph | -NHtBu | 1 |
| 3-27 | Cl | Ph | -NHPh | 1 |
| 3-28 | Cl | Ph | -NHBz | 1 |

Table 3 (cont)

| Cpd No. | $R^1$ | $R^2$ | $R^5$ | n |
|---|---|---|---|---|
| 3-29 | Cl | Ph | $-NMe_2$ | 1 |
| 3-30 | Cl | Ph | 1-Pyrd | 1 |
| 3-31 | Cl | Ph | Mor | 1 |
| 3-32 | Cl | Ph | 1-Piz | 1 |
| 3-33 | Cl | Ph | 4-Me-1-Piz | 1 |
| 3-34 | Cl | Ph | 1-Pip | 1 |
| 3-35 | Me | Ph | $-NH_2$ | 1 |
| 3-36 | Et | Ph | $-NH_2$ | 1 |
| 3-37 | iPr | Ph | $-NH_2$ | 1 |
| 3-38 | iBu | Ph | $-NH_2$ | 1 |
| 3-39 | All | Ph | $-NH_2$ | 1 |
| 3-40 | $MeCH=CHCH_2-$ | Ph | $-NH_2$ | 1 |
| 3-41 | $HC\equiv C-CH_2-$ | Ph | $-NH_2$ | 1 |
| 3-42 | Ph | Ph | $-NH_2$ | 1 |
| 3-43 | Bz | Ph | $-NH_2$ | 1 |
| 3-44 | H | H | $-NH_2$ | 1 |
| 3-45 | H | Me | $-NH_2$ | 1 |
| 3-46 | H | Et | $-NH_2$ | 1 |
| 3-47 | H | Pr | $-NH_2$ | 1 |
| 3-48 | H | iPr | $-NH_2$ | 1 |
| 3-49 | H | Bu | $-NH_2$ | 1 |
| 3-50 | H | iBu | $-NH_2$ | 1 |
| 3-51 | H | sBu | $-NH_2$ | 1 |
| 3-52 | H | tBu | $-NH_2$ | 1 |
| 3-53 | Cl | Me | $-NH_2$ | 1 |
| 3-54 | H | 4-ClPh | $-NH_2$ | 1 |
| 3-55 | H | 3-ClPh | $-NH_2$ | 1 |
| 3-56 | H | $4-NO_2Ph$ | $-NH_2$ | 1 |

Table 3 (cont)

| Cpd No. | $R^1$ | $R^2$ | $R^5$ | n |
|---|---|---|---|---|
| 3-57 | H | 4-MeOPh | $-NH_2$ | 1 |
| 3-58 | H | 3-MeOPh | $-NH_2$ | 1 |
| 3-59 | H | 2-MeOPh | $-NH_2$ | 1 |
| 3-60 | H | 4-HOPh | $-NH_2$ | 1 |
| 3-61 | H | 4-FPh | $-NH_2$ | 1 |
| 3-62 | H | 2,4-diCℓPh | $-NH_2$ | 1 |
| 3-63 | H | 2-Thi | $-NH_2$ | 1 |
| 3-64 | H | 3-Pyr | $-NH_2$ | 1 |
| 3-65 | H | Ph | $-NH_2$ | 0 |
| 3-66 | H | Ph | -NHMe | 0 |
| 3-67 | H | Ph | -NHEt | 0 |
| 3-68 | H | Ph | -NHPr | 0 |
| 3-69 | H | Ph | -NH<u>i</u>Pr | 0 |
| 3-70 | H | Ph | -NHBu | 0 |
| 3-71 | H | Ph | -NH<u>i</u>Bu | 0 |
| 3-72 | H | Ph | -NH<u>s</u>Bu | 0 |
| 3-73 | H | Ph | -NH<u>t</u>Bu | 0 |
| 3-74 | H | Ph | -NHPh | 0 |
| 3-75 | H | Ph | -NHBz | 0 |
| 3-76 | H | Ph | $-NMe_2$ | 0 |
| 3-77 | H | Ph | 1-Pyrd | 0 |
| 3-78 | H | Ph | Mor | 0 |
| 3-79 | H | Ph | 1-Piz | 0 |
| 3-80 | H | Ph | 4-Me-1-Piz | 0 |
| 3-81 | H | Ph | 1-Pip | 0 |
| 3-82 | Cℓ | Ph | $-NH_2$ | 0 |
| 3-83 | Cℓ | Ph | -NHMe | 0 |
| 3-84 | Cℓ | Ph | -NHEt | 0 |

Table 3 (cont)

| Cpd No. | $R^1$ | $R^2$ | $R^5$ | n |
|---|---|---|---|---|
| 3-85 | Cl | Ph | -NHPr | 0 |
| 3-86 | Cl | Ph | -NH*i*Pr | 0 |
| 3-87 | Cl | Ph | -NHBu | 0 |
| 3-88 | Cl | Ph | -NH*i*Bu | 0 |
| 3-89 | Cl | Ph | -NH*s*Bu | 0 |
| 3-90 | Cl | Ph | -NH*t*Bu | 0 |
| 3-91 | Cl | Ph | -NHPh | 0 |
| 3-92 | Cl | Ph | -NHBz | 0 |
| 3-93 | Cl | Ph | $-NMe_2$ | 0 |
| 3-94 | Cl | Ph | 1-Pyrd | 0 |
| 3-95 | Cl | Ph | Mor | 0 |
| 3-96 | Cl | Ph | 1-Piz | 0 |
| 3-97 | Cl | Ph | 4-Me-1-Piz | 0 |
| 3-98 | Cl | Ph | 1-Pip | 0 |
| 3-99 | Me | Ph | Mor | 0 |
| 3-100 | Et | Ph | $-NH_2$ | 0 |
| 3-101 | *i*Pr | Ph | $-NH_2$ | 0 |
| 3-102 | *i*Bu | Ph | $-NH_2$ | 0 |
| 3-103 | All | Ph | $-NH_2$ | 0 |
| 3-104 | $MeCH=CHCH_2-$ | Ph | $-NH_2$ | 0 |
| 3-105 | $HC\equiv C-CH_2-$ | Ph | $-NH_2$ | 0 |
| 3-106 | Ph | Ph | $-NH_2$ | 0 |
| 3-107 | Bz | Ph | $-NH_2$ | 0 |
| 3-108 | H | H | $-NH_2$ | 0 |
| 3-109 | H | Me | $-NH_2$ | 0 |
| 3-110 | H | Et | $-NH_2$ | 0 |
| 3-111 | H | Pr | $-NH_2$ | 0 |
| 3-112 | H | *i*Pr | $-NH_2$ | 0 |

Table 3 (cont)

| Cpd No. | $R^1$ | $R^2$ | $R^5$ | n |
|---|---|---|---|---|
| 3-113 | H | Bu | $-NH_2$ | 0 |
| 3-114 | H | iBu | $-NH_2$ | 0 |
| 3-115 | H | sBu | $-NH_2$ | 0 |
| 3-116 | H | tBu | $-NH_2$ | 0 |
| 3-117 | Cl | Me | $-NH_2$ | 0 |
| 3-118 | H | 4-ClPh | $-NH_2$ | 0 |
| 3-119 | H | 3-ClPh | $-NH_2$ | 0 |
| 3-120 | H | $4-NO_2Ph$ | $-NH_2$ | 0 |
| 3-121 | H | 4-MeOPh | $-NH_2$ | 0 |
| 3-122 | H | 3-MeOPh | $-NH_2$ | 0 |
| 3-123 | H | 2-MeOPh | $-NH_2$ | 0 |
| 3-124 | H | 4-HOPh | $-NH_2$ | 0 |
| 3-125 | H | 4-FPh | $-NH_2$ | 0 |
| 3-126 | H | 2,4-diClPh | $-NH_2$ | 0 |
| 3-127 | H | 2-Thi | $-NH_2$ | 0 |
| 3-128 | H | 3-Pyr | $-NH_2$ | 0 |

Table 4

| Cpd. No. | $R^6$ | $R^7$ | $R^8$ | $R^9$ | $R^5$ | n |
|---|---|---|---|---|---|---|
| 4-1 | H | H | H | H | $-NH_2$ | 1 |
| 4-2 | H | H | H | H | -NHMe | 1 |
| 4-3 | H | H | H | H | -NHEt | 1 |
| 4-4 | H | H | H | H | -NHPr | 1 |
| 4-5 | H | H | H | H | -NHiPr | 1 |
| 4-6 | H | H | H | H | -NHBu | 1 |
| 4-7 | H | H | H | H | -NHiBu | 1 |
| 4-8 | H | H | H | H | -NHsBu | 1 |
| 4-9 | H | H | H | H | -NHtBu | 1 |
| 4-10 | H | H | H | H | -NHPh | 1 |
| 4-11 | H | H | H | H | -NHBz | 1 |
| 4-12 | H | H | H | H | $-NMe_2$ | 1 |
| 4-13 | H | H | H | H | 1-Pyrd | 1 |
| 4-14 | H | H | H | H | Mor | 1 |
| 4-15 | H | H | H | H | 1-Piz | 1 |
| 4-16 | H | H | H | H | 4-Me-1-Piz | 1 |
| 4-17 | H | H | H | H | 1-Pip | 1 |
| 4-18 | H | Cl | H | H | -NHMe | 1 |
| 4-19 | H | Cl | H | H | -NHEt | 1 |
| 4-20 | H | Cl | H | H | -NHPr | 1 |
| 4-21 | H | Cl | H | H | -NHiPr | 1 |
| 4-22 | H | Cl | H | H | -NHBu | 1 |
| 4-23 | H | Cl | H | H | -NHiBu | 1 |
| 4-24 | H | Cl | H | H | -NHsBu | 1 |
| 4-25 | H | Cl | H | H | -NHtBu | 1 |
| 4-26 | H | Cl | H | H | -NHPh | 1 |
| 4-27 | H | Cl | H | H | -NHBz | 1 |
| 4-28 | H | Cl | H | H | $-NMe_2$ | 1 |

Table 4 (cont)

| Cpd. No. | $R^6$ | $R^7$ | $R^8$ | $R^9$ | $R^5$ | n |
|---|---|---|---|---|---|---|
| 4-29 | H | Cℓ | H | H | 1-Pyrd | 1 |
| 4-30 | H | Cℓ | H | H | Mor | 1 |
| 4-31 | H | Cℓ | H | H | 1-Piz | 1 |
| 4-32 | H | Cℓ | H | H | 4-Me-1-Piz | 1 |
| 4-33 | H | Cℓ | H | H | 1-Pip | 1 |
| 4-34 | H | Cℓ | H | H | $-NH_2$ | 1 |
| 4-35 | H | H | H | Me | $-NH_2$ | 1 |
| 4-36 | H | $-NH_2$ | H | H | $-NH_2$ | 1 |
| 4-37 | H | -NHAc | H | H | $-NH_2$ | 1 |
| 4-38 | H | -OMe | H | H | $-NH_2$ | 1 |
| 4-39 | H | H | H | H | -NHPh | 0 |
| 4-40 | H | H | H | H | -NHBz | 0 |
| 4-41 | H | H | H | H | $-NMe_2$ | 0 |
| 4-42 | H | H | H | H | 1-Pyrd | 0 |
| 4-43 | H | H | H | H | Mor | 0 |
| 4-44 | H | H | H | H | 1-Piz | 0 |
| 4-45 | H | H | H | H | 4-Me-1-Piz | 0 |
| 4-46 | H | H | H | H | 1-Pip | 0 |
| 4-47 | H | Cℓ | H | H | $-NMe_2$ | 0 |
| 4-48 | H | Cℓ | H | H | -N(Me)Et | 0 |
| 4-49 | H | Cℓ | H | H | -N(Me)Pr | 0 |
| 4-50 | H | Cℓ | H | H | -NHPh | 0 |
| 4-51 | H | Cℓ | H | H | -NHBz | 0 |
| 4-52 | H | Cℓ | H | H | $-NEt_2$ | 0 |
| 4-53 | H | Cℓ | H | H | 1-Pyrd | 0 |
| 4-54 | H | Cℓ | H | H | Mor | 0 |
| 4-55 | H | Cℓ | H | H | 1-Piz | 0 |

Table 4 (cont)

| Cpd. No. | $R^6$ | $R^7$ | $R^8$ | $R^9$ | $R^5$ | n |
|---|---|---|---|---|---|---|
| 4-56 | H | Cl | H | H | 4-Me-1-Piz | 0 |
| 4-57 | H | Cl | H | H | 1-Pip | 0 |
| 4-58 | H | H | Cl | H | $-NH_2$ | 0 |
| 4-59 | H | H | H | Me | $-NH_2$ | 0 |
| 4-60 | H | $-NH_2$ | H | H | $-NH_2$ | 0 |
| 4-61 | H | -NHAc | H | H | $-NH_2$ | 0 |
| 4-62 | H | -OMe | H | H | $-NH_2$ | 0 |
| 4-63 | H | F | H | H | $-NH_2$ | 1 |
| 4-64 | H | Br | H | H | $-NH_2$ | 1 |
| 4-65 | H | Me | H | H | $-NH_2$ | 1 |
| 4-66 | H | Pr | H | H | $-NH_2$ | 1 |
| 4-67 | H | iBu | H | H | $-NH_2$ | 1 |
| 4-68 | H | -OPr | H | H | $-NH_2$ | 1 |
| 4-69 | H | -OiBu | H | H | $-NH_2$ | 1 |

Of the compounds listed above, the following are particularly preferred, that is to say Compounds No. 1-13, 1-33, 1-34, 1-35, 1-37, 1-38, 1-39, 1-41, 1-45, 1-62, 1-100, 2-34, 3-14, 3-15, 3-16, 3-31, 3-32, 3-33, 3-55, 3-57, 3-58, 3-59, 3-61, 3-62, 3-78, 3-95, 3-96, 3-97, 3-98, 3-99, 3-118, 3-119, 3-121, 3-122, 3-123, 3-125, 3-126, 4-1, 4-14, 4-14, 4-15, 4-16, 4-17, 4-30, 4-31, 4-32, 4-33, 4-34, 4-43, 4-44, 4-45, 4-46, 4-54, 4-55, 4-56 and 4-57, and the following are the most preferred compounds :

1-13. 2-(2-Hydroxy-3-morpholinopropyl)-4-methyl-5-phenyl-3-isoxazolone ;
3-55. 3-(3-Carbamoyloxy-2-hydroxypropoxy)-5-(m-chlorophenyl)isoxazole ;
3-78. 3-(2-Hydroxy-3-morpholinopropoxy)-5-phenylisoxazole ;
3-99. 3-(2-Hydroxy-3-morpholinopropoxy)-4-methyl-5-phenylisoxazole ;
4-34. 3-(3-Carbamoyloxy-2-hydroxypropoxy)-5-chloro-1,2-benzisoxazole ;
and pharmaceutically acceptable salts thereof, especially the hydrochlorides.

The compounds of the present invention may be prepared by a variety of processes, each well known in themselves. In general terms, the process of the present invention may be represented as comprising the steps: (i) reacting a compound of formula (III) :

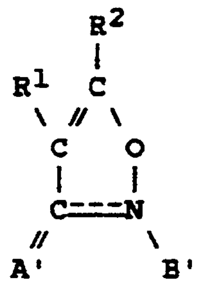

(III)

[in which : either

(a) the dotted line (- - -) represents a single bond ;

A′ = represents a oxygen atom ; and
B′ represents a group of formula (IV) :

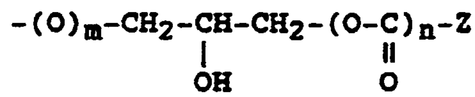

(IV)

in which m is 0 and n is 0 or 1 ; or

(b) the dotted line (- - -) represents a double bond ;

A′ = represents said group of formula (IV) in which m is 1 and n is 0 or 1 ; and
B′ is absent :

Z represents a leaving group ;
and $R^1$ and $R^2$ are as defined above]
with an amine of formula (V) :

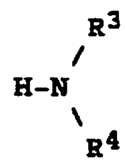

(V)

(in which $R^3$ and $R^4$ are as defined above) ;
and, if necessary, salifying the resulting product.

In more detail, preferred processes for preparing the compounds of the present invention are illustrated below in the following Methods A to D.

Method A :

Compounds of formula (I) in which n is 0, A represents an oxygen atom and B represents said group of formula (II), that is to say compounds of formula (Ia), can be prepared by reacting a compound of formula (VI):

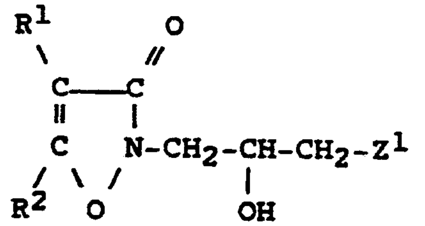

(VI)

(in which $R^1$ and $R^2$ are as defined above and $Z^1$ represents a halogen atom, for example a chlorine, bromine or iodine atom) with an amine of formula (V), defined above, to give the compound of formula (Ia) :

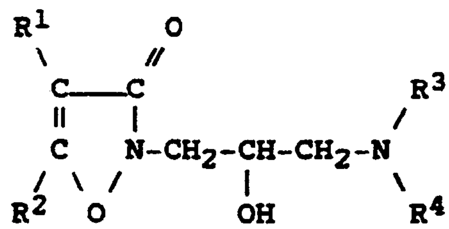

(Ia)

(in which $R^1$, $R^2$, $R^3$ and $R^4$ are as defined above).

This reaction may be carried out by condensing the 3-isoxazolone derivative of formula (VI) with an amine of formula (V) in the presence of a base. The nature of the amine of formula (V) will, of course, depend on the nature of the compound which it is desired to prepare. Examples of such compounds include : ammonia, methylamine, ethylamine, propylamine, isopropylamine, butylamine, isobutylamine, sec-butylamine, t-butylamine, dimethylamine, diethylamine, phenylamine, benzylamine, morpholine, piperazine, 1-methylpiperazine, pyrrolidine and piperidine.

The reaction is carried out in the presence of a base, the nature of which is not critical, provided that it has no adverse effect on other parts of the molecule. Examples of suitable bases include : alkali metal hydrides, such as sodium hydride ; alkali metal hydroxides, such as sodium hydroxide or potassium hydroxide ; alkali metal alkoxides, such as potassium methoxide or sodium ethoxide ; and alkali metal carbonates, such as sodium carbonate or potassium carbonate. The reaction is also preferably effected in an inert solvent. There is no particular restriction on the nature of the solvent to be employed, provided that it has no adverse effect on the reaction or on the reagents involved. Examples of suitable solvents include : alcohols, such as methanol, ethanol, propanol, isopropanol and butanol ; aromatic hydrocarbons, such as benzene, toluene or xylene ; ketones, such as acetone, methyl butyl ketone or methyl amyl ketone ; and halogenated hydrocarbons, which may be aliphatic or aromatic, such as tetrachloroethane, chlorobenzene or dichlorobenzene.

The reaction can take place over a wide range of temperatures, and the precise reaction temperature is not critical to the invention. In general, we find it convenient to carry out the reaction at a temperature from 0°C to 150°C, more preferably from 20°C to 130°C. The time required for the reaction may also vary widely, depending on many factors, notably the reaction temperature and the nature of the reagents. However, provided that the reaction is effected under the preferred conditions outlined above, a period of from 1 to 4 hours will usually suffice.

The 3-isoxazolone derivative of formula (VI) used as the starting material in this process can be prepared, for example, by the method described in Japanese Patent Provisional Publication No. Sho. 55-83766, the disclosure of which is incorporated herein by reference.

<u>Method B :</u>

Compounds of formula (I) in which <u>n</u> is 1, A = represents a hydrogen atom and said group of formula (II), defined above, and B is absent, that is to say compounds of formula (Ib), can be prepared by reacting, in a first step, an oxirane compound of formula (VII) :

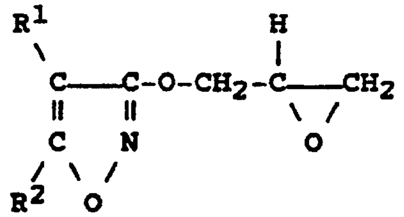

(VII)

(in which $R^1$ and $R^2$ are as defined above) with an alkali to prepare the dihydroxy compound of formula (VIII):

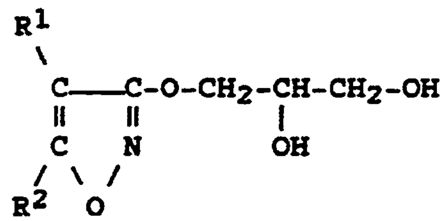

(VIII)

(in which $R^1$ and $R^2$ are as defined above), then, in a second step, reacting said compound of formula (VIII) with phosgene or a halocarbonate ester in the presence of a tertiary amine, and then, in a third step, reacting the resulting carbonate with an amine of formula (V), to give a compound of formula (Ib) :

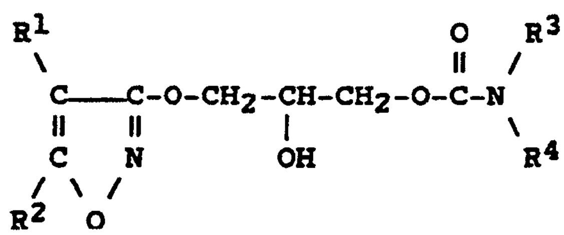

(Ib)

(in which $R^1$, $R^2$, $R^3$ and $R^4$ are as defined above).

In the first step, the reaction for preparing the dihydroxy compound of formula (VIII) is carried out by heating the oxirane compound of formula (VII) in an alkali, preferably in an aqueous solution of an alkali metal carbonate or an alkali metal hydroxide and in the presence of an organic solvent. There is no particular restriction on the nature of the solvent to be employed, provided that it has no adverse effect on the reaction or on the reagents involved. Examples of suitable solvents include : nitriles, such as acetonitrile ; alcohols, such as methanol or ethanol ; ketones, such as acetone or methyl ethyl ketone ; amides, especially fatty acid amides, such as dimethylformamide or dimethylacetamide ; and sulphoxides, such as dimethyl sulphoxide. The reagent employed in this reaction is an alkali metal carbonate (such as potassium carbonate or sodium carbonate) or an alkali metal hydroxide (such as potassium hydroxide or sodium hydroxide). The reaction can take place over a wide range of temperatures, and the precise reaction temperature is not critical to the invention. In general, we find it convenient to carry out the reaction at around the reflux temperature of the reaction mixture. The time required for the reaction may also vary widely, depending on many factors, notably the reaction temperature and the nature of the reagents. However, provided that the reaction is effected under the preferred conditions outlined above, a period of from 30 minutes to 5 hours will usually suffice.

After the reaction is complete, the dihydroxy compound of formula (VIII) can be recovered from the reaction mixture by conventional means. Thus, in one suitable recovery process, the reaction mixture, if required, is mixed with an aqueous solution of sodium chloride for salting-out ; and is then extracted with an organic solvent; the organic layer is washed and dried, and the solvent is removed by distillation by evaporation under reduced pressure, to give the desired compound. This compound may, if required, be further purified by such conventional techniques as, for example, recrystallization or the various chromatography techniques, notably column chromatography.

The second step of the reaction is effected in two stages.

The first of these stages consists of the reaction of the dihydroxy compound of formula (VIII) with phosgene or a halocarbonate ester, followed by the addition of a tertiary amine to the carbonate ester intermediate. The reaction is normally effected in the presence of an organic solvent. There is no particular restriction on the nature of the solvent to be employed, provided that it has no adverse effect on the reaction or on the reagents involved.

Examples of suitable solvents include : ethers, such as tetrahydrofuran or dioxane ; and aromatic hydrocarbons, such as benzene or toluene. A suitable halocarbonate ester is, for example, trichloromethyl chloroformate, and a suitable tertiary amine is, for example, triethylamine, dimethylaniline or pyridine. The reaction can take place over a wide range of temperatures, and the precise reaction temperature is not critical to the invention. In general, we find it convenient to carry out the reaction at around room temperature. The time required for the reaction may also vary widely, depending on many factors, notably the reaction temperature and the nature of the reagents. However, provided that the reaction is effected under the preferred conditions outlined above, a period of from 1 to 6 hours will usually suffice.

After completion of the reaction, insoluble materials are removed by filtration, and the filtrate may then be subjected to the reaction in the subsequent stage.

The reaction in the second stage consists of the reaction of an amine of formula (V) with the carbonate intermediate obtained in the preceding stage. Suitable amines of formula (V) which may be employed in this stage include those referred to in connection with Method A, although, of course, the amine chosen will depend on the nature of the desired final product. The reaction can take place over a wide range of temperatures, and the precise reaction temperature is not critical to the invention. The time required for the reaction may also vary widely, depending on many factors, notably the reaction temperature and the nature of the reagents. In general, we find it convenient to carry out the reaction at around room temperature for a period of from 1 to 5 hours, and then under reflux of the solvent employed for a further period of from 1 to 5 hours. In order to complete the reaction, if required, the reaction mixture may be concentrated, a further quantity of the amine of formula (V) and an alcohol (such as methanol or ethanol) are added to the residue, and the mixture may be heated under reflux for a period of from 1 to 5 hours.

The oxirane compound of formula (VII) used as the starting material in the above reactions can be prepared from a 3-hydroxyisoxazole compound and an epihalohydrin according to the method described in Japanese Patent Provisional Publication No. Sho. 52-31070, the disclosure of which is incorporated herein by reference.

Method C :

Compounds of formula (I) in which n is 0, A = represents a hydrogen atom and said group of formula (II), defined above, and B is absent, that is to say compounds of formula (Ic), can be prepared by reacting, in a first step, an oxirane compound of formula (VII), as defined above in Method B, with an amine of formula (V), as defined above in Method A, to give the compound of formula (Ic) :

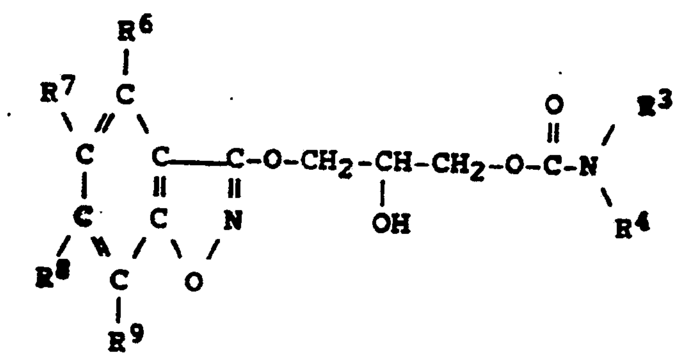

(Ic)

(in which $R^3$, $R^4$, $R^6$, $R^7$, $R^8$ and $R^9$ are as defined above).

This reaction is, in essence, the same as that described above in the second stage of the second step of Method B, and may be carried out employing the same reagents and starting materials.

Method D :

Compounds of formula (I) in which n is 1, A represents an oxygen atom and B represents said group of formula (II), that is to say compounds of formula (Id), can be prepared by reacting a compound of formula (IX):

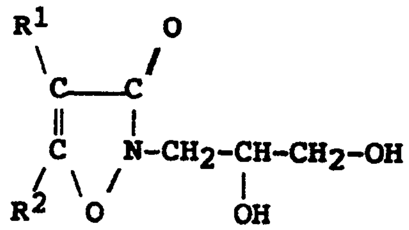

(IX)

(in which $R^1$ and $R^2$ are as defined above) with phosgene or a halocarbonate ester in the presence of a tertiary amine, and then, in a further step, reacting the resulting carbonate with an amine of formula (V), to give the compound of formula (Id) :

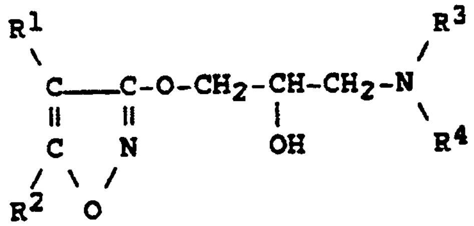

(Id)

(in which $R^1$, $R^2$, $R^3$ and $R^4$ are as defined above). These reactions are essentially the same as those in the second step of Method B, and may be carried out using the same reagents and reaction conditions.

The dihydroxy compound of formula (IX) used as the starting material in the above reaction can be prepared from a 3-hydroxyisoxazole compound and an epihalohydrin according to the method described in Japanese Patent Provisional Publication No. Sho. 55-83766, the disclosure of which is incorporated herein by reference.

After completion of any of the above reactions, the desired compound can be recovered from the reaction mixture by conventional means. For instance, if the desired compound has crystallized out from the reaction mixture, it may be collected by filtration, or, if the desired compound is in a liquid state, the solvent may be removed by distillation from the reaction mixture and the residue dissolved in a solvent which is slightly soluble in water ; the resulting solution may then be washed with an acid and water, in that order, and the desired compound can then be obtained by evaporation of the solvent. The product may, if required, be further purified by various conventional techniques such as, for example, recrystallization, vacuum distillation or the various chromatography techniques, such as column chromatography or preparative thin layer chromatography.

As shown in the pharmacological activity and toxicity tests described in the Experiments given hereafter, the compounds of the present invention have an excellent ability to counter some symptoms elicited by cerebral ischemia and have a centrally-acting muscle relaxant activity, combined with a low toxicity. Moreover, the compounds of the present invention do not induce much drowsiness, which makes their administration less difficult than that of other similar drugs.

Typically the compounds of the present invention are rapidly absorbed after oral administration, intraduodenal or intraperitoneal administration. They are expected to be particularly useful as centrally acting muscle relaxants for cerebral stroke infarction sequelae and cerebrotraumatic sequelae, and for spasrigido hemiplegia, post-operative sequelae (including tumor of the brain and spinal cord), traumatic sequelae (spinal cord injury, cephalic trauma), amyotrophic lateral sclerosis, cerebral palsy, spinocerebellar degeneration, disorders of the spinal cord vessel, SMON (sub-acute myelo-optic neuropathy), caisson disease, spastic paralysis due to any other spinal cord disease, and increased myotony such as systemic cramp or shoulder stiffness. They are also expected to be useful as cerebro-active drugs for the treatment of the acute and chronic phases of cerebral stroke infarction or for the postoperative treatment of patients with a cerebral tumor, a head injury, or the like.

The compounds of the present invention may therefore be used in the treatment of such disorders, and, for this purpose, may be formulated as conventional pharmaceutical preparations, as is well known in the art. Thus, the compounds may be administered orally, e.g. in the form of tablets, capsules, granules, powders, syrups, or other such well known forms, or parenterally, e.g. by injections, suppositories, etc.

These pharmaceutical preparations can be prepared by conventional means and may contain known adjuvants of a type commonly used in this field, for example vehicles, binders, disintegrators, lubricants, correctives, etc. depending upon the intended use and form of the preparation. The dose will depend upon the condition, age, and body weight of the patient as well as upon the nature and severity of the disorder to be treated, but

in the case of oral administration to an adult human patient, we would normally suggest a total daily dose of from 5 mg to 50 mg, which may be administered in a single dose or in divided doses, e.g. from one to three times a day.

The preparation of the compounds of the invention is further illustrated by the following Examples 1 to 20, whilst their use for the preparation of pharmaceutical formulations is illustrated in Examples 21 to 26. Preparation of certain intermediates for use in the preparation of the compounds of the present invention is then illustrated in the subsequent Preparations. In these Examples, all sieve sizes are Tyler standard mesh. The subsequent Experiments illustrate the biological activity of the compounds of the present invention.

## EXAMPLE 1

### 2-(2-Hydroxy-3-morpholinopropyl)-4-methyl-5-phenyl-3-isoxazolone

1.55 g (17.9 mmoles) of morpholine and 2.46 g (17.9 mmoles) of anhydrous potassium carbonate powder were added to a solution of 4.00 g (14.9 mmoles) of 2-(3-chloro-2-hydroxypropyl)-4-methyl-5-phenyl-3-isoxazolone in 50 ml of ethanol, and the mixture was heated under reflux for 4 hours. At the end of this time, the reaction mixture was cooled by allowing it to stand, and then insoluble materials were removed by filtration. The filtrate was then concentrated by evaporation under reduced pressure, and the residue was purified by column chromatography through silica gel, using ethyl acetate containing 5% by volume methanol as the eluent, to give 4.21 g (yield 83.5%) of the title compound as a colourless syrupy substance.

$$n_D^{24} = 1.5740.$$

Infrared Absorption Spectrum ($CHCl_3$) $\nu_{max}$ $cm^{-1}$ : 3400 (OH), 1660 (shoulder), 1645 (C = O).
Nuclear Magnetic Resonance Spectrum ($CDCl_3$) δ ppm :

| | |
|---|---|
| 2.10 | (3H, singlet) ; |
| 2.23-2.83 | (2H × 3, multiplet) ; |
| 3.70 | (2H × 2, triplet, J = 4.5 Hz) ; |
| 3.93 | (1H, broad singlet) ; |
| 4.10 | (2H, doublet, J = 3.0 Hz) ; |
| 3.80-4.30 | (1H, multiplet) ; |
| 7.26-7.83 | (5H, multiplet). |

## EXAMPLE 2

### 2-(2-Hydroxy-3-morpholinopropyl)-4-methyl-5-phenyl-3-isoxazolone hydrochloride

2.00 ml of a 4N solution of hydrogen chloride in dioxane were added to a solution of 2.50 g (7.4 mmoles) of 2-(2-hydroxy-3-morpholinopropyl)-4-methyl-5-phenyl-3-isoxazolone (prepared as described in Example 1) in 25 ml of ethanol, and the mixture was stirred at room temperature for 3 hours. At the end of this time, the crystals which deposited were collected by filtration, washed with 10 ml of isopropanol, and recrystallized from ethanol, to give 2.30 g (yield 87.7%) of the title compound as a colourless powder, melting at 130-133°C.
Infrared Absorption Spectrum (KBr) $\nu_{max}$ $cm^{-1}$ :

3700-3100 (—OH), 1646 (C = O).

Nuclear Magnetic Resonance Spectrum ($D_2O$) δ ppm :

| | |
|---|---|
| 2.40 | (3H, singlet) ; |
| 3.60-4.10 | (2H × 3, multiplet) ; |
| 4.44 | (2H, doublet, J = 4.5 Hz) ; |
| 4.53 | (2H × 2, triplet, J = 4.5 Hz) ; |
| 4.83-5.23 | (1H, multiplet) ; |
| 7.86-8.26 | (5H, multiplet). |

EXAMPLE 3

3-(3-Carbamoyloxy-2-hydroxypropoxy)-5-chloro-1,2-benzisoxazole

3(a) 5-Chloro-3-(2,3-dihydroxypropoxy)-1,2-benzisoxazole

256 ml of a 10% w/v aqueous solution of potassium carbonate were added to a suspension of 21.0 g (92.7 mmoles) of 5-chloro-3-(2,3-epoxypropoxy)-1,2-benzisoxazole (prepared according to the method described in Japanese Patent Provisional Publication No. Sho. 52-31070) in 100 ml of acetonitrile, and the mixture was then heated under reflux for 3 hours. At the end of this time, the reaction mixture was cooled by allowing it to stand, and then 500 ml of water were added. The reaction mixture was then extracted with 1 liter of ethyl acetate. The organic extract was dried over anhydrous magnesium sulphate, the desiccant was removed by filtration, and the solvent was removed by distillation to give a solid residue. This was then recrystallized from a mixture of ethyl acetate and diethyl ether, to give 16.7 g (yield 74.2%) of the title compound as a colourless amorphous powder, melting at 61-62°C.
Infrared Absorption Spectrum (KBr) $\nu_{max}$ $cm^{-1}$ :
3410 (OH), 1600, 1539 (C = N, Ar).
Nuclear Magnetic Resonance Spectrum ($CDC\ell_3$) δ ppm :

| | |
|---|---|
| 2.90 | (1H × 2, broad singlet) ; |
| 3.60-4.00 | (2H, multiplet) ; |
| 4.00-4.40 | (1H, multiplet) ; |
| 4.52 | (2H, doublet, J = 4.5 Hz) ; |
| 7.20-7.70 | (3H, multiplet). |

3(b) 3-(3-Carbamoyloxy-2-hydroxypropoxy)-5-chloro-1,2-benzisoxazole

5.9 g (29.8 mmoles) of trichloromethyl chloroformate were added dropwise at 10°C to a solution of 16.0 g (55.8 mmoles) of 5-chloro-3-(2,3-dihydroxypropoxy)-1,2-benzisoxazole [prepared as described in step (a) above] in 500 ml of dry benzene ; the mixture was then stirred at the same temperature for 30 minutes, after which 6.1 g (60.3 mmoles) of triethylamine were added dropwise at 10-15°C. The mixture was then stirred for a further 1 hour at 5-10°C, after which insoluble materials were removed by filtration and washed with 100 ml of dry benzene. The filtrate and washings were combined, and cooled to 10°C, and then 38.0 ml (304.0 mmoles) of 28% v/v aqueous ammonia were added thereto at one time. The mixture was then stirred at room temperature for 2 hours, after which it was heated under reflux for an additional 2 hours. The reaction mixture was then concentrated by evaporation under reduced pressure, and 38.0 ml (304.0 mmoles) of 28% v/v aqueous ammonia and 200 ml of ethanol were added to the residue. The resulting mixture was then heated under reflux for a further 2 hours. At the end of this time, the reaction mixture was cooled by allowing it to stand, and the cooled mixture was then concentrated by evaporation under reduced pressure. The residue was purified by column chromatography through silica gel, eluted with a 1 : 2 by volume mixture of benzene and ethyl acetate, followed by recrystallization from ethyl acetate, to give 11.6 g (72.5%) of the title compound as a colourless amorphous powder, melting at 123-124°C.
Infrared Absorption Spectrum (KBr) $\nu_{max}$ $cm^{-1}$ :
3395, 3340, 3280, 3200 ($NH_2$, OH), 1730 (C = O).
Nuclear Magnetic Resonance Spectrum (hexadeuterated dimethyl sulphoxide) δ ppm :

| | |
|---|---|
| 3.96-4.50 | (2H + 2H + 1H, multiplet) |
| 5.34 | (1H, doublet, J = 4.5 Hz) ; |
| 6.50 | (2H, broad singlet) ; |
| 7.60-7.90 | (3H, multiplet). |

EXAMPLE 4

3-(3-Carbamoyloxy-2-hydroxypropoxy)-5-(m-chlorophenyl)isoxazole

4(a) 5-(m-Chlorophenyl)-3-(2,3-dihydroxypropoxy)isoxazole

100 ml (72.3 mmoles) of a 10% w/v aqueous solution of potassium carbonate were added to a solution of

15.0 g (59.6 mmoles) of 5-(m-chlorophenyl)-3-(2,3-epoxypropoxy)isoxazole (prepared as described in Preparation 2) in 50 ml of acetonitrile, and the mixture was heated under reflux for 3 hours. At the end of this time, the reaction mixture was cooled by allowing it to stand, and then 400 ml of a 10% w/v aqueous solution of sodium chloride were added thereto, and the mixture was extracted twice, each time with 400 ml of ethyl acetate. The combined organic extracts were dried over anhydrous magnesium sulphate, and the solvent was removed by distillation under reduced pressure. The resulting residue was then purified by column chromatography through silica gel, eluted with a 1 : 2 by volume mixture of benzene and ethyl acetate, to give 13.2 g (yield 82.5%) of the title compound as a colourless amorphous powder, melting at 92-93°C.
Infrared Absorption Spectrum (KBr) $\nu_{max}$ $cm^{-1}$ :
3370 (OH), 3105 (Hetero-H).
Nuclear Magnetic Resonance Spectrum (hexadeuterated dimethyl sulphoxide) δ ppm :

| | |
|---|---|
| 3.30-3.70 | (2H, multiplet) ; |
| 3.70-4.10 | (1H, multiplet) ; |
| 4.00-4.50 | (2H, multiplet) ; |
| 4.56 | (1H, triplet, J = 4.5 Hz) ; |
| 5.06 | (1H, doublet, J = 4.5 Hz) ; |
| 6.93 | (1H, singlet) ; |
| 7.43-8.06 | (4H, multiplet). |

4(b) 3-(3-Carbamoyloxy-2-hydroxypropoxy)-5-(m-chlorophenyl)isoxazole

A solution of 10.0 g (37.0 mmoles) of 5-(m-chlorophenyl)-3-(2,3-dihydroxypropoxy)isoxazole [prepared as described in step (a) above] in 300 ml of dry tetrahydrofuran was cooled to 5°C, and then 3.96 g (20.0 mmoles) of trichloromethyl chloroformate were added dropwise to the mixture. The mixture was then stirred at 5-6°C for 30 minutes. At the end of this time, 4.04 g (40.0 mmoles) of triethylamine were added dropwise to the mixture at 5-10°C, and the mixture was stirred at 3-5°C for 1 hour. 50 ml (400.0 mmoles) of 28% v/v aqueous ammonia were then added at one time, and the mixture was stirred at room temperature for a further 18 hours. The reaction mixture was then concentrated by evaporation under reduced pressure, and the resulting residue was diluted with 400 ml of ethyl acetate. The mixture was then washed with 400 ml of a 10% w/v aqueous solution of sodium chloride. The organic layer was dried over anhydrous magnesium sulphate, the desiccant was removed by filtration, and the solvent was removed by distillation under reduced pressure to give a solid residue. This solid residue was recrystallized from ethyl acetate, to give 8.30 g (yield 72.1%) of the title compound as colourless scales, melting at 149-150°C.
Infrared Absorption Spectrum (KBr) $\nu_{max}$ $cm^{-1}$ :
3430, 3320, 3245 ($NH_2$, OH), 3120 (Hetero-H), 1683 (C = O).
Nuclear Magnetic Resonance Spectrum (hexadeuterated dimethyl sulphoxide) δ ppm :

| | |
|---|---|
| 3.86-4.33 | (2H + 2H + 1H, multiplet) |
| 5.32 | (1H, doublet, J = 4.5 Hz) ; |
| 6.50 | (2H, broad singlet) ; |
| 6.93 | (1H, singlet) ; |
| 7.50-8.00 | (4H, multiplet). |

EXAMPLE 5

3-(2-Hydroxy-3-morpholinopropoxy)-4-methyl-5-phenylisoxazole

1.40 g of morpholine was added to a solution of 3.00 g of 3-(2,3-epoxypropoxy)-4-methyl-5-phenylisoxazole (prepared as described in Preparation 3) in 50 ml of ethanol, and the mixture was heated under reflux for 3 hours. At the end of this time, the reaction mixture was concentrated by evaporation under reduced pressure, and the resulting solid residue was recrystallized from diethyl ether, to give 3.75 g (yield 90.7%) of the title compound as a colourless powder, melting at 94-95°C.
Infrared Absorption Spectrum (KBr) $\nu_{max}$ $cm^{-1}$ :
3380 (OH).
Nuclear Magnetic Resonance Spectrum ($CDC\ell_3$) δ ppm :

| | |
|---|---|
| 2.10 | (3H, singlet) ; |

| | |
|---|---|
| 2.30-2.85 | (2H × 3, multiplet) ; |
| 2.70-3.40 | (1H, broad) ; |
| 3.71 | (2H × 2, triplet, J = 4.5 Hz) ; |
| 4.00-4.55 | (1H, multiplet) ; |
| 4.25-4.50 | (2H, multiplet) ; |
| 7.35-7.85 | (5H, multiplet). |

EXAMPLE 6

3-(2-Hydroxy-3-morpholinopropoxy)-4-methyl-5-phenylisoxazole hydrochloride

2.75 ml of a 4N solution of hydrogen chloride in dioxane were added to a solution of 3.18 g of 3-(2-hydroxy-3-morpholinopropoxy)-4-methyl-5-phenylisozazole (prepared as described in Example 5) in 50 ml of ethanol, and the mixture was stirred at room temperature for 3 hours. At the end of this time, the crystalline substance which deposited was recrystallized from ethanol, to give 3.20 g (yield 90.4%) of the title compound as colourless granules, melting at 189-191°C (with decomposition).

Infrared Absorption Spectrum (KBr) $\nu_{max}$ $cm^{-1}$ :

3405 (OH).

Nuclear Magnetic Resonance Spectrum ($D_2O$) δ ppm :

| | |
|---|---|
| 2.30 | (3H, singlet) ; |
| 3.60-4.10 | (2H × 3, multiplet) ; |
| 4.50 | (2H, doublet, J = 4.5 Hz) ; |
| 4.63 | (2H, doublet, J = 4.5 Hz) ; |
| 4.70-5.20 | (1H, multiplet) ; |
| 7.90 | (5H, singlet). |

EXAMPLES 7 TO 14

Following a procedure similar to that described in Examples 5 and 6, the compounds listed in the following Table 5 were prepared.

Table 5

| Example No. | Compound | Melting Point(°C) |
|---|---|---|
| 7 | 3-(2-Hydroxy-3-morpholinopropoxy)-5-phenylisoxazole | 123 - 124 |
| 8 | 4-Chloro-3-(2-hydroxy-3-morpholinopropoxy)-5-phenylisoxazole | 75 - 76 |
| 9 | 5-(p-Chlorophenyl)-3-(2-hydroxy-3-morpholinopropoxy)isoxazole | 114 - 115 |
| 10 | 3-(2-Hydroxy-3-morpholinopropoxy)-5-phenylisoxazole . HCℓ | 149 - 150 |
| 11 | 3-(3-Hexylamino-2-hydroxypropoxy)-5-phenylisoxazole | 115 - 116 |
| 12 | 5-(m-Chlorophenyl)-3-(2-hydroxy-3-morpholinopropoxy)isoxazole | 76 - 77 |
| 13 | 5-Chloro-3-(2-hydroxy-3-morpholinopropoxy)-1,2-benzisoxazole | 73 - 74 |
| 14 | 4-Chloro-3-(2-hydroxy-3-morpholinopropoxy)-5-phenylisoxazole . HCℓ | 200 - 202 (with decomp.) |

## EXAMPLE 15

### 2-(3-Carbamoyloxy-2-hydroxypropyl)-5-chloro-1,2-benzisoxazol-3-one

0.40 g of trichloromethyl chloroformate was added to a solution of 1.00 g of 5-chloro-2-(2,3-dihydroxypropyl)-1,2-benzisoxazol-3-one (prepared as disclosed in Japanese Patent Application Kokai No. Sho. 55-83766) in 20 ml of dry tetrahydrofuran and the mixture was then stirred at room temperature for 30 minutes. After this, 0.42 g of triethylamine was added dropwise at 5°C, and the mixture was again stirred at the same temperature for 30 minutes. At the end of this time, 5.0 ml of 28% v/v aqueous ammonia were added, and the mixture was stirred at room temperature for 2 hours. It was then heated under reflux for an additional 3 hours. The reaction mixture was then concentrated by evaporation under reduced pressure, and the residue was dissolved in 100 ml of ethyl acetate, washed with a 10% w/v aqueous solution of sodium chloride, and dried over anhydrous magnesium sulphate. The solvent was then removed by distillation under reduced pressure, and the residue was purified by column chromatography through silica gel, eluted with ethyl acetate, to give 0.75 g (yield 64.1%) of the title compound as a colourless powder, melting at 161-162°C.

Infrared Absorption Spectrum (KBr) $\nu_{max}$ $cm^{-1}$ :

3420, 3320, 3260 (OH, $NH_2$), 1683, 1662 (C = O).

Nuclear Magnetic Resonance Spectrum (hexadeuterated dimethyl sulphoxide) δ ppm :

3.86-4.46 (5H, broad) ;
5.31 (1H, doublet, J = 4.5 Hz) ;
6.50 (2H, singlet) ;
7.46-7.90 (3H, multiplet).

## EXAMPLES 16 TO 18

Following a procedure similar to that described in Example 15, the compounds listed in the following Table 6 were prepared.

### Table 6

| Example No. | Compound | Melting Point(°C) |
|---|---|---|
| 16 | 2-(3-Carbamoyloxy-2-hydroxypropyl)-5-phenyl-3-isoxazolone | 183 - 184 |
| 17 | 2-(3-Carbamoyloxy-2-hydroxypropyl)-4-chloro-5-phenyl-3-isoxazolone | 114 - 115 |
| 18 | 2-(3-Carbamoyloxy-2-hydroxypropyl)-5-(m-chlorophenyl)-3-isoxazolone | 138 - 140 |

EXAMPLE 19

3-(2-Hydroxy-3-morpholinopropoxy)-5-phenylisoxazole

17.6 g of morpholine were added to a solution of 40.0 g of 3-(2,3-epoxypropoxy)-5-phenylisoxazole (prepared as described in Preparation 4) in 400 ml of ethanol, and the mixture was heated under reflux for 5 hours. At the end of this time, the reaction mixture was concentrated by evaporation under reduced pressure, and the resulting solid residue was recrystallized from ethyl acetate, to give 50.0 g (yield 89.2%) of the title compound as colourless columnar crystals, melting at 123-124°C.
Infrared Absorption Spectrum (KBr) $\nu_{max}$ $cm^{-1}$ :
3190, 1624, 1511, 1440.
Nuclear Magnetic Resonance Spectrum ($CDC\ell_3$) δ ppm :

2.30-2.85 (2H × 3, multiplet) ;
3.20-3.70 (1H, broad) ;
3.73 (2H × 2, triplet, J = 4.5 Hz) ;
3.90-4.55 (1H, multiplet) ;
4.15-4.50 (2H, multiplet) ;
6.18 (1H, singlet) ;
7.35-7.85 (5H, multiplet).

EXAMPLE 20

3-(2-Hydroxy-3-morpholinopropoxy)-5-phenylisoxazole hydrochloride

5.0 ml of a 4N solution of hydrogen chloride in dioxane were added to a solution of 5.00 g of 3-(2-hydroxy-3-morpholinopropoxy)-5-phenylisoxazole (prepared as described in Example 19) in 200 ml of ethyl acetate, and the mixture was stirred at room temperature for 10 minutes. At the end of this time, the reaction mixture was concentrated by evaporation under reduced pressure, and the resulting solid residue was recrystallized from ethyl acetate, to give 5.21 g (yield 93.0%) of the title compound as a colourless powder, melting at 149-150°C.
Infrared Absorption Spectrum (KBr) $\nu_{max}$ $cm^{-1}$ :
3215, 1625, 1513, 1461.
Nuclear Magnetic Resonance Spectrum ($D_2O$) δ ppm :

3.66-4.13 (2H × 3, multiplet) ;
4.50 (2H × 2, triplet, J = 4.5 Hz) ;
4.69 (2H, doublet, J = 4.5 Hz) ;
4.80-5.20 (1H, multiplet) ;
6.83 (1H, singlet) ;
7.80-8.30 (5H, multiplet).

EXAMPLE 21

Capsules

The following powders were mixed :

| | |
|---|---|
| 2-(2-Hydroxy-3-morpholinopropyl)-4-methyl-5-phenyl-3-isoxazolone hydrochloride (compound of Example 2) | 25.0 mg |
| Lactose | 153.6 mg |
| Corn starch | 100.0 mg |
| Magnesium stearate | 1.4 mg |
| Total | 280.0 mg |

and then passed through a 60 mesh sieve. 280 mg of the resulting powder were packed into a No. 3 gelatin capsule.

EXAMPLE 22

Capsules

The following powders were mixed :

| | |
|---|---|
| 3-(3-Carbamoyloxy-2-hydroxypropoxy)-5-chloro-1,2-benzisoxazole (The compound of Example 3) | 25.0 mg |
| Lactose | 153.6 mg |
| Corn starch | 100.0 mg |
| Magnesium stearate | 1.4 mg |
| Total | 280.0 mg |

and then passed through a 60 mesh sieve. 280 mg of the resulting powder were packed into a No. 3 gelatin capsule.

EXAMPLE 23

Capsules

The following powders were mixed :

| | |
|---|---|
| 3-(2-Hydroxy-3-morpholinopropoxy)-4-methyl-5-phenylisoxazole (The compound of Example 5) | 25.0 mg |
| Lactose | 153.6 mg |
| Corn starch | 100.0 mg |
| Magnesium stearate | 1.4 mg |
| Total | 280.0 mg |

and then passed through a 60 mesh sieve. 280 mg of the resulting powder were packed into a No. 3 gelatin capsule.

EXAMPLE 24

Capsules

The following powders were mixed :

| | |
|---|---|
| 3-(3-Carbamoyloxy-2-hydroxypropoxy)-5-(m-chlorophenyl)isoxazole (the compound of Example 4) | 25.0 mg |
| Lactose | 153.6 mg |
| Corn starch | 100.0 mg |
| Magnesium stearate | 1.4 mg |
| Total | 280.0 mg |

and then passed through a 60 mesh sieve. 280 mg of the resulting powder were packed into a No. 3 gelatin capsule.

EXAMPLE 25

Capsules

The following powders were mixed :

| | |
|---|---|
| 2-(3-Carbamoyloxy-2-hydroxypropyl)-5-chlorobenzisoxazolin-3-one (The compound of Example 15) | 25.0 mg |
| Lactose | 153.6 mg |
| Corn starch | 100.0 mg |
| Magnesium stearate | 1.4 mg |
| Total | 280.0 mg |

and then passed through a 60 mesh sieve. 280 mg of the resulting powder were packed into a No. 3 gelatin capsule.

EXAMPLE 26

Capsules

The following powders were mixed :

| | |
|---|---|
| 3-(2-Hydroxy-3-morpholinopropoxy)-5-phenylisoxazole hydrochloride (the compound of Example 20) | 25.0 mg |
| Lactose | 153.6 mg |
| Corn starch | 100.0 mg |
| Magnesium stearate | 1.4 mg |
| Total | 280.0 mg |

and then passed through a 60 mesh sieve. 280 mg of the resulting powder were packed into a No. 3 gelatin capsule.

PREPARATION 1

2-(3-Chloro-2-hydroxypropyl)-4-methyl-5-phenyl-3-isoxazolone

A mixture of 6.00 g (34.2 mmoles) of 3-hydroxy-4-methyl-5-phenylisoxazole and 6.33 g (68.4 mmoles) of epichlorohydrin was stirred, whilst heating at 75°C, for 5 hours. At the end of this time, the excess epichlorohydrin was removed by distillation under reduced pressure, and the resulting residue was purified by column chromatography through silica gel, using a 2 : 1 by volume mixture of cyclohexane and ethyl acetate as eluent, to give 6.51 g (yield 71.1%) of the title compound as a colourless powder, melting at 82-83°C.

Infrared Absorption Spectrum (KBr) $\nu_{max}$ $cm^{-1}$ :

3265 (OH), 1651, 1637 (C = O).

Nuclear Magnetic Resonance Spectrum ($CDCℓ_3$) δ ppm :

2.10 (3H, singlet) ;
3.40-3.83 (2H, multiplet) ;
4.23 (2H, doublet, J = 3.0 Hz) ;
3.90-4.50 (1H, broad singlet) ;
4.63 (1H, broad singlet) ;

| | |
|---|---|
| 7.30-7.83 | (5H, multiplet). |

PREPARATION 2

5-(m-Chlorophenyl)-3-(2,3-epoxypropoxy)isoxazole

29.6 g (0.153 moles) of sodium methoxide (as a 28% w/v methanolic solution) were added to a solution of 30.0 g (0.153 moles) of 5-(m-chlorophenyl)-3-hydroxyisoxazole in 300 ml of dimethylformamide, and the mixture was stirred at room temperature for 30 minutes. At the end of this time, 41.9 g (0.306 moles) of epibromohydrin were added dropwise, and the mixture was stirred for a further 3 days. The reaction mixture was then concentrated by evaporation under reduced pressure, and the residue was diluted with ethyl acetate. The resulting mixture was washed with 800 ml of a 10% w/v aqueous solution of sodium chloride. The organic layer was then dried over anhydrous magnesium sulphate, the desiccant was removed by filtration, and the solvent was removed by distillation under reduced pressure. The residue was purified by column chromatography through silica gel, eluted with a 2 : 1 by volume mixture of benzene and ethyl acetate, to give a solid residue, which was recrystallized from diisopropyl ether, to give 23.6 g (yield 73.5%) of the title compound as colourless needles, melting at 86-87°C.

Infrared Absorption Spectrum (KBr) $\nu_{max}$ $cm^{-1}$ :

3120 (Hetero-H), 1620, 1593 (C = N, Ar).

Nuclear Magnetic Resonance Spectrum ($CDC\ell_3$) δ ppm :

| | |
|---|---|
| 2.72 | (1H, AB-doublet of doublets, J = 4.5 & 3.0 Hz) ; |
| 2.88 | (1H, AB-doublet of doublets, J = 4.5 & 4.5 Hz) ; |
| 3.26-3.50 | (1H, multiplet) ; |
| 4.18 | (1H, AB-doublet of doublets, J = 12.0 & 6.0 Hz) ; |
| 4.58 | (1H, AB-doublet of doublets, J = 12.0 & 3.0 Hz) ; |
| 6.20 | (1H, singlet) ; |
| 7.23-7.83 | (4H, multiplet). |

PREPARATION 3

3-(2,3-Epoxypropoxy)-4-methyl-5-phenylisoxazole

7.32 g of sodium methoxide were added to a solution of 6.00 g of 3-hydroxy-4-methyl-5-phenylisoxazole in 500 ml of dimethylformamide, and the mixture was stirred at room temperature for 10 minutes. 10.4 g of epibromohydrin were then added dropwise at room temperature to the mixture, and the mixture was stirred at room temperature for 48 hours. At the end of this time, the reaction mixture was concentrated by evaporation under reduced pressure, and the residue was purified by column chromatography through silica gel, eluted with a 2: 1 by volume mixture of cyclohexane and ethyl acetate, to give 5.60 g of the title compound as colourless cotton-like crystals, melting at 87-88°C.

Infrared Absorption Spectrum (KBr) $\nu_{max}$ $cm^{-1}$ :

1519.

Nuclear Magnetic Resonance Spectrum ($CDC\ell_3$) δ ppm :

| | |
|---|---|
| 2.10 | (3H, singlet) ; |
| 2.71 | (1H, AB-doublet of doublets, J = 4.5, 3.0 Hz) ; |
| 2.86 | (1H, AB-doublet of doublets, J = 4.5, 4.5 Hz) ; |
| 3.26-3.53 | (1H, multiplet) ; |
| 4.20 | (1H, AB-doublet of doublets, J = 12.0, 6.0 Hz) ; |
| 4.60 | (1H, AB-doublet of doublets, J = 12.0, 3.0 Hz) ; |
| 7.20-7.83 | (5H, multiplet). |

PREPARATION 4

3-(2,3-Epoxypropoxy)-5-phenylisoxazole

10.28 g of anhydrous potassium carbonate and 6.89 g of epichlorohydrin were added to a solution of 10.00 g of 3-hydroxy-5-phenylisoxazole in 50 ml of hexamethylphosphoric triamide, and the mixture was stirred at

room temperature for 24 hours. At the end of this time, insoluble materials were removed by filtration from the reaction mixture, and 200 ml of ethyl acetate were added thereto. The mixture was then washed twice, each time with 200 ml of a 10% w/v aqueous solution of sodium chloride. The organic layer was then dried over anhydrous magnesium sulphate, the solvent was removed by distillation under reduced pressure, and the residue was purified by column chromatography through silica gel, eluted with a 4 : 1 by volume mixture of cyclohexane and ethyl acetate, to give 11.00 g (yield 82%) of the title compound as colourless needles, melting at 98-99°C.

Infrared Absorption Spectrum (KBr) $\nu_{max}$ $cm^{-1}$ :

1615, 1585, 1511, 1459, 1418.

Nuclear Magnetic Resonance Spectrum ($CDC\ell_3$) $\delta$ ppm :

| | |
|---|---|
| 2.73 | (1H, AB-doublet of doublets, J = 4.5 & 3.0 Hz) ; |
| 2.87 | (1H, AB-doublet of doublets, J = 4.5 & 4.5 Hz) ; |
| 3.26-3.50 | (1H, multiplet) ; |
| 4.20 | (1H, AB-doublet of doublets, J = 12.0 & 6.0 Hz) ; |
| 4.58 | (1H, AB-doublet of doublets, J = 12.0 & 3.0 Hz) ; |
| 6.20 | (H, singlet) ; |
| 7.30-7.90 | (5H, multiplet). |

## EXPERIMENT 1

### Suppression of decerebrate rigidity in rats

The suppression of decerebrate rigidity in laboratory animals, such as rats, is a well known model for control of human spasticity and/or rigidity.

In the test method, a rat was anesthetized with Halothane (Trade Mark) and fixed on a stereotaxic apparatus (SR-5, Narishige). In accordance with the brain atlas by Pellegrino et al., [L J Pellegrino, A S Pellegrino & A. J. Cushman : A Stereotaxic Atlas of the Rat Brain, Plenum Press, New York and London (1967)], an electrode 0.7 mm in diameter, which was insulated except for 1 mm at the tip, was inserted bilaterally into the midbrain reticular formation (AP : 0, L : ±1.5, H : −3.0). A clip serving as the reference electrode was fixed on the temporal muscle. Through these electrodes, a high frequency electric current (100 KHz, 10 to 20 mA) was applied from a lesion generator (Glass Co., LM4A) for 2 to 3 minutes to cauterize electrically this brain region.

The rat was then immediately set free from the stereotaxic apparatus. A polyethylene cannula was inserted into the duodenum and fixed with an acrylic resin (Aron Alfa, Sankyo Co., Ltd.). After completion of these operations, Halothane anesthesia was immediately discontinued.

After 1.5 hours, when the animal awoke from the anesthesia, the rat was fixed on an apparatus devised in our laboratory for a hind leg fixation. Both hind legs were fixed at the anterior part of the ankle joints. Both hindlimb paws were then pushed in 4 mm for 6 seconds in every minute. The resulting repulsive tension was drawn on a polygraph through a strain gauge.

The test compounds were suspended in a 0.5% w/v CMC (carboxymethyl cellulose) aqueous solution and were administered orally (p.o.) or intraperitoneally (i.p.).

The results are summarized in the following Table 7, where the compounds of the present invention are identified by the number of the foregoing Example in which each was prepared, Compound X is the known compound eperisone hydrochloride, and Compound Y is the known compound afloqualone [6-amino-2-fluoromethyl-3-o-tolylquinozolin-4(3H)one].

Table 7

Inhibition of decerebrate rigidity in rats

| Cpd. of Ex. No. | Dose (mg/kg) (Administration route) | Onset of the effect (minutes) | Maximum inhibition (%) | Duration of the effect (minutes) |
|---|---|---|---|---|
| 2 | 100 (i.p.) | 5 | 60 | >90 |
| 3 | 20 (p.o.) | 5 | 60 | >90 |
| 3 | 30 (p.o.) | 5 | 90 | >90 |
| 3 | 100 (p.o.) | 5 | 90 | >90 |
| 5 | 100 (p.o.) | 5 | 70 | >90 |
| 15 | 50 (i.p.) | 5 | 50 | >60 |
| X | 10 (p.o.) | 10 | 30 | >30 |
| X | 30 (p.o.) | 10 | 30 | 45 |
| X | 50 (p.o.) | 10 | 50 | 30 |
| X | 100 (p.o.) | 5 | 50 | 60 |
| Y | 5 (p.o.) | 10 | 25 | 60 |
| Y | 10 (p.o.) | 10 | 55 | 45 |
| Y | 30 (p.o.) | 10 | 70 | 120 |

All of the compounds of the present invention were effective in suppressing decerebrate rigidity in rats, which is recognised as a model for spasticity and/or rigidity in humans, showing an activity comparable with, or better than, that of eperisone hydrochloride (Compound X) or afloqualone (Compound Y), both of which have already been used clinically as therapeutic agents for myotonia caused by cerebrovascular disorder sequelae.

EXPERIMENT 2

Improvement of ischemia-induced neurological symptoms in the Mongolian Gerbil caused by ligation of the bilateral common carotid arteries for 30 minutes

The test animals employed were male adult Mongolian gerbils (20 weeks old). These were used in groups each consisting of from 10 to 20 animals. The bilateral common carotid arteries were occluded for 30 minutes under anesthesia with pentobarbital (30 mg/kg, intraperitoneally) and halothane (it was introduced to a mixture of 95% oxygen and 5% carbon dioxide at a concentration of 1.5% by volume) and then the occlusion was released to allow the blood to flow. The animal was then placed in a supine position, and we measured (1) the

time taken from recommencement of blood flow until convulsions occurred and (2) the survival time. Observations to determine the onset time of convulsions and the survival time were continued for 6 and 7 hours, respectively, after recommencement of blood flow. If no convulsions occurred within 6 hours after the recommencement of blood flow, the onset time of convulsions was assumed to be 360 minutes, and, if the animal did not die within 7 hours, the survival time was recorded as 420 minutes for calculation purposes. The compound to be tested was suspended in a 0.5% w/v carboxymethyl cellulose (CMC) aqueous solution and administered intraperitoneally upon recommencement of blood flow in the common carotid arteries. The dose of test compound administered was 100 mg/kg, and the compound used is as shown in the following Table 8. On the other hand, a 0.5% CMC solution containing no test compound was administered likewise to a control group. Statistical analysis was carried out using the Mann-Whitney U-test between the control group and the test group.

The results are shown in Table 8. As can be seen from these results, in the group to which the compounds of the present invention were administered at a dose of 100 mg/kg, both onset time of convulsions and survival time were significantly prolonged.

**Table 8**

| Cpd. of Ex. No. | No. of animals | Dose (mg/kg) | Recovery time of righting reflex (min.) | Onset time of convulsions (min.) | Survival time (min.) |
|---|---|---|---|---|---|
| 4 | 18 | 100 | 115.7 ±8.7 | 233.9 ±12.2* | 344.2 ±15.1*** |
| Control | 18 | - | 107.7 ±10.0 | 183.5 ±17.4 | 282.3 ±13.9 |

* = $p < 0.05$.

*** = $p < 0.01$.

EXPERIMENT 3

Improvement in ischemia-induced neurological symptoms in Spontaneously Hypertensive Rats Stroke Prone (SHR SP) caused by ligation of the bilateral common carotid arteries

The test animals employed were male adult SHR SPs (13 or 15 weeks old). They were used in groups each consisting of about 10 animals. Under halothane anesthesia, both common carotid arteries of each animal were ligated to prepare a brain ischemia model. The halothane anesthesia was stopped immediately after the ligation, and then the time taken to recover the righting reflex, the onset time of convulsions and the survival time were determined. The test solution was prepared in the same manner as described in Experiment 1 and was administered to the animal intraperitoneally 30 minutes before the ligation. A 0.5% CMC solution was

administered to the control group in the same manner. The statistical analysis was carried out in a similar manner to that in Experiment 1. The statistical analysis of the survival rate was carried out by the $\chi^2$ test.

The results are shown in Table 9.

Table 9

| Cpd. of Ex. No. | Dose (mg/kg) | No. & (age) of animals | Recovery time of righting reflex (min.) | Onset time of convulsions (min.) | Survival time (min.) | Survival rate (%) |
|---|---|---|---|---|---|---|
| Cont | - | 10 (13) | 9.9 ±2.7 | 97.5 ±20.7 | 200.6 ±35.2 | 0.0 |
| 4 | 100 | 9 (13) | 10.1 ±1.8 | 97.6 ±18.0 | 322.4 ±34.8** | 44.4 |
| Cont | - | 10 (13) | 9.1 ±3.2 | 59.7 ±7.0 | 273.5 ±45.0 | 0.0 |
| 3 | 30 | 10 (13) | 7.1 ±0.9 | 106.5 ±9.9*** | 392.2 ±21.2 | 80.0 |
| 3 | 100 | 10 (13) | 36.3 ±16.7* | 306.9 ±17.1*** | 420.0 ±0.0* | 100** |
| Cont | - | 11 (15) | 8.7 ±1.8 | 48.3 ±6.6 | 225.6 ±34.2 | 18.2 |
| 20 | 30 | 11 (15) | 6.5 ±0.6 | 71.9 ±9.3* | 295.9 ±32.3 | 36.4 |
| 20 | 100 | 11 (15) | 6.9 ±0.9 | 176.7 ±25.8** | 371.9 ±26.3*** | 63.6# |

# = p <0.1.

* = p <0.05.

** = p <0.02.

*** = p <0.01.

## EXPERIMENT 4

### Effect on survival time under hypoxic conditions

The test animals employed were male adult mice (5 weeks old and about 30 g body weight) of the ddy strain. The animals were employed in groups, each containing from 7 to 17 animals. Each animal was given intraperitoneally a test compound in suspension (as in the preceding Experiments). 30 minutes after this administration, 2 or 3 animals were placed in a 1.5 liter gas chamber made of acrylate resin, and a mixture of 4% by volume oxygen and 96% nitrogen was supplied at the rate of 10 liters/minute. The time of death of the animal was measured (determined by the cessation of respiration).

The results are shown in the following Table 10.

Table 10

| Cpd. of Ex. No. | Dose (mg/kg) | No. of test animals | Survival time (seconds) |
|---|---|---|---|
| Cont. | - | 11 | 115.5 ± 8.3 |
| 3 | 30 | 7 | 213.0 ± 27.5** |
| 3 | 100 | 7 | 446.7 ± 36.9**** |
| Cont. | - | 17 | 92.2 ± 5.3 |
| 20 | 30 | 14 | 124.6 ± 15.4*** |
| 20 | 100 | 9 | 349.5 ± 59.1*** |

** = p <0.02.
*** = p <0.01.
**** = p <0.001.

As can be seen from the above results, the compounds of the present invention were found to improve significantly the survival time of the test animals under hypoxic conditions.

## EXPERIMENT 5

### Acute toxicity

Each of the test compounds used in the above Experiments 1 to 4 was suspended in a 0.5% w/v CMC

aqueous solution, and 1000 mg/kg of the compound was orally administered to each of 3 male adult mice of the ddy strain (weighting 20 to 25 g each) which were then observed for 5 days. In some cases, systemic hypotonia attributed to the effect of the drug was observed for about 3 hours after administration, but all of the mice survived.

## Claims

### Claims for the following Contracting States : AT, BE, CH, DE, FR, GB, IT, LI, LU, NL, SE

1. Compounds of formula (I) :

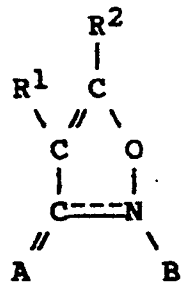

(I)

in which : either

(a) the dotted line (- - -) represents a single bond ;

A = represents a oxygen atom ; and
B represents a group of formula (II) :

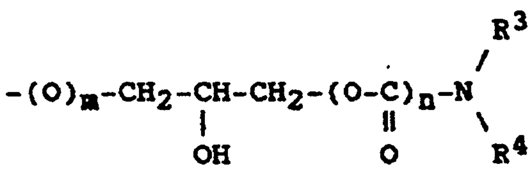

(II)

in which m is 0 and n is 0 or 1 ; or
(b) the dotted line (- - -) represents a double bond ;

A = represents said group of formula (II) in which m is 1 and n is 0 or 1 ; and
B is absent ; and

$R^1$ represents a hydrogen atom, a halogen atom, a $C_1$-$C_4$ alkyl group, a $C_2$-$C_4$ alkenyl group, a $C_2$-$C_4$ alkynyl group, a benzyl group, a substituted benzyl group having at least one of substituents (a), defined below, a phenyl group or a substituted phenyl group having at least one of substituents (a), defined below ;
$R^2$ represents a hydrogen atom, a halogen atom, a $C_1$-$C_4$ alkyl group, a phenyl group, a substituted phenyl group having at least one of substituents (a), defined below, or a heterocyclic group having 5 or 6 ring atoms, of which from 1 to 3 are nitrogen and/or oxygen and/or sulphur hetero-atoms, said heterocyclic group being unsubstituted or having at least one of substituents (a), defined below ; or $R^1$ and $R^2$, together with the carbon atoms to which they are attached, represent a hydrocarbon ring fused to the isoxazole ring and having, in total, from 5 to 7 ring carbon atoms, said hydrocarbon ring being unsubstituted or having at least one of substituents (b), defined below ;
$R^3$ and $R^4$ are the same or different and each represents a hydrogen atom, a $C_1$-$C_4$ alkyl group, a benzyl group, a substituted benzyl group having at least one of substituents (a), defined below, a phenyl group or a subtituted phenyl group having at least one of substituents (a), defined below ; or $R^3$, $R^4$ and the nitrogen atom to which they are attached together represent an alicyclic amino group

having a total of 5 or 6 ring atoms, of which one is said nitrogen atom and 0 or 1 is an additional nitrogen and/or oxygen and/or sulphur hetero-atom, said alicyclic amino group being unsubstituted or, where there is an additional nitrogen hetero-atom, having at least one of substituents (b), defined below, on that additional nitrogen hetero-atom ;

substituents (a) :

$C_1$-$C_3$ alkyl groups, $C_1$-$C_3$ alkoxy groups, hydroxy groups, halogen atoms, nitro groups, amino groups and $C_2$-$C_4$ aliphatic carboxylic acyl groups ;

substituents (b) :

$C_1$-$C_3$ alkyl groups, $C_1$-$C_3$alkoxy groups, hydroxy groups, halogen atoms, nitro groups, amino groups, $C_2$-$C_4$ aliphatic carboxylic acylamino groups, benzyl groups, substituted benzyl groups having at least one of substituents (a), defined above, phenyl groups and substituted phenyl groups having at least one of substituents (a), defined above ;

provided that :

(i) where B represents amid group of formula (II) and n is 0, then $R^1$ does not represent a hydrogen or halogen atom ; and

(ii) where A represents said group of formula (II) and n is 0, $R^1$ and $R^2$ together represent a benzene ring fused to the isoxazole ring and $R^4$ represents an alkyl group, then $R^3$ does not represent a hydrogen atom;

(iii) when (- - -) represents a single bond, $R^1$ and $R^2$, together with the carbon atoms to which they are attached, form a $C_{6-7}$ hydrocarbon ring, then n = 1 ;

and pharmaceutically acceptable salts thereof ; but

excluding

the compound 5-chloro-2-(3-piperidino-2-hydroxy)-propylbenzisoxazolin-3-one.

2. Compounds according to Claim 1, represented by the formula (Ia) :

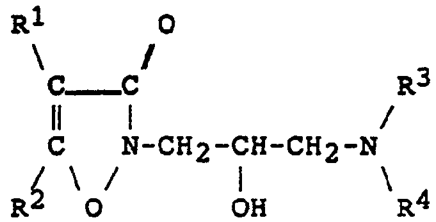

(Ia)

in which :

$R^1$ represents a $C_1$-$C_4$ alkyl group, a $C_2$-$C_4$ alkenyl group, a $C_2$-$C_4$ alkynyl group, a benzyl group, a substituted benzyl group, a phenyl group or a substituted phenyl group, the substituents being selected from substituents (a), defined in Claim 1 ;

$R^2$ represents hydrogen atom, a $C_1$-$C_4$ alkyl group, a phenyl group, a substituted phenyl group, or a heterocyclic group as defined in Claim 1 ;

$R^3$ and $R^4$ are the same or different and each represents a hydrogen atom, a $C_1$-$C_4$ alkyl group, a benzyl group, a substituted benzyl group, a phenyl group or a substituted phenyl group, or $R^3$ and $R^4$ may be taken together with the nitrogen atom to which they are attached to form an alicyclic amino group, as defined in Claim 1 ;

and pharmaceutically acceptable salts thereof.

3. Compounds according to Claim 1, represented by the formula (Ib) :

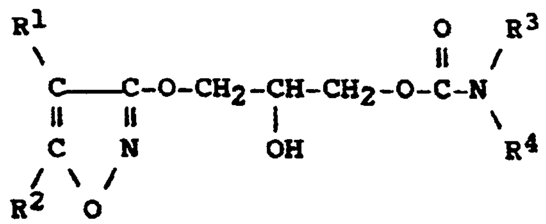

(Ib)

in which :

$R^1$ represents a hydrogen atom, a halogen atom, a $C_1$-$C_4$alkyl group, a $C_2$-$C_4$ alkenyl group, a $C_2$-$C_4$ alkynyl group, a benzyl group, a substituted benzyl group having at least one of substituents (a), defined in Claim 1, a phenyl group or a substituted phenyl group having at least one of substituents (a), defined in Claim 1 ;

$R^2$ represents a hydrogen atom, a $C_1$-$C_4$ alkyl group, a phenyl group, a substituted phenyl group having at least

one of substituents (a), defined in Claim 1, or a heterocyclic group as defined in Claim 1 ; or
$R^1$ and $R^2$, together with the carbon atoms to which they are attached, represent a hydrocarbon ring fused to the isoxazole ring and as defined in Claim 1 ;
$R^3$ and $R^4$ are the same or different and each represents a hydrogen atom, a $C_1$-$C_4$ alkyl group, a benzyl group, a substituted benzyl group having at least one of substituents (a), defined in Claim 1, a phenyl group of a substituted phenyl group having at least one of substituents (a), defined in Claim 1 ; or
$R^3$, $R^4$ and the nitrogen atom to which they are attached together represent an alicyclic amino group as defined in Claim 1 ;
and pharmaceutically acceptable salts thereof.

4. Compounds according to Claim 1, represented by the formula (Ic) :

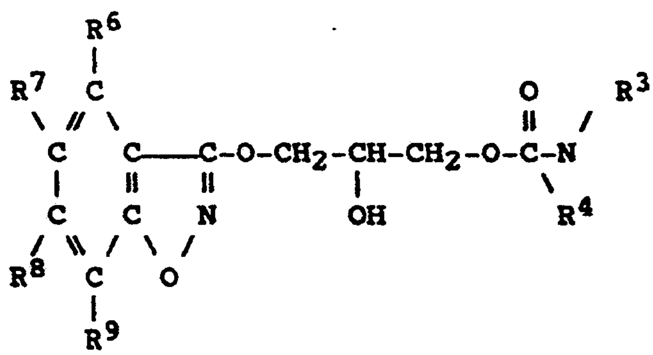

(Ic)

in which :
$R^3$ and $R^4$ are the same or different and each represents a hydrogen atom, a $C_1$-$C_4$ alkyl group, a benzyl group, a substituted benzyl group having at least one of substituents (a), defined in Claim 1, a phenyl group or a substituted phenyl group having at least one of substituents (a), defined in Claim 1 ; or
$R^3$, $R^4$ and the nitrogen atom to which they are attached together represent an alicyclic amino group as defined in Claim 1 ; and
one of $R^6$, $R^7$, $R^8$ and $R^9$ represents a hydrogen atom, a halogen atom, a $C_1$-$C_4$ alkyl group or a $C_1$-$C_4$ alkoxy group, and the others all represent hydrogen atoms ;
and pharmaceutically acceptable salts thereof.

5. Compounds according to Claim 1, represented by the formula (Id) :

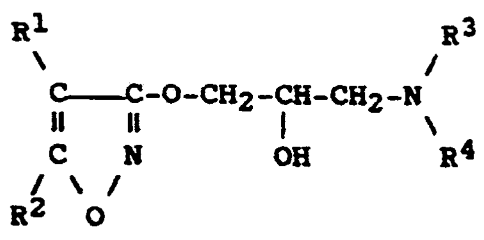

(Id)

in which :
$R^1$ represents a hydrogen atom, a halogen atom, a $C_1$-$C_4$ alkyl group, a $C_2$-$C_4$ alkenyl group, a $C_2$-$C_4$ alkynyl group, a benzyl group, a substituted benzyl group having at least one of substituents (a), defined in Claim 1, a phenyl group or a substituted phenyl group having at least one of substituents (a), defined in Claim 1 ;
$R^2$ represents a hydrogen atom, a $C_1$-$C_4$ alkyl group, a phenyl group, a substituted phenyl group having at least one of substituents (a), defined in Claim 1, or a heterocyclic group as defined in Claim 1 ; or
$R^1$ and $R^2$, together with the carbon atoms to which they are attached, represent a hydrocarbon ring fused to the isoxazole ring and as defined in Claim 1 ;
$R^3$ and $R^4$ are the same or different and each represents a hydrogen atom, a $C_1$-$C_4$ alkyl group, a benzyl group, a substituted benzyl group having at least one of substituents (a), defined in Claim 1, a phenyl group or a substituted phenyl groups having at least one of substituents (a), defined in Claim 1 ; or
$R^3$, $R^4$ and the nitrogen atom to which they are attached together represent an alicyclic amino group as defined

in Claim 1 ;
provided that, where $R^1$ and $R^2$ together represent a benzene ring fused to the isoxazole ring and $R^4$ represents an alkyl group, then $R^3$ does not represent a hydrogen atom ;
and pharmaceutically acceptable salts thereof.

6. Compounds according to Claim 1, represented by the formula (Ie) :

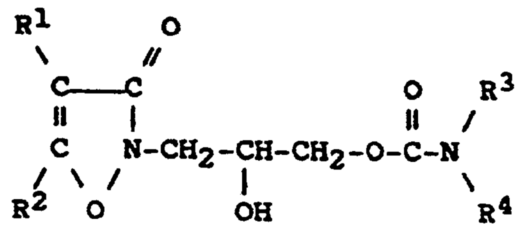
(Ie)

in which :
$R^1$ represents a hydrogen atom, a halogen atom, a $C_1$-$C_4$ alkyl group, a $C_2$-$C_4$ alkenyl group, a $C_2$-$C_4$ alkynyl group, a benzyl group, a substituted benzyl group having at least one of substituents (a), defined in Claim 1, a phenyl group or a substituted phenyl group having at least one of substituents (a), defined in Claim 1 ;
$R^2$ represents a hydrogen atom, a $C_1$-$C_4$ alkyl group, a phenyl group, a substituted phenyl group having at least one of substituents (a), defined in Claim 1, or a heterocyclic group as defined in Claim 1 ; or
$R^1$ and $R^2$, together with the carbon atoms to which they are attached, represent a hydrocarbon ring fused to the isoxazole ring and as defined in Claim 1 ;
$R^3$ and $R^4$ are the same or different and each represents a hydrogen atom, a $C_1$-$C_4$ alkyl group, a benzyl group, a substituted benzyl group having at least one of substituents (a), defined in Claim 1, a phenyl group or a substituted phenyl group having at least one of substituents (a), defined in Claim 1 ; or
$R^3$, $R^4$ and the nitrogen atom to which they are attached together represent an alicyclic amino group as defined in Claim 1 ;
and pharmaceutically acceptable salts thereof.

7. Compounds according to Claim 1, represented by the formula (If) :

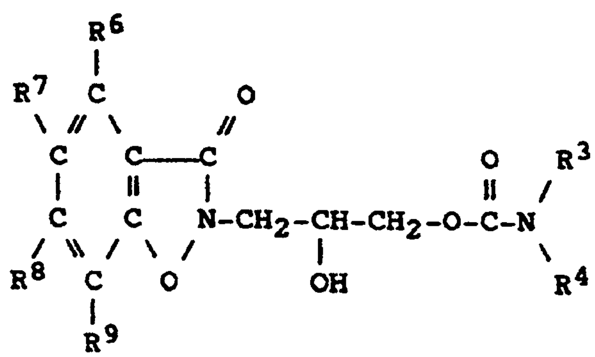
(If)

in which :
$R^3$ and $R^4$ are the same or different and each represents a hydrogen atom, a $C_1$-$C_4$ alkyl group, a benzyl group, a substituted benzyl group having at least one of substituents (a), defined in Claim 1, a phenyl group or a substituted phenyl group having at least one of substituents (a), defined in Claim 1 ; or
$R^3$, $R^4$ and the nitrogen atom to which they are attached together represent an alicyclic amino group as defined in Claim 1 ; and
one of $R^6$, $R^7$, $R^8$ and $R^9$ represents a hydrogen atom, a halogen atom, a $C_1$-$C_4$ alkyl group or a $C_1$-$C_4$ alkoxy group, and the others all represent hydrogen atoms ;
and pharmaceutically acceptable salts thereof.

8. Compounds according to Claim 1, wherein $R^1$ represents a hydrogen atom, a halogen atom or a $C_1$-$C_4$ alkyl group.

9. Compounds according to Claim 1, wherein : $R^2$ represents a phenyl group or a substituted phenyl group

having at least one of substituents (c), defined below ;

substituents (c) :

$C_1$-$C_3$ alkoxy groups, hydroxy groups and halogen atoms.

10. Compounds according to Claim 1, wherein : $R^1$ and $R^2$, together with the carbon atoms to which they are attached, represent a 6-membered hydrocarbon ring fused to the isoxazole ring and being unsubstituted or having at least one of halogen atoms.

11. Compounds according to Claim 1, wherein : $R^3$ and $R^4$ are the same or different and each represents a hydrogen atom, a $C_1$-$C_4$ alkyl group, a benzyl group or a phenyl group.

12. Compounds according to Claim 1, wherein : $R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substitutent on that additional nitrogen hetero-atom selected from substituents (d), defined below ;

substituents (d) :

$C_1$-$C_3$ alkyl groups, benzyl groups and phenyl groups.

13. Compounds according to Claim 1, wherein : $R^1$ represents a hydrogen atom, a chlorine atom or a $C_1$ or $C_2$ alkyl group.

14. Compounds according to Claim 1, wherein : $R^2$ represents a phenyl group or a substituted phenyl group having at least one of substituents (c'), defined below ;

substituents (c') :

methoxy groups and halogen atoms.

15. Compounds according to Claim 1, wherein : $R^1$ and $R^2$, together with the carbon atoms to which they are attached, represent a benzene ring fused to the isoxazole ring and being unsubstituted or having one substituent selected from halogen atoms.

16. Compounds according to Claim 1, wherein : $R^3$ and $R^4$ are the same or different and each represents a hydrogen atom, a methyl group or an ethyl group.

17. Compounds according to Claim 1, wherein : $R^3$, $R^4$ and the nitrogen atom to which they are attached together represent a morpholino group, a 1-piperazinyl group, a 4-methyl-1-piperazinyl group, a 1-pyrrolidinyl group or a piperidino group.

18. Compounds according to Claim 1, wherein :

$R^1$ represents a hydrogen atom, a halogen atom or a $C_1$-$C_4$ alkyl group ;

$R^2$ represents a phenyl group or a substituted phenyl group having at least one of substituents (c), defined below ;

substituents (c) :

$C_1$-$C_3$ alkoxy groups, hydroxy groups and halogen atoms ;

$R^3$ and $R^4$ are the same or different and each represents a hydrogen atom, a $C_1$-$C_4$ alkyl group, a benzyl group or a phenyl group ; or

$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

substituents (d) :

$C_1$-$C_3$ alkyl groups, benzyl groups and phenyl groups.

19. Compounds according to Claim 1, wherein :

$R^1$ and $R^2$, together with the carbon atoms to which they are attached, represent a 6-membered hydrocarbon ring fused to the isoxazole ring and being unsubstituted or having at least one of halogen atoms ;

$R^3$ and $R^4$ are the same or different and each represents a hydrogen atom, a $C_1$-$C_4$ alkyl group, a benzyl group or a phenyl group ; or

$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

substituents (d) :

$C_1$-$C_3$ alkyl groups, benzyl groups and phenyl groups.

20. Compounds according to Claim 1, wherein :

$R^1$ represents a hydrogen atom, a chlorine atom or a $C_1$ or $C_2$ alkyl group ;

$R^2$ represents a phenyl group or a substituted phenyl group having at least one of substituents (c'), defined below ;

substituents (c'):
methoxy groups and halogen atoms ;
$R^3$ and $R^4$ are the same or different and each represents a hydrogen atom, a methyl group or an ethyl group ; or
$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

21. Compounds according to Claim 1, wherein :
$R^1$ and $R^2$, together with the carbon atoms to which they are attached, represent a benzene ring fused to the isoxazole ring and being unsubstituted or having one of halogen atoms ;
$R^3$ and $R^4$ are the same or different and each represents a hydrogen atom, a methyl group or an ethyl group ; or
$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

22. Compounds according to Claim 3, represented by the formula (Ib) :

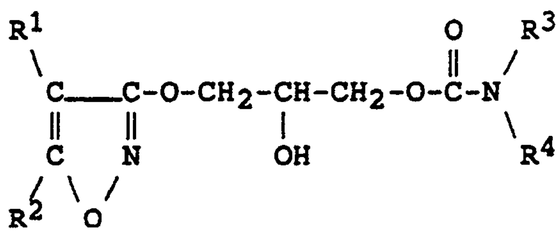

(Ib)

in which :
$R^1$ represents a hydrogen atom, a halogen atom or a $C_1$-$C_4$ alkyl group ;
$R^2$ represents a phenyl group or a substituted phenyl group having at least one of substituents (c), defined below ;
substituents (c):
$C_1$-$C_3$ alkoxy groups, hydroxy groups and halogen atoms ; or
$R^1$ and $R^2$, together with the carbon atoms to which they are attached, represent a 6-membered hydrocarbon ring fused to the isoxazole ring and being unsubstituted or having at least one of halogen atoms ; and
$R^3$ and $R^4$ are the same or different and each represents a hydrogen atom, a $C_1$-$C_4$ alkyl group, a benzyl group or a phenyl group ; or
$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;
substituents (d):
$C_1$-$C_3$ alkyl groups, benzyl groups and phenyl groups.

23. Compounds according to Claim 22, represented by the formula (Ib) :

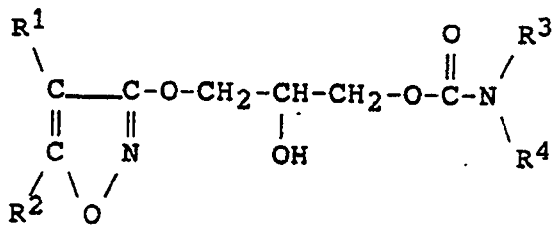

(Ib)

in which
$R^1$ represents a hydrogen atom, a chlorine atom or a $C_1$ or $C_2$ alkyl group ;
$R^2$ represents a phenyl group or a substituted phenyl group having at least one of substituents (c'), defined below ; or
$R^1$ and $R^2$, together with the carbon atoms to which they are attached, represent a benzene ring fused to the isoxazole ring and being unsubstituted or having one of halogen atoms ;
substituents (c'):
methoxy groups and halogen atoms ; and
$R^3$ and $R^4$ are the same or different and each represents a hydrogen atoms, methyl groups and ethyl groups ;

or
$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

24. 2-(2-Hydroxy-3-morpholinopropyl)-4-methyl-5-phenyl-3-isoxoazolone and pharmaceutically acceptable salts thereof.

25. 3-(3-Carbamoyloxy-2-hydroxypropoxy)-5-(m-chlorophenyl)isoxazole and pharmaceutically acceptable salts thereof.

26. 3-(2-Hydroxy-3-morpholinopropoxy)-5-phenylisoxazole and pharmaceutically acceptable salts thereof.

27. 3-(2-Hydroxy-3-morpholinopropoxy)-4-methyl-5-phenylisoxazole and pharmaceutically acceptable salts thereof.

28. 3-(3-Carbamoyloxy-2-hydroxypropoxy)-5-chloro-1,2-benzisoxazole and pharmaceutically acceptable salts thereof.

29. The use for the manufacture of a medicament for treating a cerebrovascular disorder of at least one cerebro-active drug, wherein the cerebro-active drug is a compound of formula (I) :

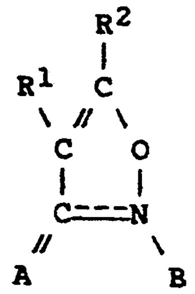

(I)

in which : either

(a) the dotted line (- - -) represents a single bond ;

A = represents a oxygen atom ; and
B represents a group of formula (II) :

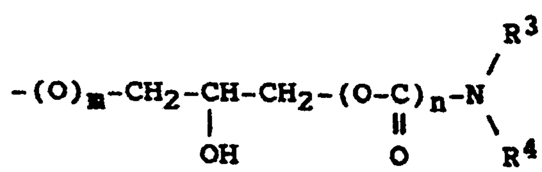

(II)

in which m is 0 and n is 0 or 1 ; or

(b) the dotted line (- - -) represents a double bond ;

A = represents said group of formula (II) in which m is 1 and n is 0 or 1 ; and
B is absent ; and

$R^1$ represents a hydrogen atom, a halogen atom, a $C_1$-$C_4$ alkyl group, a $C_2$-$C_4$ alkenyl group, a $C_2$-$C_4$ alkynyl group, a benzyl group, a substituted benzyl group having at least one of substituents (a), defined below, a phenyl group or a substituted phenyl group having at least one of substituents (a), defined below ;
$R^2$ represents a hydrogen atom, a halogen atom, a $C_1$-$C_4$ alkyl group, a phenyl group, a substituted phenyl group having at least one of substituents (a), defined below, or a heterocyclic group having 5 or 6 ring atoms, of which from 1 to 3 are nitrogen and/or oxygen and/or sulphur hetero-atoms, said heterocyclic group being unsubstituted or having at least one of substituents (a), defined below ; or
$R^1$ and $R^2$, together with the carbon atoms to which they are attached, represent a hydrocarbon ring fused to the isoxazole ring and having, in total, from 5 to 7 ring carbon atoms, said hydrocarbon ring

being unsubstituted or having at least one of substituents (b), defined below ;

$R^3$ and $R^4$ are the same or different and each represents a hydrogen atom, a $C_1$-$C_4$ alkyl group, a benzyl group, a substituted benzyl group having at least one of substituents (a), defined below, a phenyl group or a substituted phenyl group having at least one of substituents (a), defined below ;

or

$R^3$, $R^4$ and the nitrogen atom to which they are attached together represent an alicyclic amino group having a total of 5 or 6 ring atoms, of which one is said nitrogen atom and 0 or 1 is an additional nitrogen and/or oxygen and/or sulphur hetero-atom, said alicyclic amino group being unsubstituted or, where there is an additional nitrogen hetero-atom, having at least one of substituents (b), defined below, on that additional nitrogen hetero-atom ;

substituents(a) :

$C_1$-$C_3$ alkyl groups, $C_1$-$C_3$ alkoxy groups, hydroxy groups, halogen atoms, nitro groups, amino groups and $C_2$-$C_4$ aliphatic carboxylic acyl groups ;

substituents (b) :

$C_1$-$C_3$ alkyl groups, $C_1$-$C_3$ alkoxy groups, hydroxy groups, halogen atoms, nitro groups, amino groups, $C_2$-$C_4$ aliphatic carboxylic acylamino groups, benzyl groups, substituted benzyl groups having at least one of substituents (a), defined above, phenyl groups and substituted phenyl groups having at least one of substituents (a), defined above ;

provided that :

where B represents said group of formula (II) and n is 0, then $R^1$ does not represent a hydrogen or halogen atom ;

and pharmaceutically acceptable salts thereof.

30. The use according to Claim 29, wherein said cerebro-active drug is represented by the formula (Ib) :

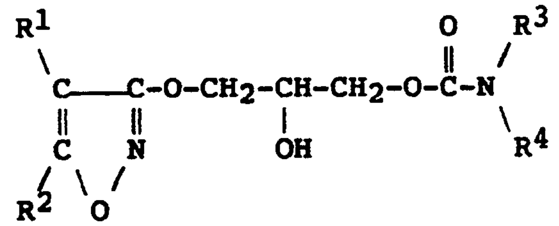

(Ib)

in which :

$R^1$ represents a hydrogen atom, a halogen atom or a $C_1$-$C_4$ alkyl group ;

$R^2$ represents a phenyl group or a phenyl group having at least one of substituents (c), defined below ;

substituents (c) :

$C_1$- $C_3$ alkoxy groups, hydroxy groups and halogen atoms ; or

$R^1$ and $R^2$, together with the carbon atoms to which they are attached, represent a 6-membered hydrocarbon ring fused to the isoxazole ring and being unsubstituted or having at least one of halogen atoms ; and

$R^3$ and $R^4$ are the same or different and each represents a hydrogen atom, a $C_1$-$C_4$ alkyl group, a benzyl group or a phenyl group ; or

$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

substituents (d) :

$C_1$-$C_3$ alkyl groups, benzyl groups and phenyl groups.

31. The use according to Claim 29, wherein said cerebro-active drug is represented by the formula (Ib) :

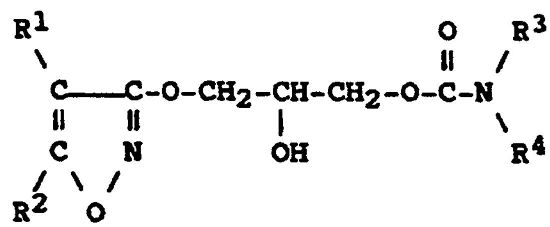

(Ib)

in which :
$R^1$ represents a hydrogen atom, a chlorine atom or a $C_1$ or $C_2$ alkyl group ;
$R^2$ represents a phenyl group or a phenyl group having at least one of substituents (c'), defined below ; or
$R^1$ and $R^2$, together with the carbon atoms to which they are attached, represent a benzene ring fused to the isoxazole ring and being unsubstituted or having one of halogen atoms ; and
substituents (c') :
methoxy groups and halogen atoms ;
$R^3$ and $R^4$ are the same or different and each represents a hydrogen atom, a methyl group or an ethyl group ; or
$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

32. The use according to Claim 29, wherein said cerebro-active drug is at least one of the compounds :
2-(2-hydroxy-3-morpholinopropyl)-4-methyl-5-phenyl-3-isoxazolone ;
3-(3-carbamoyloxy-2-hydroxypropoxy)-5-(m-chlorophenyl)isoxazole ;
3-(2-hydroxy-3-morpholinopropoxy)-5-phenylisoxazole ;
3-(2-hydroxy-3-morpholinopropoxy)-4-methyl-5-phenylisoxazole ;
3-(3-carbamoyloxy-2-hydroxypropoxy)-5-chloro-1,2-benzisoxazole ;
and pharmaceutically acceptable salts thereof.

33. The use for the manufacture of a medicament for effecting centrally-acting muscle relaxant activity of an active compound, wherein the active compound is a compound of formula (I) or a pharmaceutically acceptable salt thereof, as claimed in Claim 29.

34. The use according to Claim 33, wherein said active compound is represented by the formula (Ib) :

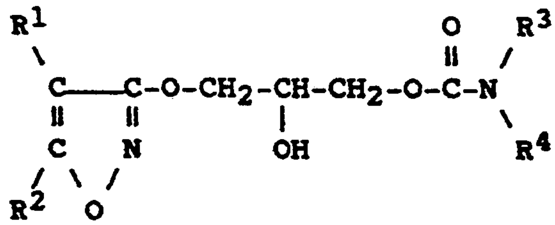

(Ib)

in which :
$R^1$ represents a hydrogen atom, a halogen atom or a $C_1$-$C_4$ alkyl group ;
$R^2$ represents a phenyl group or a phenyl group having at least one of substituents (c), defined below ;
substituents (c) :
$C_1$-$C_3$ alkoxy groups, hydroxy groups and halogen atoms ; or
$R^1$ and $R^2$, together with the carbon atoms to which they are attached, represent a 6-membered hydrocarbon ring fused to the isoxazole ring and being unsubstituted or having at least one of halogen atoms ; and
$R^3$ and $R^4$ are the same or different and each represents a hydrogen atom, a $C_1$-$C_4$ alkyl group, a benzyl group or a phenyl group ; or
$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or,where there is an additional nitrogen hetero-atom, having at least one substituent on that additional nitrogen hetero-atom selected from substituents (d), defined below ;
substituents (d) :
$C_1$-$C_3$ alkyl groups, benzyl groups and phenyl groups.

35. The use according to Claim 33, wherein said active compound is represented by the formula (Ib) :

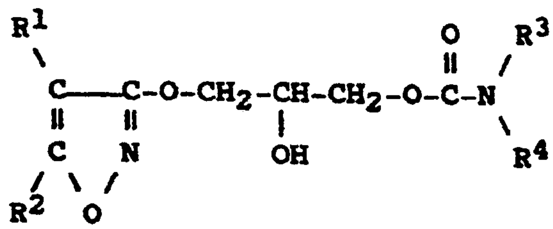

(Ib)

in which :

$R^1$ represents a hydrogen atom, a chlorine atom or a $C_1$ or $C_2$ alkyl group ;
$R^2$ represents a phenyl group or a phenyl group having at least one of substituents (c′), defined below ; or
$R^1$ and $R^2$, together with the carbon atoms to which they are attached, represent a benzene ring fused to the isoxazole ring and being unsubstituted or having one of halogen atoms ; and

substituents (c′) :
methoxy groups and halogen atoms ;

$R^3$ and $R^4$ are the same or different and each represents a hydrogen atom, a methyl group or an ethyl group ; or
$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

36. The use according to Claim 33, wherein said active compound is at least one of the compounds :
2-(2-hydroxy-3-morpholinopropyl)-4-methyl-5-phenyl-3-isoxazolone ;
3-(3-carbamoyloxy-2-hydroxypropoxy)-5-(m-chlorophenyl)isoxazole ;
3-(2-hydroxy-3-morpholinopropoxy)-5-phenylisoxazole ;
3-(2-hydroxy-3-morpholinopropoxy)-4-methyl-5-phenylisoxazole ;
3-(3-carbamoyloxy-2-hydroxypropoxy)-5-chloro-1,2-benzisoxazole ;
or a pharmaceutically acceptable salt thereof.

37. A pharmaceutical composition for the treatment of cerebrovascular disorders or for use as a centrally-acting muscle relaxant, which composition comprises an active compound in admixture with a pharmaceutically acceptable carrier or diluent, wherein the active compound is a compound of formula (I) or a pharmaceutically acceptable salt thereof, as defined in Claim 29.

38. A composition according to Claim 37, wherein said active compound is represented by the formula (Ib):

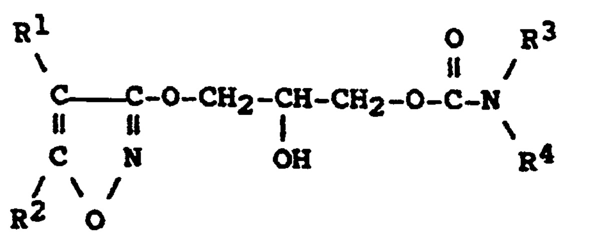

(Ib)

in which :
$R^1$ represents a hydrogen atom, a halogen atom or a $C_1$-$C_4$ alkyl group ;
$R^2$ represents a phenyl group or a substituted phenyl group having at least one of substituents (c), defined below ;

substituents (c) :
$C_1$-$C_3$ alkoxy groups, hydroxy groups and halogen atoms ; or

$R^1$ and $R^2$, together with the carbon atoms to which they are attached, represent a 6-membered hydrocarbon ring fused to the isoxazole ring and being unsubstituted or having at least one of halogen atoms ; and
$R^3$ and $R^4$ are the same or different and each represents a hydrogen atom, a $C_1$-$C_4$ alkyl group, a benzyl group or a phenyl group ; or
$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

substituents (d) :
$C_1$-$C_3$ alkyl groups, benzyl groups and phenyl groups.

39. A composition according to Claim 37, wherein said active compound is represented by the formula (Ib):

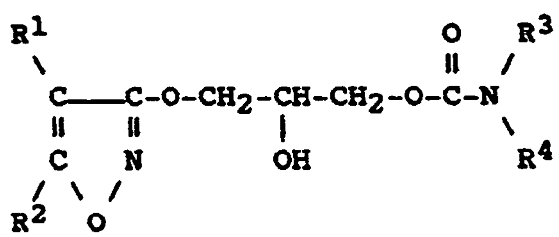

(Ib)

in which :
$R^1$ represents a hydrogen atom, a chlorine atom or a $C_1$ or $C_2$ alkyl group ;
$R^2$ represents a phenyl group or a phenyl group having at least one of substituents (c′), defined below ; or
$R^1$ and $R^2$, together with the carbon atoms to which they are attached, represent a benzene ring fused to the isoxazole ring and being unsubstituted or having one of halogen atoms ; and

substituents (c′) :
methoxy groups and halogen atoms ;

$R^3$ and $R^4$ are the same or different and each represents a hydrogen atom, a methyl group or an ethyl group ; or
$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

40. A composition according to Claim 37, wherein said active compound is at least one of the compounds:
2-(2-hydroxy-3-morpholinopropyl)-4-methyl-5-phenyl-3-isoxazolone ;
3-(3-carbamoyloxy-2-hydroxypropoxy)-5-(m-chlorophenyl)isoxazole ;
3-(2-hydroxy-3-morpholinopropoxy)-5-phenylisoxazole ;
3-(2-hydroxy-3-morpholinopropoxy)-4-methyl-5-phenylisoxazole ;
3-(3-carbamoyloxy-2-hydroxypropoxy)-5-chloro-1,2-benzisoxazole ;
and pharmaceutically acceptable salts thereof.

41. A process for preparing a compound according to any one of Claims 1 to 28, which comprises the steps:
(i) reacting a compound of formula (III) :

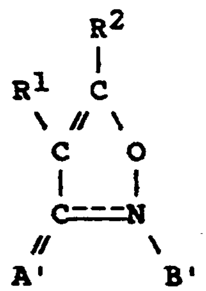

(III)

[in which : either
(a) the dotted line (- - -) represents a single bond ;

A′ = represents a oxygen atom ; and
B′ represents a group of formula (IV) :

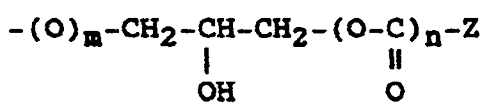

(IV)

in which m is 0 and n is 0 or 1 ; or

(b) the dotted line (- - -) represents a double bond ;

A′ = represents said group of formula (IV) in which m is 1 and n is 0 or 1 ; and
B′ is absent ;
Z represents a leaving group ;
and $R^1$ and $R^2$ are as defined in Claim 1]
with an amine of formula (V) :

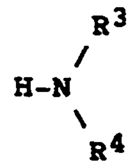

(V)

(in which $R^3$ and $R^4$ are as defined in Claim 1) ; and, if necessary, salifying the resulting product.

**Claims for the following Contracting States : ES, GR**

1. A process for preparing a compound of formula (I) :

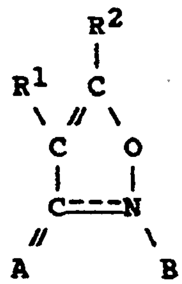

(I)

in which : either

(a) the dotted line (- - -) represents a single bond ;

A = represents a oxygen atom ; and

B represents a group of formula (II) :

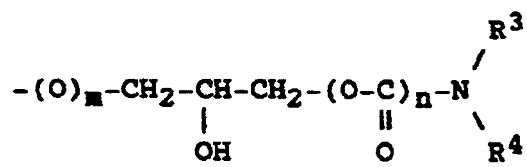

(II)

in which m is 0 and n is 0 or 1 ; or

(b) the dotted line (- - -) represents a double bond ;

A = represents said group of formula (II) in which m is 1 and n is 0 or 1 ; and

B is absent ; and

$R^1$ represents a hydrogen atom, a halogen atom, a $C_1$-$C_4$ alkyl group, a $C_2$-$C_4$ alkenyl group, a $C_2$-$C_4$ alkynyl group, a benzyl group, a substituted benzyl group having at least one of substituents (a), defined below, a phenyl group or a substituted phenyl group having at least one of substituents (a), defined below ;

$R^2$ represents a hydrogen atom, a halogen atom, a $C_1$-$C_4$ alkyl group, a phenyl group, a substituted phenyl group having at least one of substituents (a), defined below, or a heterocyclic group having 5 or 6 ring atoms, of which from 1 to 3 are nitrogen and/or oxygen and/or sulphur hetero-atoms, said heterocyclic group being unsubstituted or having at least one of substituents (a), defined below ; or

$R^1$ and $R^2$, together with the carbon atoms to which they are attached, represent a hydrocarbon ring fused to the isoxazole ring and having, in total, from 5 to 7 ring carbon atoms, said hydrocarbon ring being unsubstituted or having at least one of substituents (b), defined below ;

$R^3$ and $R^4$ are the same or different and each represents a hydrogen atom, a $C_1$-$C_4$ alkyl group, a benzyl group, a substituted benzyl group having at least one of substituents (a), defined below, a phenyl group or a substituted phenyl group having at least one of substituents (a), defined below ;

or

$R^3$, $R^4$ and the nitrogen atom to which they are attached together represent an alicyclic amino group having a total of 5 or 6 ring atoms, of which one is said nitrogen atom and 0 or 1 is an additional nitrogen and/or oxygen and/or sulphur hetero-atom, said alicyclic amino group being unsubstituted or, where there is an additional nitrogen hetero-atom, having at least one of substituents (b), defined below, on that additional nitrogen hetero-atom ;

substituents (a) :

$C_1$-$C_3$ alkyl groups, $C_1$-$C_3$ alkoxy groups, hydroxy groups, halogen atoms, nitro groups, amino groups and $C_2$-$C_4$ aliphatic carboxylic acyl groups ;

substituents (b) :

$C_1$-$C_3$ alkyl groups, $C_1$-$C_3$ alkoxy groups, hydroxy groups, halogen atoms, nitro groups, amino groups, $C_2$-$C_4$ aliphatic carboxylic acylamino groups, benzyl groups, substituted benzyl groups having at least one of substituents (a), defined above, phenyl groups and substituted phenyl groups having at least one of substituents (a), defined above ;

provided that :

(i) where B represents said group of formula (II) and n is 0, then $R^1$ does not represent a hydrogen or halogen atom ; and

(ii) where A represents said group of formula (II) and n is 0, $R^1$ and $R^2$ together represent a benzene ring fused to the isoxazole ring and $R^4$ represents an alkyl group, then $R^3$ does not represent a hydrogen atom;

(iii) when (- - -) represents a single bond, $R^1$ and $R^2$, together with the carbon atoms to which they are attached, form a $C_{6-7}$ hydrocarbon ring, then n = 1 ;

or a pharmaceutically acceptable salt thereof but

excluding : the compound 5-chloro-2-(3-piperidino-2-hydroxy)propylbenzisoxazolin-3-one, which process comprises the steps :

(i) reacting a compound of formula (III) :

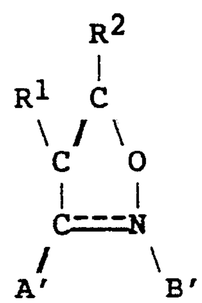

(III)

[in which : either

(a) the dotted line (- - -) represents a single bond ;

A' = represents a oxygen atom ; and

B' represents a group of formula (IV) :

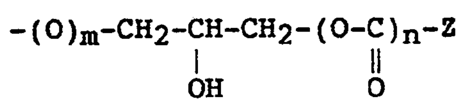

(IV)

in which m is 0 and n is 0 or 1 ; or

(b) the dotted line (- - -) represents a double bond ;

A' = represents said group of formula (IV) in which m is 1 and n is 0 or 1 ; and

B' is absent ;

Z represents a leaving group ;

and $R^1$ and $R^2$ are as defined above]

with an amine of formula (V) :

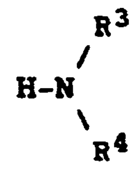

(V)

(in which $R^3$ and $R^4$ are as defined above) ;
and, if necessary, salifying the resulting product.

2. A process according to Claim 1, in which the reagents and reaction conditions are so chosen as to prepare a compound of formula (Ia) :

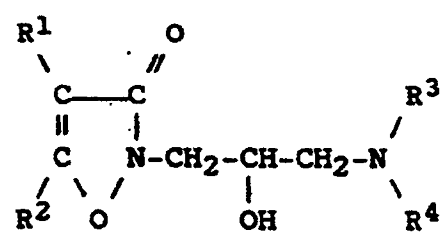

(Ia)

in which :
$R^1$ represents a $C_1$-$C_4$ alkyl group, a $C_2$-$C_4$ alkenyl group, a $C_2$-$C_4$ alkynyl group, a benzyl group, a substituted benzyl group, a phenyl group or a substituted phenyl group, the substituents being selected from substituents (a), defined in Claim 1 ;
$R^2$ represents hydrogen atom, a $C_1$-$C_4$ alkyl group, a phenyl group, a substituted phenyl group, or a heterocyclic group as defined in Claim 1 ;
$R^3$ and $R^4$ are the same or different and each represents a hydrogen atom, a $C_1$-$C_4$ alkyl group, a benzyl group, a substituted benzyl group, a phenyl group or a substituted phenyl group, or $R^3$ and $R^4$ may be taken together with the nitrogen atom to which they are attached to form an alicyclic amino group, as defined in Claim 1 ;
or a pharmaceutically acceptable salt thereof.

3. A process according to Claim 1, in which the reagents and reaction conditions are so chosen as to prepare a compound of formula (Ib) :

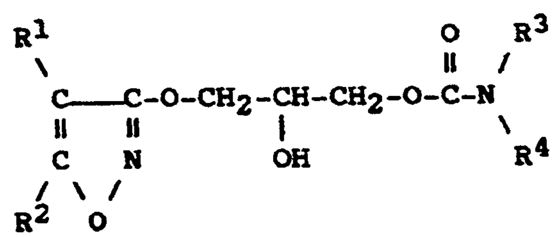

(Ib)

in which :
$R^1$ represents a hydrogen atom, a halogen atom, a $C_1$-$C_4$ alkyl group, a $C_2$-$C_4$ alkenyl group, a $C_2$-$C_4$ alkynyl group, a benzyl group, a substituted benzyl group having at least one of substituents (a), defined in Claim 1, a phenyl group or a substituted phenyl group having at least one of substituents (a), defined in Claim 1 ;
$R^2$ represents a hydrogen atom, a $C_1$-$C_4$ alkyl group, a phenyl group, a substituted phenyl group having at least one of substituents (a), defined in Claim 1, or a heterocyclic group as defined in Claim 1 ; or
$R^1$ and $R^2$, together with the carbon atoms to which they are attached, represent a hydrocarbon ring fused to the isoxazole ring and as defined in Claim 1 ;
$R^3$ and $R^4$ are the same or different and each represents a hydrogen atom, a $C_1$-$C_4$ alkyl group, a benzyl group, a substituted benzyl group having at least one of substituents (a), defined in Claim 1, a phenyl group or a substituted phenyl group having at least one of substituents (a), defined in Claim 1 ; or
$R^3$, $R^4$ and the nitrogen atom to which they are attached together represent an alicyclic amino group as defined in Claim 1 ;

or a pharmaceutically acceptable salt thereof.

4. A process according to Claim 1, in which the reagents and reaction conditions are so chosen as to prepare a compound of formula (Ic) :

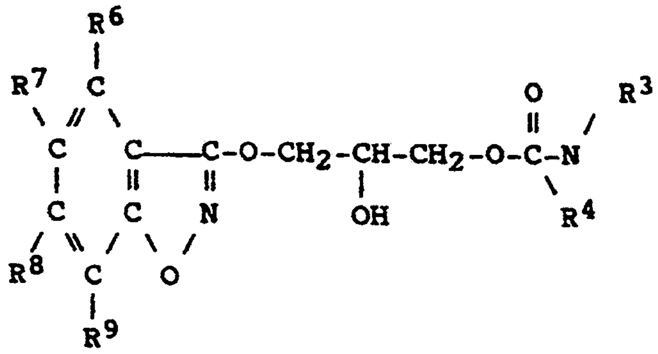

(Ic)

in which :

$R^3$ and $R^4$ are the same or different and each represents a hydrogen atom, a $C_1$-$C_4$ alkyl group, a benzyl group, a substituted benzyl group having at least one of substituents (a), defined in Claim 1, a phenyl group or a substituted phenyl group having at least one of substituents (a), defined in Claim 1 ; or

$R^3$, $R^4$ and the nitrogen atom to which they are attached together represent an alicyclic amino group as defined in Claim 1 ; and

one of $R^6$, $R^7$, $R^8$ and $R^9$ represents a hydrogen atom, a halogen atom, a $C_1$-$C_4$ alkyl group or a $C_1$-$C_4$ alkoxy group, and the others all represent hydrogen atoms ;

or a pharmaceutically acceptable salt thereof.

5. A process according to Claim 1, in which the reagents and reaction conditions are so chosen as to prepare a compound of formula (Id) :

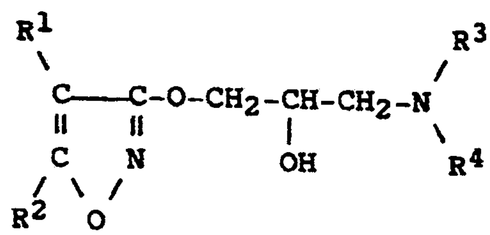

(Id)

in which :

$R^1$ represents a hydrogen atom, halogen atom, a $C_1$-$C_4$ alkyl group, a $C_2$-$C_4$ alkenyl group, a $C_2$-$C_4$ alkynyl group, a benzyl group, a substituted benzyl group having at least one of substituents (a), defined in Claim 1, a phenyl group or a substituted phenyl group having at least one of substituents (a), defined in Claim 1 ;

$R^2$ represents a hydrogen atom, a $C_1$-$C_4$ alkyl group, a phenyl group, a substituted phenyl group having at least one of substituents (a), defined in Claim 1, or a heterocyclic group as defined in Claim 1 ; or

$R^1$ and $R^2$, together with the carbon atoms to which they are attached, represent a hydrocarbon ring fused to the isoxazole ring and as defined in Claim 1 ;

$R^3$ and $R^4$ are the same or different and each represents a hydrogen atom, a $C_1$-$C_4$ alkyl group, a benzyl group, a substituted benzyl group having at least one of substituents (a), defined in Claim 1, a phenyl group or a substituted phenyl groups having at least one of substituents (a), defined in Claim 1 ; or

$R^3$, $R^4$ and the nitrogen atom to which they are attached together represent an alicyclic amino group as defined in Claim 1 ;

provided that, where $R^1$ and $R^2$ together represent a benzene ring fused to the isoxazole ring and $R^4$ represents an alkyl group, then $R^3$ does not represent a hydrogen atom ;

or a pharmaceutically acceptable salt thereof.

6. A process according to Claim 1, in which the reagents and reaction conditions are so chosen as to prepare a compound of formula (Ie) :

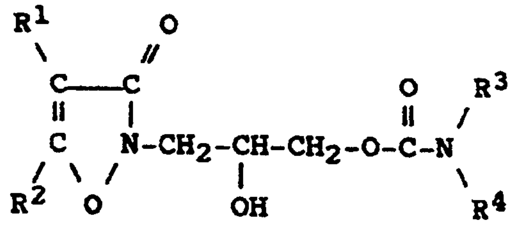

(Ie)

in which :

$R^1$ represents a hydrogen atom, a halogen atom, a $C_1$-$C_4$ alkyl group, a $C_2$-$C_4$ alkenyl group, a $C_2$ - $C_4$ alkynyl group, a benzyl group, a substituted benzyl group having at least one of substituents (a), defined in Claim 1, a phenyl group or a substituted phenyl group having at least one of substituents (a), defined in Claim 1 ;

$R^2$ represents a hydrogen atom, a $C_1$-$C_4$ alkyl group, a phenyl group, a substituted phenyl group having at least one of substituents (a), defined in Claim 1, or a heterocyclic group as defined in Claim 1 ; or

$R^1$ and $R^2$, together with the carbon atoms to which they are attached, represent a hydrocarbon ring fused to the isoxazole ring and as defined in Claim 1 ;

$R^3$ and $R^4$ are the same or different and each represents a hydrogen atom, a $C_1$-$C_4$ alkyl group, a benzyl group, a substituted benzyl group having at least one of substituents (a), defined in Claim 1, a phenyl group or a substituted phenyl group having at least one of substituents (a), defined in Claim 1 ; or

$R^3$, $R^4$ and the nitrogen atom to which they are attached together represent an alicyclic amino group as defined in Claim 1 ;

or a pharmaceutically acceptable salt thereof.

7. A process according to Claim 1, in which the reagents and reaction conditions are so chosen as to prepare a compound of formula (If) :

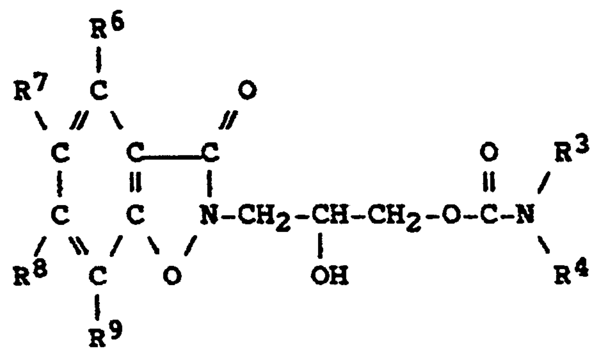

(If)

in which :

$R^3$ and $R^4$ are the same or different and each represents a hydrogen atom, a $C_1$-$C_4$ alkyl group, a benzyl group, a substituted benzyl group having at least one of substituents (a), defined in Claim 1, a phenyl group or a substituted phenyl group having at least one of substituents (a), defined in Claim 1 ; or

$R^3$, $R^4$ and the nitrogen atom to which they are attached together represent an alicyclic amino group as defined in Claim 1 ; and

one of $R^6$, $R^7$, $R^8$ and $R^9$ represents a hydrogen atom, a halogen atom, a $C_1$-$C_4$ alkyl group or a $C_1$-$C_4$ alkoxy group, and the others all represent hydrogen atoms ;

or a pharmaceutically acceptable salt thereof.

8. A process according to Claim 1, wherein the reagents and reaction conditions are so chosen as to prepare a compound of formula (I) or salt thereof, in which $R^1$ represents a hydrogen atom, a halogen atom or a $C_1$-$C_4$ alkyl group.

9. A process according to Claim 1, wherein the reagents and reaction conditions are so chosen as to prepare a compound of formula (I) or salt thereof, in which : $R^2$ represents a phenyl group or a substituted phenyl group having at least one of substituents (c), defined below ;

<u>substituents (c) :</u>

$C_1$-$C_3$ alkoxy groups, hydroxy groups and halogen atoms.

10. A process according to Claim 1, wherein the reagents and reaction conditions are so chosen as to prepare a compound of formula (I) or salt thereof, in which : $R^1$ and $R^2$, together with the carbon atoms to which they are attached, represent a 6-membered hydrocarbon ring fused to the isoxazole ring and being unsubstituted or having at least one of halogen atoms.

11. A process according to Claim 1, wherein the reagents and reaction conditions are so chosen as to prepare a compound of formula (I) or salt thereof, in which : $R^3$ and $R^4$, are the same or different and each represents a hydrogen atom, a $C_1$-$C_4$ alkyl group, a benzyl group or a phenyl group.

12. A process according to Claim 1, wherein the reagents and reaction conditions are so chosen as to prepare a compound of formula (I) or salt thereof, in which : $R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

substituents (d) :

$C_1$-$C_3$ alkyl groups, benzyl groups and phenyl groups.

13. A process according to Claim 1, wherein the reagents and reaction conditions are so chosen as to prepare a compound of formula (I) or salt thereof, in which : $R^1$ represents a hydrogen atom, a chlorine atom or a $C_1$ or $C_2$ alkyl group.

14. A process according to Claim 1, wherein the reagents and reaction conditions are so chosen as to prepare a compound of formula (I) or salt thereof, in which : $R^2$ represents a phenyl group or a substituted phenyl group having at least one of substituents (c'), defined below ;

substituents (c') :

methoxy groups and halogen atoms.

15. A process according to Claim 1, wherein the reagents and reaction conditions are so chosen as to prepare a compound of formula (I) or salt thereof, in which : $R^1$ and $R^2$, together with the carbon atoms to which they are attached, represent a benzene ring fused to the isoxazole ring and being unsubstituted or having one substituent selected from halogen atoms.

16. A process according to Claim 1, wherein the reagents and reaction conditions are so chosen as to prepare a compound of formula (I) or salt thereof, in which : $R^3$ and $R^4$, are the same or different and each represents a hydrogen atom, a methyl group or an ethyl group.

17. A process according to Claim 1, wherein the reagents and reaction conditions are so chosen as to prepare a compound of formula (I) or salt thereof, in which : $R^3$, $R^4$ and the nitrogen atom to which they are attached together represent a morpholino group, a 1-piperazinyl group, a 4-methyl-1-piperazinyl group, a 1-pyrrolidinyl group or a piperidino group.

18. A process according to Claim 1, wherein the reagents and reaction conditions are so chosen as to prepare a compound of formula (I) or salt thereof, in which :

$R^1$ represents a hydrogen atom, a halogen atom or a $C_1$-$C_4$ alkyl group ;

$R^2$ represents a phenyl group or a substituted phenyl group having at least one of substituents (c), defined below ;

substituents (c) :

$C_1$-$C_3$ alkoxy groups, hydroxy groups and halogen atoms ;

$R^3$ and $R^4$ are the same or different and each represents a hydrogen atom, a $C_1$-$C_4$ alkyl group, a benzyl group or a phenyl group ; or

$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

substituents (d) :

$C_1$-$C_3$ alkyl groups, benzyl groups and phenyl groups.

19. A process according to Claim 1, wherein the reagents and reaction conditions are so chosen as to prepare a compound of formula (I) or salt thereof, in which :

$R^1$ and $R^2$, together with the carbon atoms to which they are attached, represent a 6-membered hydrocarbon ring fused to the isoxazole ring and being unsubstituted or having at least one of halogen atoms ;

$R^3$ and $R^4$ are the same or different and each represents a hydrogen atom, a $C_1$-$C_4$ alkyl group, a benzyl group or a phenyl group ; or

$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

substituents (d) :

$C_1$-$C_3$ alkyl groups, benzyl groups and phenyl groups.

20. A process according to Claim 1, wherein the reagents and reaction conditions are so chosen as to prepare a compound of formula (I) or salt thereof, in which :

$R^1$ represents a hydrogen atom, a chlorine atom or a $C_1$ or $C_2$ alkyl group ;

$R^2$ represents a phenyl group or a substituted phenyl group having at least one of substituents (c'), defined below ;

substituents (c') :

methoxy groups and halogen atoms ;

$R^3$ and $R^4$ are the same or different and each represents a hydrogen atom, a methyl group or an ethyl group ; or

$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

21. A process according to Claim 1, wherein the reagents and reaction conditions are so chosen as to prepare a compound of formula (I) or salt thereof, in which :

$R^1$ and $R^2$, together with the carbon atoms to which they are attached, represent a benzene ring fused to the isoxazole ring and being unsubstituted or having one of halogen atoms ;

$R^3$ and $R^4$ are the same or different and each represents a hydrogen atom, a methyl group or an ethyl group ; or

$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

22. A process according to Claim 3, in which the reagents and reaction conditions are so chosen as to prepare a compound of formula (Ib) :

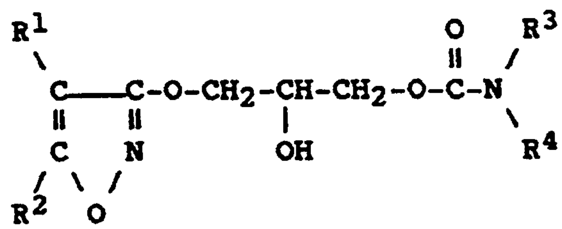

(Ib)

in which :

$R^1$ represents a hydrogen atom, a halogen atom or a $C_1$-$C_4$ alkyl group ;

$R^2$ represents a phenyl group or a substituted phenyl group having at least one of substituents (c), defined below ;

substituents (c) :

$C_1$-$C_3$ alkoxy groups, hydroxy groups and halogen atoms ; or

$R^1$ and $R^2$, together with the carbon atoms to which they are attached, represent a 6-membered hydrocarbon ring fused to the isoxazole ring and being unsubstituted or having at least one of halogen atoms ; and

$R^3$ and $R^4$ are the same or different and each represents a hydrogen atom, a $C_1$-$C_4$ alkyl group, a benzyl group or a phenyl group ; or

$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

substituents (d) :

$C_1$-$C_3$ alkyl groups, benzyl groups and phenyl groups,

or a pharmaceutically acceptable salt thereof.

23. A process according to Claim 22, in which the reagents and reaction conditions are so chosen as to prepare a compound of formula (Ib) :

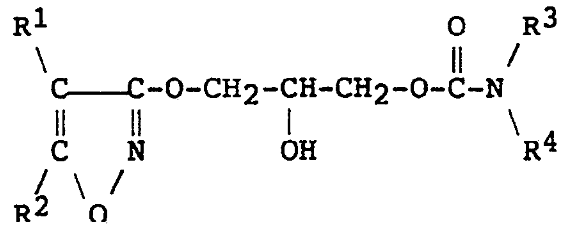

(Ib)

in which :

$R^1$ represents a hydrogen atom, a chlorine atom or a $C_1$ or $C_2$ alkyl group ;

$R^2$ represents a phenyl group or a substituted phenyl group having at least one of substituents (c'), defined below ; or

$R^1$ and $R^2$, together with the carbon atoms to which they are attached, represent a benzene ring fused to the isoxazole ring and being unsubstituted or having one of halogen atoms ;

substituents (c') :

methoxy groups and halogen atoms ; and

$R^3$ and $R^4$ are the same or different and each represents a hydrogen atoms, a methyl groups and ethyl groups; or

$R^3$, $R^4$ and the nitrogen atom to which they are attached together represent a morpholino group, a 1-piperazinyl group, a 4-methyl-1-piperazinyl group, a 1-pyrrolidinyl group or a piperidino group,

or a pharmaceutically acceptable salt thereof.

24. A process according to Claim 1, in which the reagents and reaction conditions are so chosen as to prepare :

2-(2-Hydroxy-3-morpholinopropyl)-4-methyl-5-phenyl-3-isoxazolone ;

3-(3-Carbamoyloxy-2-hydroxypropoxy)-5-(m-chlorophenyl)isoxazole ;

3-(2-Hydroxy-3-morpholinopropoxy)-5-phenylisoxazole ;

3-(2-Hydroxy-3-morpholinopropoxy)-4-methyl-5-phenylisoxazole ;

3-(3-Carbamoyloxy-2-hydroxypropoxy)-5-chloro-1,2- benzisoxazole ; or

a pharmaceutically acceptable salt thereof.

25. A method of preparing a pharmaceutical composition for treating a cerebrovascular disorder in a mammal suffering from or prone to cerebrovascular disorders or for effecting centrally-acting muscle relaxant activity, which comprises mixing at least one cerebro-active drug with a pharmaceutically acceptable carrier or diluent, wherein the cerebro-active drug is a compound of formula (I) :

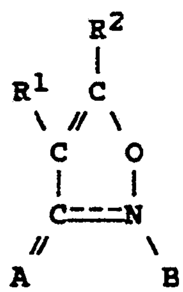

(I)

in which : either

(a) the dotted line (- - -) represents a single bond ;

A = represents a oxygen atom ; and

B represents a group of formula (II) :

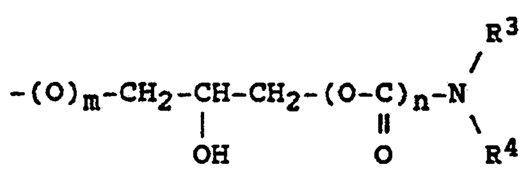

(II)

in which m is 0 and n is 0 or 1 ; or

(b) the dotted line (- - -) represents a double bond ;

A = represents said group of formula (II) in which m is 1 and n is 0 or 1 ; and
B is absent ; and

$R^1$ represents a hydrogen atom, a halogen atom, a $C_1$-$C_4$ alkyl group, a $C_2$-$C_4$ alkenyl group, a $C_2$-$C_4$ alkynyl group, a benzyl group, a substituted benzyl group having at least one of substituents (a), defined below, a phenyl group or a substituted phenyl group having at least one of substituents (a), defined below ;
$R^2$ represents a hydrogen atom, a halogen atom, a $C_1$-$C_4$ alkyl group, a phenyl group, a substituted phenyl group having at least one of substituents (a), defined below, or a heterocyclic group having 5 or 6 ring atoms, of which from 1 to 3 are nitrogen and/or oxygen and/or sulphur hetero-atoms, said heterocyclic group being unsubstituted or having at least one of substituents (a), defined below ; or
$R^1$ and $R^2$, together with the carbon atoms to which they are attached, represent a hydrocarbon ring fused to the isoxazole ring and having, in total, from 5 to 7 ring carbon atoms, said hydrocarbon ring being unsubstituted or having at least one of substituents (b), defined below ;
$R^3$ and $R^4$ are the same or different and each represents a hydrogen atom, a $C_1$-$C_4$ alkyl group, a benzyl group, a substituted benzyl group having at least one of substituents (a), defined below, a phenyl group or a substituted phenyl group having at least one of substituents (a), defined below ; or
$R^3$, $R^4$ and the nitrogen atom to which they are attached together represent an alicyclic amino group having a total of 5 or 6 ring atoms, of which one is said nitrogen atom and 0 or 1 is an additional nitrogen and/or oxygen and/or sulphur hetero-atom, said alicyclic amino group being unsubstituted or, where there is an additional nitrogen hetero-atom, having at least one of substituents (b), defined below, on that additional nitrogen hetero-atom ;

substituents (a) :
$C_1$-$C_3$ alkyl groups, $C_1$-$C_3$ alkoxy groups, hydroxy groups, halogen atoms, nitro groups, amino groups and $C_2$-$C_4$ aliphatic carboxylic acyl groups ;

substituents (b) :
$C_1$-$C_3$ alkyl groups, $C_1$-$C_3$ alkoxy groups, hydroxy groups, halogen atoms, nitro groups, amino groups, $C_2$-$C_4$ aliphatic carboxylic acylamino groups, benzyl groups, substituted benzyl groups having at least one of substituents (a), defined above, phenyl groups and substituted phenyl groups having at least one of substituents (a), defined above ;

provided that :
(i) where B represents said group of formula (II) and n is 0, then $R^1$ does not represent a hydrogen or halogen atom ;
(ii) when (- - -) represents a single bond, $R^1$ and $R^2$ together with the carbon atoms to which they are attached, form a $C_{6-7}$ hydrocarbon ring, then n = 1 ;
and pharmaceutically acceptable salts thereof.

26. A method according to Claim 25, wherein said cerebro-active drug is represented by the formula (Ib):

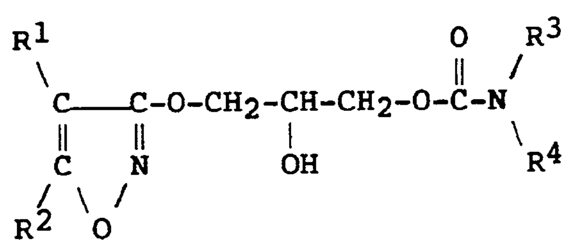

(Ib)

in which :
$R^1$ represents a hydrogen atom, a halogen atom or a $C_1$-$C_4$ alkyl group ;
$R^2$ represents a phenyl group or a phenyl group having at least one of substituents (c), defined below ;

substituents (c) :
$C_1$-$C_3$ alkoxy groups, hydroxy groups and halogen atoms ; or
$R^1$ and $R^2$, together with the carbon atoms to which they are attached, represent a 6-membered hydrocarbon ring fused to the isoxazole ring and being unsubstituted or having at least one of halogen atoms ; and

$R^3$ and $R^4$ are the same or different and each represents a hydrogen atom, a $C_1$-$C_4$ alkyl group, a benzyl group or a phenyl group ; or

$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

substituents (d) :

$C_1$-$C_3$ alkyl groups, benzyl groups and phenyl groups.

27. A method according to Claim 25, wherein said cerebro-active drug is represented by the formula (Ib):

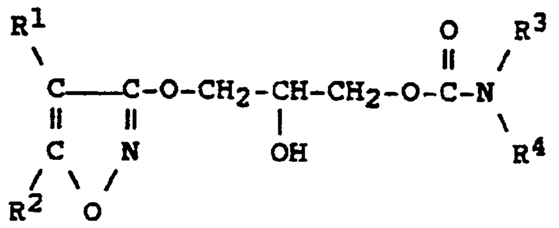

(Ib)

in which :

$R^1$ represents a hydrogen atom, a chlorine atom or a $C_1$ or $C_2$ alkyl group ;

$R^2$ represents a phenyl group or a phenyl group having at least one of substituents (c'), defined below ; or

$R^1$ and $R^2$, together with the carbon atoms to which they are attached, represent a benzene ring fused to the isoxazole ring and being unsubstituted or having one of halogen atoms ; and

substituents (c') :

methoxy groups and halogen atoms ;

$R^3$ and $R^4$ are the same or different and each represents a hydrogen atom, a methyl group or an ethyl group ; or

$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

28. A method according to Claim 25, wherein said cerebro-active drug is at least one of the compounds :

2-(2-hydroxy-3-morpholinopropyl)-4-methyl-5-phenyl-3-isoxazolone ;

3-(3-carbamoyloxy-2-hydroxyproproxy)-5-(m-chlorophenyl)isoxazole ;

3-(2-hydroxy-3-morpholinopropoxy)-5-phenylisoxazole ;

3-(2-hydroxy-3-morpholinopropoxy)-4-methyl-5-phenyl isoxazole ;

3-(3-carbamoyloxy-2-hydroxypropoxy)-5-chloro-1,2-benzisoxazole ;

and pharmaceutically acceptable salts thereof.

29. The use for the manufacture of a medicament for treating a cerebrovascular disorder of at least one cerebro-active drug, wherein the cerebro-active drug is a compound of formula (I) :

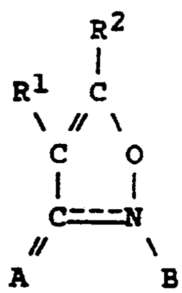

(I)

in which : either

(a) the dotted line (---) represents a single bond ;

A = represents a oxygen atom ; and

B represents a group of formula (II) :

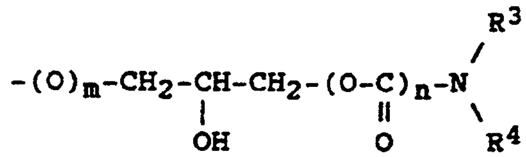

(II)

in which m is 0 and n is 0 or 1 ; or

(b) the dotted line (- - -) represents a double bond ;

A = represents said group of formula (II) in which m is 1 and n is 0 or 1 ; and
B is absent ; and

$R^1$ represents a hydrogen atom, a halogen atom, a $C_1$-$C_4$ alkyl group, a $C_2$-$C_4$ alkenyl group, a $C_2$-$C_4$ alkynyl group, a benzyl group, a substituted benzyl group having at least one of substituents (a), defined below, a phenyl group or a substituted phenyl group having at least one of substituents (a), defined below ;
$R^2$ represents a hydrogen atom, a halogen atom, a $C_1$-$C_4$ alkyl group, a phenyl group, a substituted phenyl group having at least one of substituents (a), defined below, or a hetercyclic group having 5 or 6 ring atoms, of which from 1 to 3 are nitrogen and/or oxygen and/or sulphur hetero-atoms, said heterocyclic group being unsubstituted or having at least one of substituents (a), defined below ; or
$R^1$ and $R^2$, together with the carbon atoms to which they are attached, represent a hydrocarbon ring fused to the isoxazole ring and having, in total, from 5 to 7 ring carbon atoms, said hydrocarbon ring being unsubstituted or having at least one of substituents (b), defined below ;
$R^3$ and $R^4$ are the same or different and each represents a hydrogen atom, a $C_1$-$C_4$ alkyl group, a benzyl group, a substituted benzyl group having at least one of substituents (a), defined below, a phenyl group or a substituted phenyl group having at least one of substituents (a), defined below ; or
$R^3$, $R^4$ and the nitrogen atom to which they are attached together represent an alicyclic amino group having a total of 5 or 6 ring atoms, of which one is said nitrogen atom and 0 or 1 is an additional nitrogen and/or oxygen and/or sulphur hetero-atom, said alicyclic amino group being unsubstituted or, where there is an additional nitrogen hetero-atom, having at least one of substituents (b), defined below, on that additional nitrogen hetero-atom ;

substituents (a) :
$C_1$-$C_3$alkyl groups, $C_1$-$C_3$ alkoxy groups, hydroxy groups, halogen atoms, nitro groups, amino groups and $C_2$-$C_4$ aliphatic carboxylic acyl groups ;

substituents (b) :
$C_1$-$C_3$ alkyl groups, $C_1$-$C_3$ alkoxy groups, hydroxy groups, halogen atoms, nitro groups, amino groups, $C_2$-$C_4$ aliphatic carboxylic acylamino groups, benzyl groups, substituted benzyl groups having at least one of substituents (a), defined above, phenyl groups and substituted phenyl groups having at least one of substituents (a), defined above ;

provided that :
(i) where B represents said group of formula (II) and n is 0, then $R^1$ does not represent a hydrogen or halogen atom ;
(ii) when (- - -) represents a single bond, $R^1$ and $R^2$, together with the carbon atoms to which they are attached, form a $C_{6-7}$ hydrocarbon ring, then n = 1 ;
and pharmaceutically acceptable salts thereof.

30. The use according to Claim 31, wherein said cerebro-active drug is represented by the formula (Ib) :

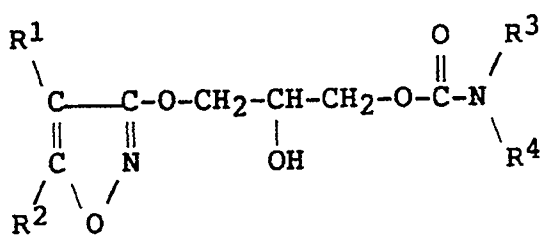

(Ib)

in which :
$R^1$ represents a hydrogen atom, a halogen atom or a $C_1$-$C_4$ alkyl group ;
$R^2$ represents a phenyl group or a phenyl group having at least one of substituents (c), defined below ;
substituents (c) :
$C_1$-$C_3$ alkoxy groups, hydroxy groups and halogen atoms ; or
$R^1$ and $R^2$, together with the carbon atoms to which they are attached, represent a 6-membered hydrocarbon ring fused to the isoxazole ring and being unsubstituted or having at least one of halogen atoms ; and $R^3$ and $R^4$ are the same or different and each represents a hydrogen atom, a $C_1$-$C_4$ alkyl group, a benzyl group or a phenyl group ; or
$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;
substituents (d) :
$C_1$-$C_3$ alkyl groups, benzyl groups and phenyl groups.

31. The use according to Claim 29, wherein said cerebro-active drug is represented by the formula (Ib) :

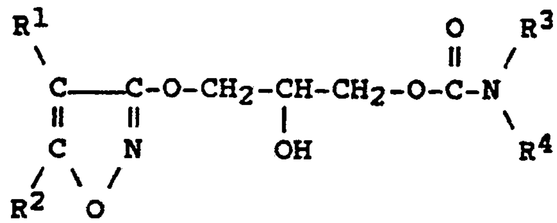

(Ib)

in which :
$R^1$ represents a hydrogen atom, a chlorine atom or a $C_1$ or $C_2$ alkyl group ;
$R^2$ represents a phenyl group or a phenyl group having at least one of substituents (c'), defined below ; or
$R^1$ and $R^2$, together with the carbon atoms to which they are attached, represent a benzene ring fused to the isoxazole ring and being unsubstituted or having one of halogen atoms ; and
substituents (c') :
methoxy groups and halogen atoms ;
$R^3$ and $R^4$ are the same or different and each represents a hydrogen atom, a methyl group or an ethyl group ; or
$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

32. The use according to Claim 29, wherein said cerebro-active drug is at least one of the compounds :
2-(2-hydroxy-3-morpholinopropyl)-4-methyl-5-phenyl-3-isoxazolone ;
3-(3-carbamoyloxy-2-hydroxyproproxy)-5-(m-chlorophenyl)isoxazole ;
3-(2-hydroxy-3-morpholinopropoxy)-5-phenylisoxazole ;
3-(2-hydroxy-3-morpholinopropoxy)-4-methyl-5-phenylisoxazole ;
3-(3-carbamoyloxy-2-hydroxypropoxy)-5-chloro-1,2-benzisoxazole ;
and pharmaceutically acceptable salts thereof.

33. The use for the manufacture of a medicament for effecting centrally-acting muscle relaxant activity of an active compound, wherein the active compound is a compound of formula (I) or a pharmaceutically acceptable salt thereof, as claimed in Claim 29.

34. The use according to Claim 33, wherein said active compound is represented by the formula (Ib) :

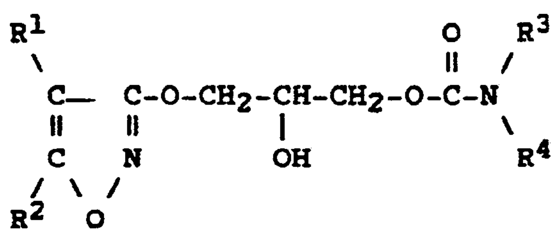

(Ib)

in which :
$R^1$ represents a hydrogen atom, a halogen atom or a $C_1$-$C_4$ alkyl group ;

$R^2$ represents a phenyl group or a phenyl group having at least one of substituents (c), defined below ;

substituents (c) :

$C_1$-$C_3$ alkoxy groups, hydroxy groups and halogen atoms ; or

$R^1$ and $R^2$, together with the carbon atoms to which they are attached, represent a 6-membered hydrocarbon ring fused to the isoxazole ring and being unsubstituted or having at least one of halogen atoms ; and

$R^3$ and $R^4$ are the same or different and each represents a hydrogen atom, a $C_1$-$C_4$ alkyl group, a benzyl group or a phenyl group ; or

$R^3$, $R^4$ and the nitrogen atom to which they are attached together represent an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from substituents (d), defined below ;

substituents (d) :

$C_1$-$C_3$ alkyl groups, benzyl groups and phenyl groups.

35. The use according to Claim 33, wherein said active compound is represented by the formula (Ib) :

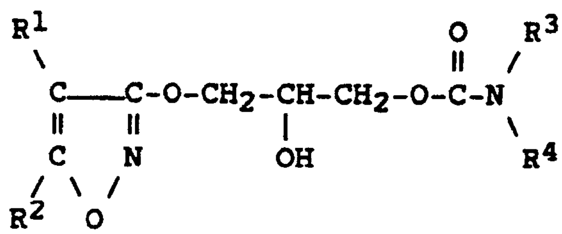

(Ib)

in which :

$R^1$ represents a hydrogen atom, a chlorine atom or a $C_1$ or $C_2$ alkyl group ;

$R^2$ represents a phenyl group or a phenyl group having at least one of substituents (c'), defined below ; or

$R^1$ and $R^2$, together with the carbon atoms to which they are attached, represent a benzene ring fused to the isoxazole ring and being unsubstituted or having one of halogen atoms ; and

substituents (c') :

methoxy groups and halogen atoms ;

$R^3$ and $R^4$ are the same or different and each represents a hydrogen atom, a methyl group or an ethyl group ; or

$R^3$, $R^4$ and the nitrogen atom to which they are attached together represent a morpholino group, a 1-piperazinyl group, a 4-methyl-1-piperazinyl group, a 1-pyrrolidinyl group or a piperidino group.

36. The use according to Claim 33, wherein said active compound is at least one of the compounds :

2-(2-hydroxy-3-morpholinopropyl)-4-methyl-5-phenyl-3-isoxazolone ;

3-(3-carbamoyloxy-2-hydroxyproproxy)-5-(m-chlorophenyl)isoxazole ;

3-(2-hydroxy-3-morpholinopropoxy)-5-phenylisoxazole ;

3-(2-hydroxy-3-morpholinopropoxy)-4-methyl-5-phenylisoxazole ;

3-(3-carbamoyloxy-2-hydroxypropoxy)-5-chloro-1,2-benzisoxazole ;

or a pharmaceutically acceptable salt thereof.

## Patentansprüche

### Patentansprüche für folgenden Vertragsstaaten : AT, BE, CH, DE,FR, GB, IT, LI, LU, NL, SE

1. Verbindungen der Formel (I)

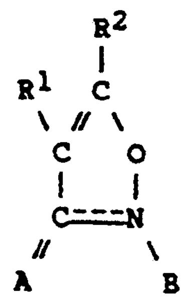

(I)

in welcher entweder

(a) die strichlierte Linie (- - -) eine Doppelbindung bedeutet,

A = für ein Sauerstoffatom steht und

B für eine Gruppe der Formel (II)

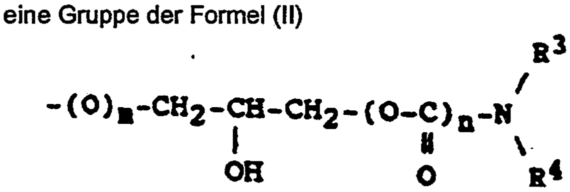

(II)

mit der Bedeutung von 0 für m und der Bedeutung von 0 oder 1 für n steht oder

(b) die strichlierte Linie (- - -) eine Doppelbindung bedeutet,

A = die erwähnte Gruppe der Formel (II) mit der Bedeutung von 1 für m und der Bedeutung von 0 oder 1 für n darstellt und

B nicht vorhanden ist und

$R^1$ ein Wasserstoffatom, ein Halogenatom, eine $C_1$-$C_4$-Alkylgruppe, eine $C_2$-$C_4$-Alkinylgruppe, eine $C_2$-$C_4$-Alkinylgruppe, eine Benzylgruppe, eine substituierte Benzylgruppe mit zumindest einem der unten definierten Substituenten (a), eine Phenylgruppe oder eine substituierte Phenylgruppe mit zumindest einem der unten definierten Substituenten (a) bedeutet,

$R^2$ ein Wasserstoffatom, ein Halogenatom, eine $C_1$-$C_4$-Alkylgruppe, eine Phenylgruppe, eine substituierte Phenylgruppe mit zumindest einem der unten definierten Substituenten (a) oder eine heterocyclische Gruppe mit 5 oder 6 Ringatomen, von welchen 1 bis 3 von Stickstoff- und/oder Sauerstoff- und/oder Schwefelatomen gebildeten Heteroatomen sind, darstellt, wobei die heterocyclische Gruppe unsubstituiert ist oder zumindest einen der unten definierten Substituenten (a) aufweist, oder

$R^1$ und $R^2$ zusammen mit den diese Reste tragenden Kohlenstoffatomen einen an den Isoxazolring ankondensierten und insesamt 5 bis 7 Ringkohlenstoffatome besitzenden Kohlenwasserstoffring bilden, welcher unsubstituiert ist oder zumindest einen der unten definierten Substituenten (b) aufweist,

$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der unten definierten Substituenten (a), eine Phenylgruppe oder eine substituierte Phenylgruppe mit zumindest einem der unten definierten Substituenten (a) darstellen oder

$R^3$ und $R^4$ zusammen mit dem diese Reste tragenden Stickstoffatom eine alicyclische Aminogruppe mit insgesamt 5 oder 6 Ringatomen bilden, von welchen eines das erwähnte Stickstoffatom ist und 0 oder 1 ein weiteres von Stickstoff und/oder Sauerstoff und/oder Schwefel gebildetes Heteroatom ist, wobei diese alicyclische Aminogruppe unsubstituiert ist oder, falls ein weiteres Stickstoffatom als Heteroatom vorliegt, an diesem von Stickstoff gebildeten zusätzlichen Heteroatom zumindest einen der unten definierten Substituenten (b) aufweist, wobei

die Substituenten (a)

$C_1$-$C_3$-Alkylgruppen, $C_1$-$C_3$-Alkoxygruppen, Hydroxygruppen, Halogenatome, Nitrogruppen, Aminogruppen und von aliphatischen Carbonsäuren abgeleitete $C_2$-$C_4$-Acylgruppen sind und

die Substituenten (b)

$C_1$-$C_3$-Alkylgruppen, $C_1$-$C_3$-Alkoxygruppen, Hydroxygruppen, Halogenatome, Nitrogruppen, Aminogruppen, von aliphatischen Carbonsäuren abgeleitete $C_2$-$C_4$-Acylaminogruppen, Benzylgruppen, substituierte Benzylgruppen mit zumindest einem der oben definierten Substituenten (a), Phenylgruppen und substituierte

Phenylgruppen mit zumindest einem der oben definierten Substituenten (a) sind,

mit der Maßgabe, daß

(i) falls B für die erwähnte Gruppe der Formel (II) steht und n gleich ist 0, $R^1$ nicht für ein Wasserstoffatom oder ein Halogenatom steht,

(ii) falls A für die erwähnte Gruppe der Formel (II) steht und n gleich ist 0, $R^1$ und $R^2$ zusammen einen an den Isoxazolring ankondensierten Benzolring bilden und, falls $R^4$ eine Alkylgruppe bedeutet, $R^3$ nicht für ein Wasserstoffatom steht, und

(iii) falls (- - -) für eine Einfachbindung steht und $R^1$ und $R^2$ zusammen mit den diese Reste tragenden Kohlenstoffatomen einen $C_6$-$C_7$-Kohlenwasserstoffring bilden, n gleich ist 1,

und pharmazeutisch annehmbare Salze hievon, jedoch

unter Ausschluß

der Verbindung 5-Chlor-2-(3-piperidino-2-hydroxy)propylbenzisoxazolin-3-on.

2. Verbindungen nach Anspruch 1 der Formel (Ia)

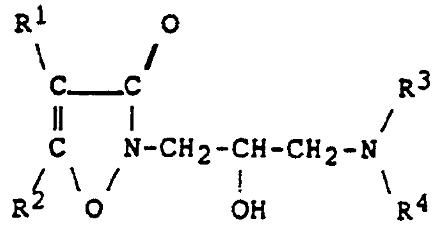

(Ia)

in welcher

$R^1$ eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe, eine Phenylgruppe oder eine substituierte Phenylgruppe darstellt und hiebei die Substituenten aus den in Anspruch 1 definierten Substituenten (a) ausgewählt sind,

$R^2$ ein Wasserstoffatom, eine $C_1$-$C_4$-Alkylgruppe, eine Phenylgruppe, eine substituierte Phenylgruppe oder eine in Anspruch 1 definierte heterocyclische Gruppe bedeutet und

$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe, eine Phenylgruppe oder eine substituierte Phenylgruppe bedeuten oder $R^3$ und $R^4$ zusammen mit dem diese Reste tragenden Stickstoffatom eine in Anspruch 1 definierte alicyclische Aminogruppe bilden,

und pharmazeutisch annehmbare Salze hievon.

3. Verbindungen nach Anspruch 1 der Formel (Ib)

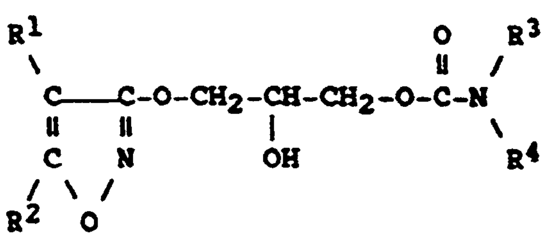

(Ib)

in welcher

$R^1$ ein Wasserstoffatom, ein Halogenatom, eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeutet,

$R^2$ ein Wasserstoffatom, eine $C_1$-$C_4$-Alkylgruppe, eine Phenylgruppe, eine substituierte Phenylgruppe mit zumindest einem der in Anspruch 1 definierten Substituenten (a) oder eine in Anspruch 1 definierte heterocyclische Gruppe darstellt oder

$R^1$ und $R^2$ zusammen mit den diese Reste tragenden Kohlenstoffatomen einen in Anspruch 1 definierten und an den Isoxazolring ankondensierten Kohlenwasserstoffring bilden,

$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem

der in Anspruch 1 definierten Substituenten (a) bedeuten oder
$R^3$, $R^4$ und das diese Reste tragende Stickstoffatom zusammen eine in Anspruch 1 definierte alicyclische Aminogruppe bilden,
und pharmazeutisch annehmare Salze hievon.

4. Verbindungen nach Anspruch 1 der Formel (Ic)

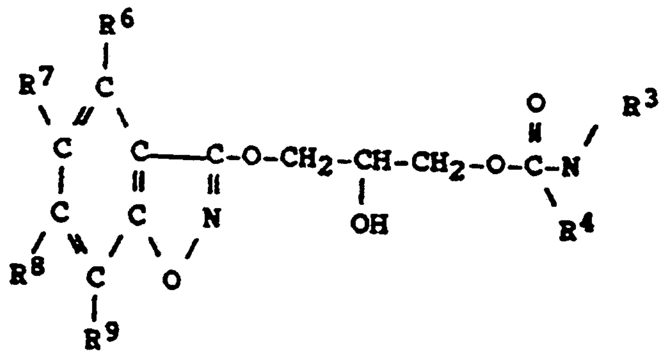

(Ic)

in welcher
$R^3$ und $R^4$ untereinander gleich oder voneinander verschieden sind und je für sich ein Wasserstoffatom, eine $C_1$-$C_4$ -Alkylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substitufnten (a) darstellen oder
$R^3$, $R^4$ und das diese Reste tragende Stickstoffatom gemeinsam eine in Anspruch 1 definierte alicyclische Aminogruppe bilden und
einer der Reste $R^6$, $R^7$, $R^8$ und $R^9$ ein Wasserstoffatom, ein Halogenatom, eine $C_1$-$C_4$-Alkylgruppe oder eine $C_1$-$C_4$-Alkoxygruppe bedeutet und die anderen Reste durchwegs für Wasserstoffatome stehen,
und pharmazeutisch annehmbare Salze hievon.

5. Verbindungen nach Anspruch 1 der Formel (Id)

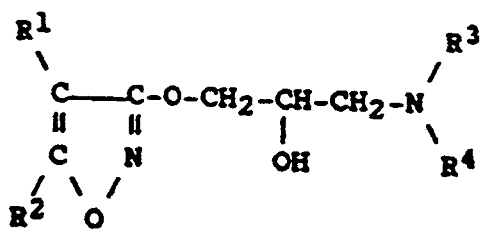

(Id)

in welcher
$R^1$ ein Wasserstoffatom, ein Halogenatom, eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeutet,
$R^2$ ein Wasserstoffatom, eine $C_1$-$C_4$-Alkylgruppe, eine Phenylgruppe, eine substituierte Phenylgruppe mit zumindest einem der in Anspruch 1 definierten Substituenten (a) oder eine in Anspruch 1 definierte heterocyclische Gruppe darstellt oder
$R^1$ und $R^2$ zusammen mit den diese Reste tragenden Kohlenstoffatomen einen in Anspruch 1 definierten und an den Isoxazolring ankondensierten Kohlenwasserstoffring bilden,
$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeuten oder
$R^3$, $R^4$ und das diese Reste tragende Stickstoffatom eine in Anspruch 1 definierte alicyclische Aminogruppe bilden,
mit der Maßgabe, daß, falls $R^1$ und $R^2$ gemeinsam einen an den Isoxazolring ankondensierten Benzolring bilden und $R^4$ für eine Alkylgruppe steht, $R^3$ nicht für ein Wasserstoffatom steht,

und pharmazeutisch annehmbare Salze hievon.

6. Verbindungen nach Anspruch 1 der Formel (Ie)

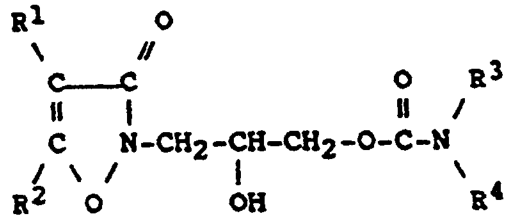

(Ie)

in welcher
$R^1$ ein Wasserstoffatom, ein Halogenatom, eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeutet,
$R^2$ ein Wasserstoffatom, eine $C_1$-$C_4$-Alkylgruppe, eine Phenylgruppe, eine substituierte Phenylgruppe mit zumindest einem der in Anspruch 1 definierten Substituenten (a) oder eine in Anspruch 1 definierte heterocyclische Gruppe darstellt oder
$R^1$ und $R^2$ zusammen mit den diese Reste tragenden Kohlenstoffatomen einen in Anspruch 1 definierten und an den Isoxazolring ankondensierten Kohlenwasserstoffring bilden,
$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) darstellt oder
$R^3$, $R^4$ und das diese Reste tragende Stickstoffatom gemeinsan eine in Anspruch 1 definierte alicyclische Aminogruppe bilden und
pharmazeutisch annehmbare Salze hievon.

7. Vrbindungen nach Anspruch 1 der Formel (If)

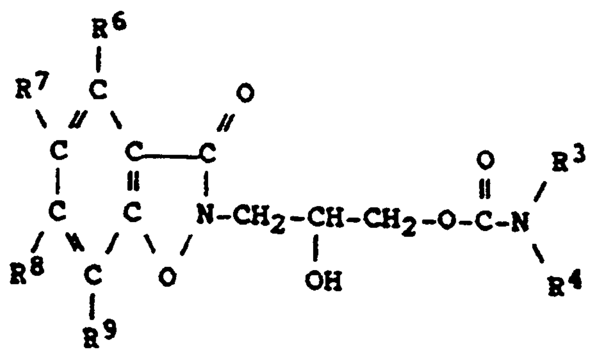

(If)

in welcher
$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeuten oder
$R^3$, $R^4$ und das diese Reste tragende Stickstoffatom gemeinsam eine in Anspruch 1 definierte alicyclische Aminogruppe bilden und
einer der Reste $R^6$, $R^7$, $R^8$ und $R^9$ ein Wasserstoffatom, ein Halogenatom, eine $C_1$-$C_4$-Alkylgruppe oder eine $C_1$-$C_4$-Alkoxygruppe bedeutet und die anderen Reste durchwegs für Wasserstoffatome stehen,
und pharmazeutisch annehmbare Salze hievon.

8. Verbindungen nach Anspruch 1, worin $R^1$ ein Wasserstoffatom, ein Halogenatom oder eine $C_1$-$C_4$-Alkylgruppe bedeutet.

9. Verbindungen nach Anspruch 1, worin $R^2$ eine Phenylgruppe oder eine substituierte Phenylgruppe mit zumindest einem der unten definierten Substituenten (c) darstellt und die

Substituenten (c)

$C_1$-$C_3$-Alkoxygruppen, Hydroxygruppen und Halogenatome sind.

10. Verbindungen nach Anspruch 1, worin $R^1$ und $R^2$ zusammen mit den diese Reste tragenden Kohlenstoffatomen einen an den Isoxazolring ankondensierten 6-gliedrigen Kohlenwasserstoffring bilden, welcher unsubstituiert ist oder zumindest ein Halogenatom aufweist.

11. Verbindungen nach Anspruch 1, worin $R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen.

12. Verbindungen nach Anspruch 1, worin $R^3$, $R^4$ und das diese Reste tragende Stickstoffatom eine alicyclische Aminogruppe mit insgesamt 5 oder 6 Ringatomen bilden, von welchen eines das erwähnte Stickstoffatom ist und 0 oder 1 ein von zusätzlichem Stickstoff gebildetes Heteroatom ist, wobei diese alicyclische Aminogruppe unsubstituiert ist oder, falls ein zusätzliches Stickstoffatom als Heteroatom vorliegt, zumindest ein Substituent an diesem zusätzlichen von Stickstoff gebildetem Heteroatom aus den folgenden Substituenten (d) ausgewählt ist und die

Substituenten (d)

$C_1$-$C_3$-Alkylgruppen, Benzylgruppen und Phenylgruppen sind.

13. Verbindungen nach Anspruch 1, worin $R^1$ ein Wasserstoffatom, ein Chloratom oder eine $C_1$- oder $C_2$-Alkylgruppe ist.

14. Verbindungen nach Anspruch 1, worin $R^2$ eine Phenylgruppe oder eine substituierte Phenylgruppe mit zumindest einem der unten definierten Substituenten (c') ist und die

Substituenten (c')

Methoxygruppen und Halogenatome sind.

15. Verbindungen nach Anspruch 1, worin $R^1$ und $R^2$ zusammen mit den diese Reste tragenden Kohlenstoffatomen einen an den Isoxazolring ankondensierten Benzolring bilden, welcher unsubstituiert ist und zumindest einen aus Halogenatomen ausgewählten Substituenten aufweist.

16. Verbindungen nach Anspruch 1, worin $R^3$ und $R^4$ untereinander gleich oder voneinander verschieden sind und je für sich ein Wasserstoffatom, eine Methylgruppe oder eine Äthylgruppe bedeuten.

17. Verbindungen nach Anspruch 1, worin $R^3$, $R^4$ und das diese Reste tragende Stickstoffatom gemeinsam eine Morpholinogruppe, eine 1-Piperazinylgruppe, eine 4-Methyl-1-piperazinylgruppe, eine 1-Pyrrolidinylgruppe oder eine Piperidinogruppe darstellen.

18. Verbindungen nach Anspruch 1, worin

$R^1$ ein Wasserstoffatom, ein Halogenatom oder eine $C_1$-$C_4$-Alkylgruppe bedeutet,

$R^2$ eine Phenylgruppe oder eine substituierte Phenylgruppe mit zumindest einem der unten definierten Substituenten (c) darstellt und die

Substituenten (c)

$C_1$-$C_3$-Alkoxygruppen, Hydroxygruppen und Halogenatome sind,

$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe bedeuten oder

$R^3$, $R^4$ und das diese Reste tragende Stickstoffatom eine alicyclische Aminogruppe mit insgesamt 5 oder 6 Ringatomen bilden, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

19. Verbindungen nach Anspruch 1, worin

$R^1$ und $R^2$ zusammen mit den diese Reste tragenden Stickstoffatomen einen an den Isoxazolring ankondensierten 6-gliedrigen Kohlenwasserstoffring bilden, welcher unsubstituiert ist oder zumindest einen von Halogenatomen gebildeten Substituenten aufweist,

$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen oder

$R^3$, $R^4$ und das diese Reste tragende Stickstoffatom gemeinsam eine alicyclische Aminogruppe mit insgesamt 5 oder 6 Ringatomen bilden, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

20. Verbindungen nach Anspruch 1, worin

$R^1$ ein Wasserstoffatom, ein Chloratom oder eine $C_1$- oder $C_2$-Alkylgruppe bedeutet,

$R^2$ eine Phenylgruppe oder eine substituierte Phenylgruppe mit zumindest einem der unten definierten Substituenten (c') darstellt und die

Substituenten (c')

Methoxygruppen und Halogenatome sind,

$R^3$ und $R^4$ untereinander gleich oder voneinander verschieden sind und je für sich ein Wasserstoffatom, eine Methylgruppe oder eine Äthylgruppe darstellen oder

$R^3$, $R^4$ und das diese Reste tragende Stickstoffatom zusammen eine Morpholinogruppe, eine 1-Piperazinylgruppe, eine 4-Methyl-1-piperazinylgruppe, eine 1-Pyrrolidinylgruppe oder eine Piperidinogruppe darstellen.

21. Verbindungen nach Anspruch 1, worin

$R^1$ und $R^2$ zusammen mit den diese Reste tragenden Kohlenstoffatomen einen an den Isoxazolring ankondensierten Benzolring bilden, welcher unsubstituiert ist oder ein Halogenatom als Substituenten aufweist,

$R^3$ und $R^4$ untereinander gleich oder voneinander verschieden sind und je für sich ein Wasserstoffatom, eine Methylgruppe oder eine Äthylgruppe bedeuten oder

$R^3$, $R^4$ und das diese Reste tragende Stickstoffatom eine Morpholinogruppe, eine 1-Piperazinylgruppe, eine 4-Methyl-1-piperazinylgruppe, eine 1-Pyrrolidinylgruppe oder eine Piperidinogruppe bedeuten.

22. Verbindungen nach Anspruch 3 der Formel (Ib)

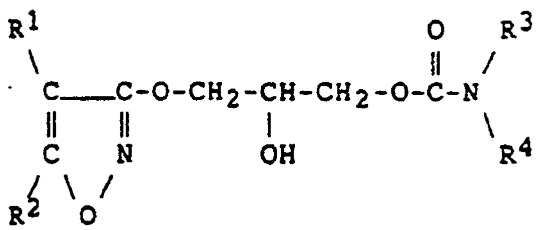

(Ib)

in welcher

$R^1$ ein Wasserstoffatom, ein Halogenatom oder eine $C_1$-$C_4$-Alkylgruppe bedeutet,

$R^2$ eine Phenylgruppe oder eine substituierte Phenylgruppe mit zumindest einem der unten definierten Substituenten (c) darstellt und die

Substituenten (c)

$C_1$-$C_3$-Alkoxygruppen, Hydroxygruppen und Halogenatome sind oder

$R^1$ und $R^2$ zusammen mit den diese Reste tragenden Kohlenstoffatomen einen an den Isoxazolring ankondensierten 6-gliedrigen Kohlenwasserstoffring darstellen, welcher unsubstituiert ist oder zumindest ein Halogen als Substituenten aufweist und

$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen oder

$R^3$, $R^4$ und das diese Reste tragende Stickstoffatom eine alicyclische Aminogruppe mit insgesamt 5 oder 6 Ringatomen bilden, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

23. Verbindungen nach Anspruch 22, der Formel (Ib)

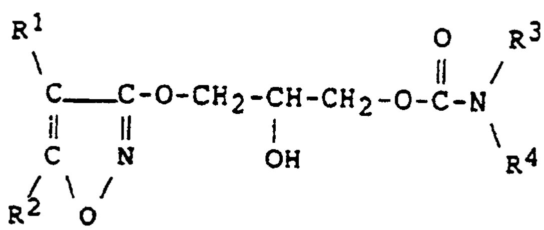

(Ib)

in welcher

$R^1$ ein Wasserstoffatom, ein Chloratom oder eine $C_1$- oder $C_2$-Alkylgruppe bedeutet,
$R^2$ eine Phenylgruppe oder eine substituierte Phenylgruppe mit zumindest einem der unten definierten Substituenten (c') darstellt oder
$R^1$ und $R^2$ zusammen mit den diese Reste tragenden Kohlenstoffatomen einen an den Isoxazolring ankondensierten Benzolring darstellen, welcher unsubstituiert ist oder ein Halogen als Substituenten aufweist, und hiebei die

Substituenten (c')
Methoxygruppen und Halogenatome sind, und

$R^3$ und $R^4$ untereinander gleich oder voneinander verschieden sind und je für sich Wasserstoffatome, Methylgruppen und Äthylgruppen darstellen oder
$R^3$, $R^4$ und das diese Reste tragende Stickstoffatom zusammen eine Morpholinogruppe, eine 1-Piperazinylgruppe, eine 4-Methyl-1-piperazinylgruppe, eine 1-Pyrrolidinylgruppe oder eine Piperidinogruppe darstellen.

24. 2-(2-Hydroxy-3-morpholinopropyl)-4-methyl-5-phenyl-3-isoxazolon und pharmazeutisch annehmare Salze hievon.

25. 3-(3-Carbamoyloxy-2-hydroxypropoxy)-5-(m-chlorphenyl)isoxazol und pharmazeutisch annehmbare Saize hievon.

26. 3-(2-Hydroxy-3-morpholinopropoxy)-5-phenylisoxazol und pharmazeutisch annehmbare Salze hievon.

27. 3-(2-Hydroxy-3-morpholinopropoxy)-4-methyl-5-phenyl-isoxazol und pharmazeutisch annehmbare Salze hievon.

28. 3-(3-Carbamoyloxy-2-hydroxypropoxy)-5-chlor-1,2-benzisoxazol und pharmazeutisch annehmbare Salze hievon.

29. Verwendung zumindest einer cerebro-aktiven Substanz zum Herstellen eines Heilmittels für die Behandlung einer cerebrovasculären Störung, worin die cerebro-aktive Substanz eine Verbindung der Formel (I)

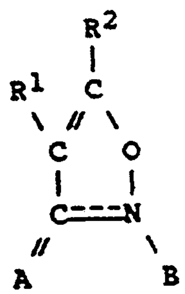

(I)

ist, in welcher entweder
(a) die strichlierte Linie (---) eine Doppelbindung bedeutet,

A = für ein Sauerstoffatom steht und
B für eine Gruppe der Formel (II)

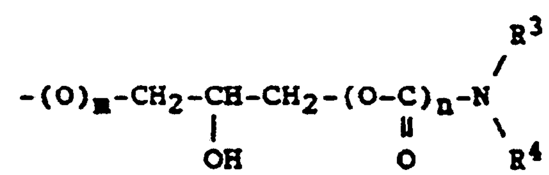

(II)

mit der Bedeutung von 0 für m und der Bedeutung von 0 oder 1 für n steht oder
(b) die strichlierte Linie (---) eine Doppelbindung bedeutet,

A = die erwähnte Gruppe der Formel (II) mit der Bedeutung von 1 für m und der Bedeutun, von 0 oder 1 für n darstellt und
B nicht vorhanden ist und

$R^1$ ein Wasserstoffatom, ein Halogenatom, eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe mit zumindest einem der unten definierten Substituenten (a), eine Phenylgruppe oder eine substituierte Phenylgruppe mit zumindest einem der unten definierten Substituenten (a) bedeutet,

$R^2$ ein Wasserstoffatom, ein Halogenatom, eine $C_1$-$C_4$-Alkylgruppe, eine Phenylgruppe, eine substituierte Phenylgruppe mit zumindest einem der unten definierten Substituenten (a) oder eine heterocyclische Gruppe mit 5 oder 6 Ringatomen, von welchen 1 bis 3 von Stickstoff- und/oder Sauerstoff- und/oder Schwefelatomen gebildeten Heteroatomen sind, darstellt, wobei die heterocyclische Gruppe unsubstituiert ist oder zumindest einen der unten definierten Substituenten (a) aufweist, oder

$R^1$ und $R^2$ zusammen mit den diese Reste tragenden Kohlenstoffatomen einen an den Isoxazolring ankondensierten und insesamt 5 bis 7 Ringkohlenstoffatome besitzenden Kohlenwasserstoffring bilden, welcher unsubstituiert ist oder zumindest einen der unten definierten Substituenten (b) aufweist,

$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der unten definierten Substituenten (a), eine Phenylgruppe oder eine substituierte Phenylgruppe mit zumindest einem der unten definierten Substituenten (a) darstellen oder

$R^3$ und $R^4$ zusammen mit dem diese Reste tragenden Stickstoffatom eine alicyclische Aminogruppe mit insgesamt 5 oder 6 Ringatomen bilden, von welchen eines das erwähnte Stickstoffatom ist und 0 oder 1 ein weiteres von Stickstoff und/oder Sauerstoff und/oder Schwefel gebildetes Heteroatom ist, wobei diese alicyclische Aminogruppe unsubstituiert ist oder, falls ein weiteres Stickstoffatom als Heteroatom vorliegt, an diesem von Stickstoff gebildeten zusätzlichen Heteroatom zumindest einen der unten definierten Substituenten (b) aufweist, wobei

<u>die Substituenten (a)</u>

$C_1$-$C_3$-Alkylgruppen, $C_1$-$C_3$-Alkoxygruppen, Hydroxygruppen, Halogenatome, Nitrogruppen, Aminogruppen und von aliphatischen Carbonsäuren abgeleitete $C_2$-$C_4$-Acylgruppen sind und

<u>die Substituenten (b)</u>

$C_1$-$C_3$-Alkylgruppen, $C_1$-$C_3$-Alkoxygrupnen, Hydroxygruppen, Halogenatome, Nitrogruppen, Aminogruppen, von aliphatischen Carbonsäuren abgeleitete $C_2$-$C_4$-Acylaminogruppen, Benzylgruppen, substituierte Benzylgruppen mit zumindest einem der oben definierten Substituenten (a), Phenylgruppen und substituierte Phenylgruppen mit zumindest einem der oben definierten Substituenten (a) sind,

<u>mit der Maßgabe, daß,</u>

falls B für die erwähnte Gruppe der Formel (II) steht und n gleich ist O, $R^1$ nicht für ein Wasserstoffatom oder ein Halogenatom steht, oder ein pharmazeutisch annehmbares Salz hievon ist.

30.Verwendung nach Anspruch 29, worin die cerebro-aktive Substanz die Formel (Ib)

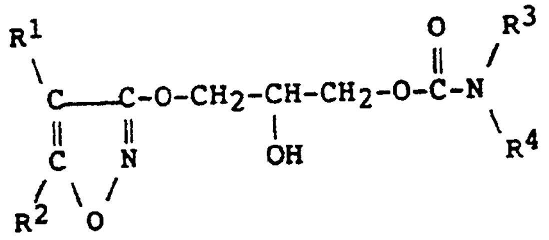

(Ib)

besitzt, in welcher

$R^1$ ein Wasserstoffatom, ein Halogenatom oder eine $C_1$-$C_4$-Alkylgruppe bedeutet,

$R^2$ eine Phenylgruppe oder eine Phenylgruppe mit zumindest einem der unten definierten Substituenten (c) darstellt und die

<u>Substituenten (c)</u>

$C_1$-$C_3$-Alkoxygruppen, Hydroxygruppen und Halogenatome sind, oder

$R^1$ und $R^2$ zusammen mit den diese Reste tragenden Kohlenstoffatomen einen an den Isoxazolring ankondensierten 6-gliedrigen Kohlenwasserstoffring bilden, welcher unsubstituiert ist oder zumindest ein Halogen als Substituenten aufweist, und

$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen oder

$R^3$, $R^4$ und das diese Reste tragende Stickstoffatom eine alicyclische Aminogruppe mit insgesamt 5 oder 6 Ringkohlenstoffatomen bilden, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

31. Verwendung nach Anspruch 29, worin die cerebro-aktive Substanz die Formel (Ib)

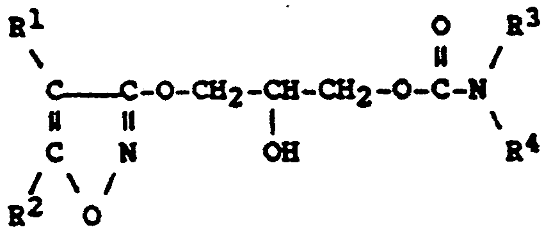

(Ib)

besitzt, in welcher

$R^1$ ein Wasserstoffatom, ein Chloratom oder eine $C_1$- oder $C_2$-Alkylgruppe bedeutet,

$R^2$ eine Phenylgruppe oder eine Phenylgruppe mit zumindest einem der unten definierten Substituenten (c') darstellt oder

$R^1$ und $R^2$ zusammen mit den diese Reste tragenden Kohlenstoffatomen einen an den Isoxazolring ankondensierten Benzolring bilden, welcher unsubstituiert ist oder ein Halogenatom als Substituenten aufweist, wobei die

Substituenten (c')

Methoxygruppen und Halogenatome sind,

$R^3$ und $R^4$ untereinander gleich oder voneinander verschieden sind und je für sich ein Wasserstoffatom, eine Methylgruppe oder eine Äthylgruppe darstellen oder

$R^3$, $R^4$ und das diese Reste tragende Stickstoffatom gemeinsam eine Morpholinogruppe, eine 1-Piperazinylgruppe, eine 4-Methyl-1-piperazinylgruppe, eine 1-Pyrrolidinylgruppe oder eine Piperidinogruppe bilden.

32. Verwendung nach Anspruch 29, worin die cerebro-aktive Substanz zumindest eine der Verbindungen
2-(2-Hydroxy-3-morpholinopropyl)-4-methyl-5-phenyl-3-isoxazolon,
3-(3- Carbamoyloxy-2-hydroxypropoxy)-5-(m-chlorphenyl)-isoxazol,
3-(2-Hydroxy-3-morpholinopropoxy)-5-phenylisoxazol,
3-(2-Hydroxy-3-morpholinopropoxy)-4-methyl-5-phenylisoxazol,
3-(3-Carbamoyloxy-2-hydroxypropoxy)-5-chlor-1,2-benzisoxazol
oder ein pharmazeutisch annehmbares Salz hievon ist.

33. Verwendung eines Wirkstoffes zum Herstellen eines Heilmittels zur Entfaltung zentral wirkender Muskelrelaxationswirkung, worin der Wirkstoff eine Verbindung der Formel (1) oder ein pharmazeutisch annehmbares Salz hievon der in Anspruch 29 beanspruchten Art ist.

34. Verwendung nach Anspruch 33, worin der Wirkstoff die Formel (Ib)

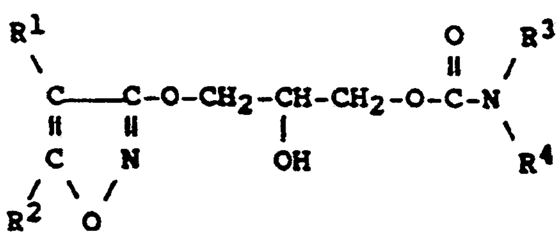

(Ib)

besitzt, in welcher

$R^1$ ein Wasserstoffatom, ein Halogenatom oder eine $C_1$-$C_4$-Alkylgruppe bedeutet,

$R^2$ eine Phenylgruppe oder eine Phenylgruppe mit zumindest einem der unten definierten Substituenten (c) darstellt und hiebei die

Substituenten (c)

$C_1$-$C_3$-Alkoxygruppen, Hydroxygruppen und Halogenatome sind oder

$R^1$ und $R^2$ zusammen mit den diese Reste tragenden Kohlenstoffatomen einen an den Isoxazolring ankondensierten 6-gliedrigen Kohlenwasserstoffring bilden, welcher unsubstituiert ist oder zumindest ein Halogen als Substituenten aufweist, und

$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen oder

$R^3$, $R^4$ und das diese Reste tragende Stickstoffatom zusammen eine alicyclische Aminogruppe mit insgesamt

5 oder 6 Ringatomen darstellen, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

35. Verwendung nach Anspruch 33, worin der Wirkstoff die Formel (Ib)

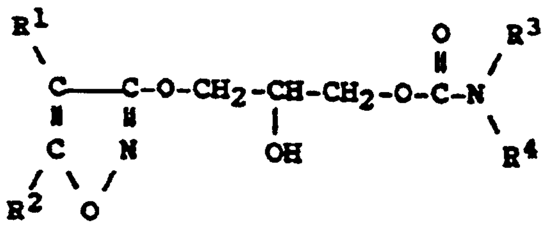

(Ib)

besitzt, in welcher

$R^1$ ein Wasserstoffatom, ein Chloratom oder eine $C_1$- oder $C_2$-Alkylgruppe bedeutet,

$R^2$ eine Phenylgruppe oder eine Phenylgruppe mit zumindest einem der unten definierten Substituenten (c') darstellt oder

$R^1$ und $R^2$ zusammen mit den diese Reste tragenden Kohlenstoffatomen einen an den Isoxazolring ankondensierten Benzolring darstellen, welcher unsubstituiert ist oder ein Halogen als Substituenten aufweist und hiebei die

Substituenten (c')

Methoxygruppen und Halogenatome sind,

$R^3$ und $R^4$ untereinander gleich oder voneinander verschieden sind und je für sich ein Wasserstoffatom, eine Methylgruppe oder eine Äthylgruppe darstellen oder

$R^3$, $R^4$ und das diese Reste tragende Stickstoffatom gemeinsam eine Morpholinogruppe, eine 1-Piperazinylgruppe, eine 4-Methyl-1-piperazinylgruppe, eine 1-Pyrrolidinylgruppe oder eine Piperidinogruppe bilden.

36. Verwendung nach Anspruch 33, worin der Wirkstoff zumindest eine der Verbindungen

2-(2-Hydroxy-3-morpholinopropyl)-4-methyl-5-phenyl-3-isoxazolon,
3-(3-Carbamoyloxy-2-hydroxypropoxy)-5-(m-chlorphenyl)-isoxazol,
3-(2-Hydroxy-3-morpholinopropoxy)-5-phenylisoxazol,
3-(2-Hydroxy-3-morpholinopropoxy)-4-methyl-5-phenyl-isoxazol,
3-(3-Carbamoyloxy-2-hydroxypropoxy)-5-chlor-1,2-benzisoxazol
oder ein pharmazeutisch annehmbares Salz hievon ist.

37. Pharmazeutische Mischung für die Behandlung von cerebro-vasculären Störungen oder zur Verwendung als zentralwirkendes Muskelrelaxans, welche Mischung einen Wirkstoff im Gemisch mit einem pharmazeutisch annehmbaren Träger oder Verdünnungsmittel enthält, worin der Wirkstoff eine in Anspruch 29 definierte Verbindung der Formel (I) oder ein pharmazeutisch annehmbares Salz hievon ist.

38. Mischung nach Anspruch 37, worin der Wirkstoff die Formel (Ib)

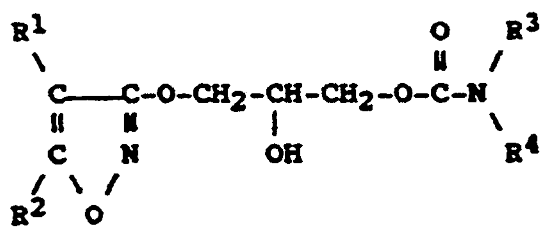

(Ib)

besitzt, in welcher

$R^1$ ein Wasserstoffatom, ein Halogenatom oder eine $C_1$-$C_4$-Alkylgruppe bedeutet,

$R^2$ eine Phenylgruppe oder eine substituierte Phenylgruppe mit zumindest einem der unten definierten Substituenten (c) darstellt und hiebei die

Substituenten (c)

$C_1$-$C_3$-Alkoxygruppen, Hydroxygruppen und Halogenatome sind oder

$R^1$ und $R^2$ zusammen mit den diese Reste tragenden Kohlenstoffatomen einen an den Isoxazolring ankondensierten 6-gliedrigen Kohlenwasserstoffring bilden, welcher unsubstituiert ist oder zumindest ein Halogen als Substituenten aufweist, und

$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen oder

$R^3$, $R^4$ und das diese Reste tragende Stickstoffatom zusammen eine alicyclische Aminogruppe mit insgesamt 5 oder 6 Ringatomen darstellen, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

39. Mischung nach Anspruch 37, worin der Wirkstoff die Formel (Ib)

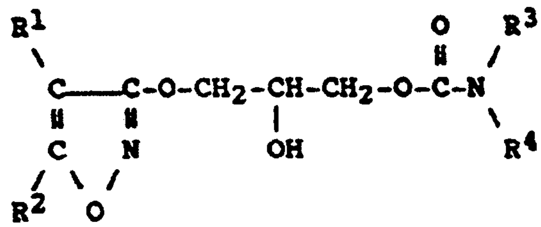

(Ib)

besitzt, in welcher

$R^1$ ein Wasserstoffatom, ein Chloratom oder eine $C_1$- oder $C_2$-Alkylgruppe bedeutet,

$R^2$ eine Phenylgruppe oder eine Phenylgruppe mit zumindest einem der unten definierten Substituenten (c') darstellt oder

$R^1$ und $R^2$ zusammen mit den diese Reste tragenden Kohlenstoffatomen einen an den Isoxazolring ankondensierten Benzolring bilden, welcher unsubstituiert ist oder ein Halogen als Substituenten aufweist, und hiebei die

Substituenten (c')

Methoxygruppen und Halogenatome sind,

$R^3$ und $R^4$ untereinander gleich oder voneinander verschieden sind und je für sich ein Wasserstoffatom, eine Methylgruppe oder eine Äthylgruppe darstellen oder

$R^3$, $R^4$ und das diese Reste tragende Stickstoffatom gemeinsam eine Morpholinogruppe, eine 1-Piperazinylgruppe, eine 4-Methyl-1-piperazinylgruppe, eine 1-Pyrrolidinylgruppe oder eine Piperidinogruppe bilden.

40. Mischung nach Anspruch 37, worin der Wirkstoff zumindest eine der Verbindungen

2-(2-Hydroxy-3-morpholinopropyl)-4-methyl-5-phenyl-3-isoxazolon,

3-(3-Carbamoyloxy-2-hydroxypropoxy)-5-(m-chlorphenyl)-isoxazol,

3-(2-Hydroxy-3-morpholinopropoxy)-5-phenylisoxazol,

3-(2-Hydroxy-3-morpholinopropoxy)-4-methyl-5-phenylisoxazol

3-(3-Carbamoyloxy-2-hydroxypropoxy)-5-chlor-1,2-benzisoxazol

oder ein pharmazeutisch annehmbares Salz hievon ist.

41. Verfahren zum Herstellen einer Verbindung gemäß irgendeinem der Ansprüche 1 bis 28, bei welchem

(i) eine Verbindung der Formel (III)

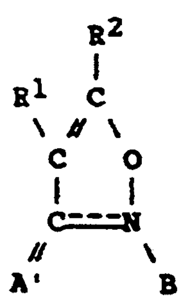

(III)

[in welcher entweder

(a) die strichlierte Linie (- - -) für eine Einfachbindung steht,

A' = für ein Sauerstoffatom steht und

B' eine Gruppe der Formel (IV)

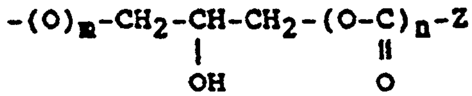

(IV)

mit der Bedeutung von 0 für m und der Bedeutung von 0 oder 1 für n darstellt oder

(b) die strichlierte Linie (- - -) eine Doppelbindung bedeutet,

A' = die erwähnte Gruppe der Formel (IV) mit der Bedeutung von 1 für m und der Bedeutung von 0 oder 1 für n darstellt und

B' fehlt,

Z eine abspaltbare Gruppe bedeutet und

$R^1$ und $R^2$ die in Anspruch 1 angegebene Bedeutung besitzen mit einem Amin der Formel (V)

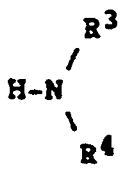

(V)

(in welcher $R^3$ und $R^4$ die in Anspruch 1 angegebene Bedeutung besitzen) umgesetzt wird und

(ii) erforderlichenfalls das erhaltene Produkt in ein Salz übergeführt wird.

**Patentansprüche für folgenden Vertragsstaaten : ES, GR**

1. Verfahren zum Herstellen einer Verbindung der Formel (I)

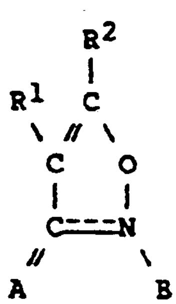

(I)

in welcher entweder

(a) die strichlierte Linie (- - -) eine Doppelbindung bedeutet,

A = für ein Sauerstoffatom steht und

B für eine Gruppe der Formel (II)

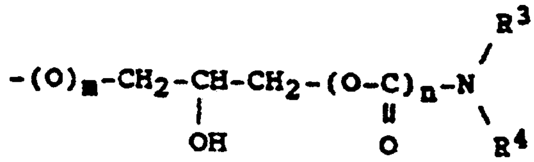

(II)

mit der Bedeutung von 0 für m und der Bedeutung von 0 oder 1 für n steht oder
(b) die strichlierte Linie (---) eine Doppelbindung bedeutet,

A = die erwähnte Gruppe der Formel (II) mit der Bedeutung von 1 für m und der Bedeutung von 0 oder 1 für n darstellt und
B nicht vorhanden ist und

$R^1$ ein Wasserstoffatom, ein Halogenatom, eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe mit zumindest einem der unten definierten Substituenten (a), eine Phenylgruppe oder eine substituierte Phenylgrupne mit zumindest einem der unten definierten Substituenten (a) bedeutet,
$R^2$ ein Wasserstoffatom, ein Halogenatom, eine $C_1$-$C_4$-Alkylgruppe, eine Phenylgruppe, eine substituierte Phenylgruppe mit zumindest einem der unten definierten Substituenten (a) oder eine heterocyclische Gruppe mit 5 oder 6 Ringatomen, von welchen 1 bis 3 von Stickstoff- und/oder Sauerstoff- und/oder Schwefelatomen gebildeten Heteroatomen sind, darstellt, wobei die heterocyclische Gruppe unsubstituiert ist oder zumindest einen der unten definierten Substituenten (a) aufweist, oder
$R^1$ und $R^2$ zusammen mit den diese Reste tragenden Kohlenstoffatomen einen an den Isoxazolring ankondensierten und insesamt 5 bis 7 Ringkohlenstoffatome besitzenden Kohlenwasserstoffring bilden, welcher unsubstituiert ist oder zumindest einen der unten definierten Substituenten (b) aufweist,
$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der unten definierten Substituenten (a), eine Phenylgruppe oder eine substituierte Phenylgruppe mit zumindest einem der unten definierten Substituenten (a) darstellen oder
$R^3$ und $R^4$ zusammen mit dem diese Reste tragenden Stickstoffatom eine alicyclische Aminogruppe mit insgesamt 5 oder 6 Ringatomen bilden, von welchen eines das erwähnte Stickstoffatom ist und 0 oder 1 ein weiteres von Stickstoff und/oder Sauerstoff und/oder Schwefel gebildetes Heteroatom ist, wobei diese alicyclische Aminogruppe unsubstituiert ist oder, falls ein weiteres Stickstoffatom als Heteroatom vorliegt, an diesem von Stickstoff gebildeten zusätzlichen Heteroatom zumindest einen der unten definierten Substituenten (b) aufweist, wobei

die Substituenten (a)
$C_1$-$C_3$-Alkylgruppen, $C_1$-$C_3$-Alkoxygruppen, Hydroxygruppen, Halogenatome, Nitrogruppen, Aminogruppen und von aliphatischen Carbonsäuren abgeleitete $C_2$-$C_4$-Acylgruppen sind und
die Substituenten (b)
$C_1$-$C_3$-Alkylgruppen, $C_1$-$C_3$-Alkoxygruppen, Hydroxygruppen, Halogenatome, Nitrogruppen, Aminogruppen, von aliphatischen Carbonsäuren abgeleitete $C_2$-$C_4$-Acylaminogruppen, Benzylgruppen, substituierte Benzylgruppen mit zumindest einem der oben definierten Substituenten (a), Phenylgrupper und substituierte Phenylgruppen mit zumindest einem der oben definierten Substituenten (a) sind,
mit der Maßgabe, daß
(i) falls B für die erwähnte Gruppe der Formel (II) steht und n gleich ist O, $R^1$ nicht für ein Wasserstoffatom oder ein Halogenatom steht,
(ii) falls A für die erwähnte Gruppe der Formel (II) steht und n gleich ist O, $R^1$ und $R^2$ zusammen einen an den Isoxazolring ankondensierten Benzolring bilden und, falls $R^4$ eine Alkylgruppe bedeutet, $R^3$ nicht für ein Wasserstoffatom steht, und
(iii) falls (---) für eine Einfachbindung steht und $R^1$ und $R^2$ zusammen mit den diese Reste tragenden Kohlenstoffatomen einen $C_6$-$C_7$-Kohlenwasserstoffring bilden, n gleich ist 1,
und pharmazeutisch annehmbare Salze hievon, jedoch
unter Auschluß
der Verbindung 5-Chlor-2-(3-piperidino-2-hydroxy)propylbenzisoxazolin-3-on,
bei welchem Verfahren
(i) eine Verbindung der Formel (III)

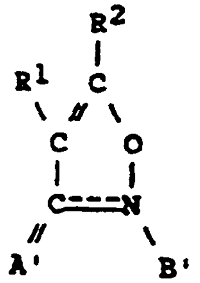

(III)

[in welcher entweder
(a) die strichlierte Linie (- - -) für eine Einfachbindung steht,

A' = für ein Sauerstoffatom steht und
B' eine Gruppe der Formel (IV)

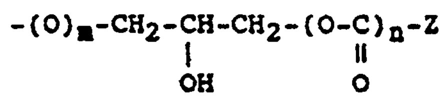

(IV)

mit der Bedeutung von 0 für m und der Bedeutung von 0 oder 1 für n darstellt oder
(b) die strichlierte Linie (- - -) eine Doppelbindung bedeutet,

A' = die erwähnte Gruppe der Formel (IV) mit der Bedeutung von 1 für m und der Bedeutung von 0 oder 1 für n darstellt und
B' fehlt,

Z eine abspaltbare Gruppe bedeutet und
$R^1$ und $R^2$ die oben angegebene Bedeutung besitzen]
mit einem Amin der Formel (V)

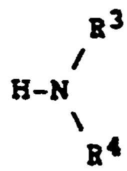

(V)

(in welcher $R^3$ und $R^4$ die oben angegebene Bedeutung besitzen) angesetzt wird und
(ii) erforderlichenfalls das erhaltene Produkt in ein Salz übergeführt wird.

2. Verfahren nach Anspruch 1, bei welchem die Reaktionsteilnehmer und Umsetzungsbedingungen so gewählt werden, daß eine Verbindung der Formel (Ia)

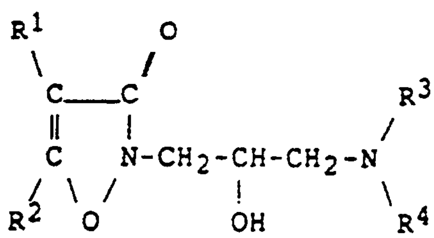

(Ia)

in welcher
$R^1$ eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe, eine Phenylgruppe oder eine substituierte Phenylgruppe darstellt und hiebei die Substituenten aus den in Anspruch 1 definierten Substituenten (a) ausgewählt sind,
$R^2$ ein Wasserstoffatom, eine $C_1$-$C_4$-Alkylgruppe, eine Phenylgruppe, eine substituierte Phenylgruppe oder eine in Anspruch 1 definierte heterocyclische Gruppe bedeutet und

$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe, eine Phenylgruppe oder eine substituierte Phenylgruppe bedeuten oder $R^3$ und $R^4$ zusammen mit dem diese Reste tragenden Stickstoffatom eine in Anspruch 1 definierte alicyclische Aminogruppe bilden,
oder ein pharmazeutisch annehmbares Salz hievon hergestellt wird.

3. Verfahren nach Anspruch 1, bei welchem die Reaktionsteilnehmer und Umsetzungsbedingungen so gewählt werden, daß eine Verbindung der formel (Ib)

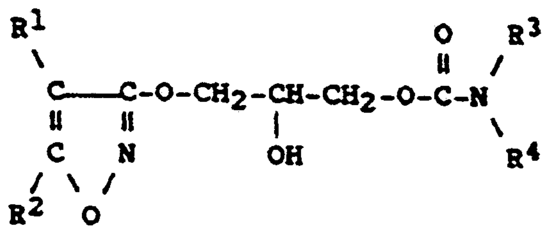

(Ib)

in welcher
$R^1$ ein Wasserstoffatom, ein Halogenatom, eine $C_1$-$C_4$-Alkylgruppe, eine $C_2$-$C_4$-Alkenylgruppe, eine $C_2$-$C_4$-Alkinylgruppe, eine Benzylgruppe, eine substituierte_Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeutet,
$R^2$ ein Wasserstoffatom, eine $C_1$-$C_4$-Alkylgruppe, eine Phenylgruppe, eine substituierte Phenylgruppe mit zumindest einem der in Anspruch 1 definierten Substituenten (a) oder eine in Anspruch 1 definierte heterocyclische Gruppe darstellt oder
$R^1$ und $R^2$ zusammen mit den diese Reste tragenden Kohlenstoffatomen einen in Anspruch 1 definierten und an den Isoxazolring ankondensierten Kohlenwasserstoffring bilden,
$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeuten oder
$R^3$, $R^4$ und das diese Reste tragende Stickstoffatom zusammen eine in Anspruch 1 definierte alicyclische Aminogruppe bilden, oder ein pharmazeutisch annehmbares Salz hievon hergestellt wird.

4. Verfahren nach Anspruch 1, bei welchem die Reaktionsteilnehmer und Umsetzungsbedingungen so gewählt werden, daß eine Verbindung der Formel (Ic)

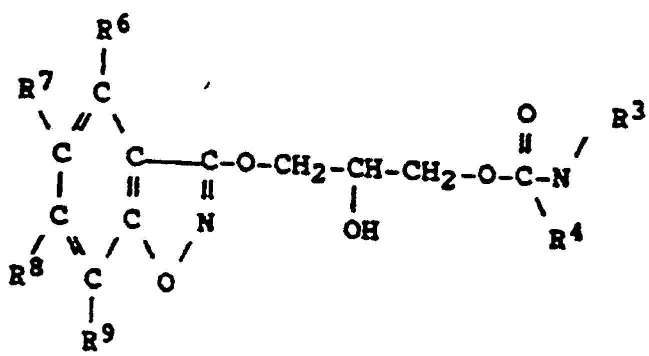

(Ic)

in welcher
$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) darstellen oder
$R^3$, $R^4$ und das diese Reste tragende Stickstoffatom gemeinsam eine in Anspruch 1 definierte alicyclische Aminogruppe bilden und
einer der Reste $R^6$, $R^7$, $R^8$ und $R^9$ ein Wasserstoffatom, ein Halogenatom, eine $C_1$-$C_4$-Alkylgruppe oder eine $C_1$-$C_4$-Alkoxygruppe bedeutet und die anderen Reste durchwegs für Wasserstoffatome stehen, oder ein pharmazeutisch annehmbares Salz hievon hergestellt wird.

5. Verfahren nach Anspruch 1, bei welchem die Reaktionsteilnehmer und Umsetzungsbedingungen so gewählt werden, daß eine Verbindung der Formel (Id)

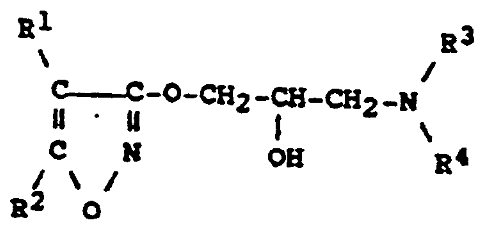

(Id)

in welcher
$R^1$ ein Wasserstoffatom, ein Halogenatom, eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeutet,
$R^2$ ein Wasserstoffatom, eine $C_1$-$C_4$-Alkylgruppe, eine Phenylgruppe, eine substituierte Phenylgruppe mit zumindest einem der in Anspruch 1 definierten Substituenten (a) oder eine in Anspruch 1 definierte heterocyclische Gruppe darstellt oder
$R^1$ und $R^2$ zusammen mit den diese Reste tragenden Kohlenstoffatomen einen in Anspruch 1 definierten und an den Isoxazolring ankondensierten Kohlenwasserstoffring bilden,
$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeuten oder
$R^3$, $R^4$ und das diese Reste tragende Stickstoffatom eine in Anspruch 1 definierte alicyclische Aminogruppe bilden, mit der Maßgabe, daß, falls $R^1$ und $R^2$ gemeinsam einen an den Isoxazolring ankondensierten Benzolring bilder und $R^4$ für eine Alkylgruppe steht, $R^3$ nicht für ein Wasserstoffatom steht, oder ein pharmazeutisch annehmbares Salz hievon hergestellt wird.

6. Verfahren nach Anspruch 1, bei welchem die Reaktionsteilnehmer und Umsetzungsbedingungen so gewählt werden, daß eine Verbindung der Formel (Ie)

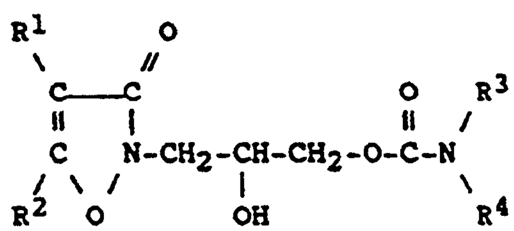

(Ie)

in welcher
$R^1$ ein Wasserstoffatom, ein Halogenatom, eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeutet,
$R^2$ ein Wasserstoffatom, eine $C_1$-$C_4$-Alkylgruppe, eine Phenylgruppe, eine substituierte Phenylgruppe mit zumindest einem der in Anspruch 1 definierten Substituenten (a) oder eine in Anspruch 1 definierte heterocyclische Gruppe darstellt oder
$R^1$ und $R^2$ zusammen mit den diese Reste tragenden Kohlenstoffatomen einen in Anspruch 1 definierten und an den Isoxazolring ankondensierten Kohlenwasserstoffring bilden,
$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindet einem der in Anspruch 1 definierten Substituenten (a) darstellt oder
$R^3$, $R^4$ und das diese Reste tragende Stickstoffatom gemeinsam eine in Anspruch 1 definierte alicyclische Aminogruppe bilden oder ein pharmazeutisch annehmbares Salz hievon hergestellt wird.

7. Verfahren nach Anspruch 1, bei welchem die Reaktionsteilnehmer und Umsetzungsbedingungen so gewählt werden, daß eine Verbindung der Formel (If)

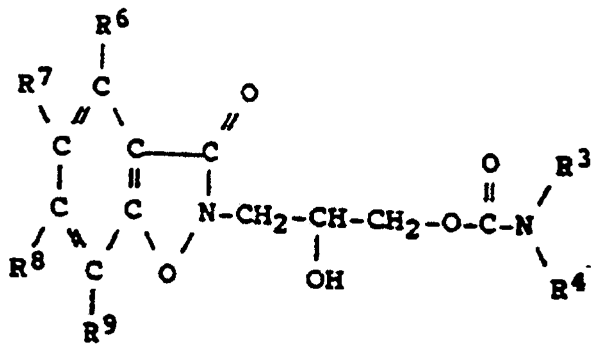

(If)

in welcher
$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der in Anspruch 1 definierten Substituenten (a), eine Phenylgruppe oder eine substituierte Phenylgruppe mit zumindest einem der in Anspruch 1 definierten Substituenten (a) bedeuten oder
$R^3$, $R^4$ und das diese Reste tragende Stickstoffatom gemeinsam eine in Anspruch 1 definierte alicyclische Aminogruppe bilden und
einer der Reste $R^6$, $R^7$, $R^8$ und $R^9$ ein Wassertoffatom, ein Halogenatom, eine $C_1$-$C_4$-Alkylgruppe oder eine $C_1$-$C_4$-Alkoxygruppe bedeutet und die anderen Reste durchwegs für Wasserstoffatome stehen, oder ein pharmazeutisch annehmbares Salz hievon hergestellt wird.

8. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin $R^1$ ein Wasserstoffatom, ein Halogenatom oder eine $C_1$-$C_4$-Alkylgruppe bedeutet.

9. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin $R^2$ eine Phenylgruppe oder eine substituierte Phenylgruppe mit zumindest einem der unten definierten Substituenten (c) darstellt und die

Substituenten (c)

$C_1$-$C_3$-Alkoxygruppen, Hydroxygruppen und Halogenatome sind.

10. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin $R^1$ und $R^2$ zusammen mit den diese Reste tragenden Kohlenstoffatomen einen an den Isoxazolring ankondensierten 6-gliedrigen Kohlenwasserstoffring bilden, welcher unsubstituiert ist oder zumindest ein Halogenatom aufweist.

11. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin $R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen.

12. Verfahren nach Anspruch 1, bei welchem die Reaktionsteilnehmer und Umsetzungsbedinguncen so gewählt werden, daß eine Verbindung der Formel (I) oder ein Salz hievon hergestellt wird, worin $R^3$, $R^4$ und das diese Reste tragende Stickstoffatam eine alicyclische Aminogruppe mit insgesamt 5 oder 6 Ringatomen bilden, von welchen eines das erwähnte Stickstoffatom ist und 0 oder 1 ein von zusätzlichem Stickstoff gebildetes Heteroatom ist, wobei diese alicyclische Aminogruppe unsubstituiert ist oder, falls ein zusätzliches Stickstoffatom als Heteroatom vorliegt, zumindest ein Substituent an diesem zusätzlichen von Stickstoff gebildetem Heteroatom aus den folgenden Substituenten (d) ausgewählt ist und die

Substituenten (d)

$C_1$-$C_3$-Alkylgruppen, Benzylgruppen und Phenylgruppen sind.

13. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin $R^1$ ein Wasserstoffatom, ein Chloratom oder eine $C_1$- oder $C_2$-Alkylgruppe ist.

14. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin $R^2$ eine Phenylgruppe oder eine substituierte Phenylgruppe mit zumindest einem der unten definierten Substituenten (c') ist und die

Substituenten (c')

Methoxygruppen und Halogenatome sind.

15. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin $R^1$ und $R^2$ zusammen mit den diese Reste tragenden Kohlenstoffatomer einen an den Isoxazolring ankondensierten Benzolring bilden, welcher unsubstituiert ist oder einen aus Halogenatomen ausgewählten Substituenten aufweist.

16. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin $R^3$ und $R^4$ untereinander gleich oder voneinander verschieden sind und je für sich ein Wasserstoffatom, eine Methylgruppe oder eine Äthylgruppe bedeuten.

17. Verfahren nach Anspruch 1, bei welchem die Reaktionsteilnehmer und Umsetzungsgsbedingungen so gewählt werden, daß eine Verbindung der Formel (I) oder ein Salz hievon hergestellt wird, worin $R^3$, $R^4$ und das diese Reste tragende Stickstoffatom gemeinsam eine Morpholinogruppe, eine 1-Piperazinylgruppe, eine 4-Methyl-1-piperaztnylgruppe, eine 1-Pyrrolidinylgruppe oder eine Piperidinogruppe darstellen.

18. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin

$R^1$ ein Wasserstoffatom, ein Halogenatom oder eine $C_1$-$C_4$-Alkylgruppe bedeutet,

$R^2$ eine Phenylgruppe oder eine substituierte Phenylgruppe mit zumindest einem der unten definierten Substituenten (c) darstellt und die

Substituenten (c)

$C_1$-$C_3$-Alkoxygruppen, Hydroxygruppen und Halogenatome sind,

$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe bedeuten oder

$R^3$, $R^4$ und das diese Reste tragende Stickstoffatom eine alicyclische Aminogruppe mit insgesamt 5 oder 6 Ringatomen bilden, von welchen eines das erwähnte Stickstoffatom und 0 oder 1 ein von einem weiteren Stickstoffatam gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

19. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin

$R^1$ und $R^2$ zusammen mit den diese Reste tragenden Stickstoffatomen einen an den Isoxazolring ankondensierten 6-gliedrigen Kohlenwasserstoffring bilden, welcher unsubstituiert ist oder zumindest einen von Halogenatomen gebildeten Substituenten aufweist,

$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen oder

$R^3$, $R^4$ und das diese Reste tragende Stickstoffatom gemeinsam eine alicyclische Aminogruppe mit insgesamt 5 oder 6 Ringatomen bilden, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

20. Verfahren nach Anspruch 1, bei welchem die Reaktionsteilnehmer und Umsetzungsbedingungen so gewählt werden, daß eine Verbindung der Formel (I) oder ein Salz hievon hergestellt wird, worin

$R^1$1 ein Wasserstoffatom, ein Chloratom oder eine $C_1$- oder $C_2$-Alkylgruppe bedeutet,

$R^2$ eine Phenylgruppe oder eine substituierte Phenylgruppe mit zumindest einem der unten definierten Substituenten (c') darstellt und die

Substituenten (c')

Methoxygruppen und Halogenatome sind,

$R^3$ und $R^4$ untereinander gleich oder voneinander verschieden sind und je für sich ein Wasserstoffatom, eine Methylgruppe oder eine Äthylgruppe darstellen oder

$R^3$, $R^4$ und das diese Reste tragende Stickstoffatom zusammen eine Morpholinogruppe, eine 1-Piperazinylgruppe, eine 4-Methyl-1-piperazinylgruppe, eine 1-Pyrrolidinylgruppe oder eine Piperidinogruppe darstellenn.

21. Verfahren nach Anspruch 1, bei welchem die Reaktionsteilnehmer und Umsetzungsbedingungen so

gewählt werden, daß eine Verbindung der Formel (I) oder ein Salz hievon hergestellt wird, worin
$R^1$ und $R^2$ zusammen mit den diese Reste tragenden Kohlenstoffatomen einen an den Isoxazolring ankondensierten Benzolring bilden, welcher unsubstituiert ist oder ein Halogenatom als Substituenten aufweist,
$R^3$ und $R^4$ untereinander gleich oder voneinander verschieden sind und je für sich ein Wasserstoffatom, eine Methylgruppe oder eine Äthylgruppe bedeuten oder
$R^3$, $R^4$ und das diese Reste tragende Stickstoffatom eine Morpholinogruppe, eine 1-Piperazinylgruppe, eine 4-Methyl-1-piperazinylgruppe, eine 1-Pyrrolidinylgruppe oder eine Piperidinogruppe bedeuten.

22. Verfahren nach Anspruch 3, bei welchem die Reaktionsteilnehmer und Umsetzungsbedingungen so gewillt werden, daß eine Verbindung der Formel (Ib)

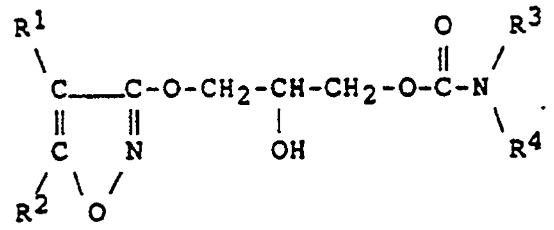

(Ib)

in welcher
$R^1$ ein Wasserstoffatom, ein Halogenatom oder eine $C_1$-$C_4$-Alkylgruppe bedeutet;
$R^2$ eine Phenylgruppe oder eine substituierte Phenylgruppe mit zumindest einem der unten definierten Substituenten (c) darstellt und die
Substituenten (c)
$C_1$-$C_3$-Alkoxygruppen, Hydroxygruppen und Halogenatome sind oder
$R^1$ und $R^2$ zusammen mit den diese Reste tragenden Kohlenstoffatomen einen an den Isoxazolring ankondensierten 6-gliedrigen Kohlenwasserstoffring darstellen, welcher unsubstituiert ist oder zumindest ein Halogen als Substituenten aufweist und
$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen oder
$R^3$, $R^4$ und das diese Reste tragende Stickstoffatom eine alicyclische Aminogruppe mit insgesamt 5 oder 6 Ringatomen bilden, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die
Substituenten (d)
$C_1$-$C_3$-Alkylgruppen, Benzylgruppen und Phenylgruppen sind, oder ein pharmazeutisch annehmbares Salz hievon hergestellt wird.

23. Verfahren nach Anspruch 22, bei welchem die Reaktionsteilnehmer und Umsetzungsbedingungen so gewählt werden, daß eine Verbindung der Formel (Ib)

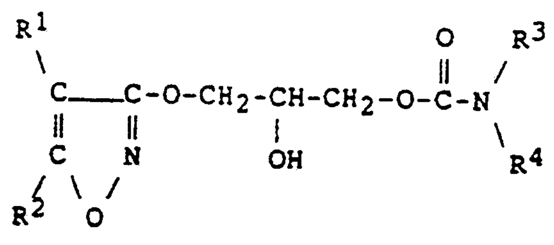

(Ib)

in welcher
$R^1$ ein Wasserstoffatom, ein Chloratom oder eine $C_1$- oder $C_2$-Alkylgruppe bedeutet,
$R^2$ eine Phenylgruppe oder eine substituierte Phenylgruppe mit zumindest einem der unten definierten Substituenten (c') darstellt oder
$R^1$ und $R^2$ zusammen mit den diese Reste tragenden Kohlenstoffatomen einen an den Isoxazolring ankondensierten Benzolring darstellen, welcher unsubstituiert ist oder ein Halogen als Substituenten aufweist, und hiebei die
Substituenten (c')
Methoxygruppen und Halogenatome sind, und

$R^3$ und $R^4$ untereinander gleich oder voneinander verschieden sind und je für sich Wasserstoffatome, Methylgruppen und Äthylgruppen darstellen oder
$R^3$, $R^4$ und das diese Reste tragende Stickstoffatom zusammen eine Morpholinogruppe, eine 1-Piperazinylgruppe, eine 4-Methyl-1-piperazinylgruppe, eine 1-Pyrrolidinylgruppe oder eine Piperidinogruppe darstellen, oder ein pharmazeutisch annehmbares Salz hievon hergestellt wird.

24. Verfahren nach Anspruch 1, bei welchem die Reaktionsteilnehmer und Umsetzungsbedingungen so gewählt werden, daß
2-(2-Hydroxy-3-morpholinopropyl)-4-methyl-5-phenyl-3-isoxazolon,
3-(3-Carbamoyloxy-2-hydroxypropoxy)-5-(m-chlorphenyl)-isoxazol,
3-(2-Hydroxy-3-morpholinopropoxy)-5-phenylisoxazol,
3-(2-Hydroxy-3-morpholinopropoxy)-4-methyl-5-phenyl-isoxazol
3-(3-Carbamoyloxy-2-hydroxypropoxy)-5-chlor-1,2-benzisoxazol
oder ein pharmazeutisch annehmbares Salz hievon hergestellt wird.

25. Verfahren zum Herstellen einer pharmazeutischen Mischung zum Behandeln einer cerebrovasculären Störung in einem an cerebrovasculären Störungen leidenden oder hiefür anfälligen Säugetier oder für die Herbeiführung einer zentral wirkenden Muskelrelaxationswirkung, bei welchem Verfahren zumindest eine cerebro-aktive Substanz mit einem pharmazeutisch annehmbaren Träger oder Verdünnungsmittel vermischt wird, worin die cerebro-aktive Substanz eine Verbindung der Formel (I)

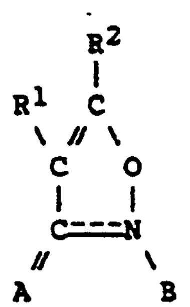

(I)

in welcher entweder

(a) die strichlierte Linie (- - -) eine Doppelbindung bedeutet,

A = für ein Sauerstoffatom steht und
B für eine Gruppe der Formel (II)

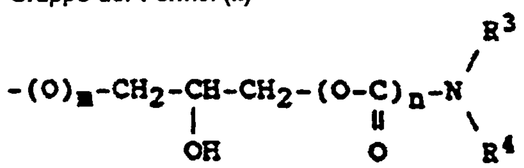

(II)

mit der Bedeutung von 0 für m und der Bedeutung von 0 oder 1 für n steht oder

(b) die strichlierte Linie (- - -) eine Doppelbindung bedeutet,

A = die erwähnte Gruppe der Formel (II) mit der Bedeutung von 1 für m und der Bedeutung von 0 oder 1 für n darstellt und
B nicht vorhanden ist und

$R^1$ ein Wasserstoffatom, ein Halogenatom, eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe mit zumindest einem der unten definierten Substituenten (a), eine Phenylgruppe oder eine substituierte Phenylgruppe mit zumindest einem der unten definierten Substituenten (a) bedeutet,
$R^2$ ein Wasserstoffatom, ein Halogenatom, eine $C_1$-$C_4$-Alkylgruppe, eine Phenylgruppe, eine substituierte Phenylgruppe mit zumindest einem der unten definierten Substituenten (a) oder eine heterocyclische Gruppe mit 5 oder 6 Ringatomen, von welchen 1 bis 3 von Stickstoff- und/oder Sauerstoff- und/oder Schwefelatomen gebildeten Heteroatomen sind, darstellt, wobei die heterocyclische Gruppe unsubstituiert ist oder zumindest einen der unten definierten Substituenten (a) aufweist, oder

$R^1$ und $R^2$ zusammen mit den diese Reste tragenden Kohlenstoffatomen einen an den Isoxazolring ankondensierten und insesamt 5 bis 7 Ringkohlenstoffatome besitzenden Kohlenwasserstoffring bilden, welcher unsubstituiert ist oder zumindest einen der unten definierten Substituenten (b) aufweist, $R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der unten definierten Substituenten (a), eine Phenylgruppe oder eine substituierte Phenylgruppe mit zumindest einem der unten definierten Substituenten (a) darstellen oder

$R^3$ und $R^4$ zusammen mit dem diese Reste tragenden Stickstoffatom eine alicyclische Aminogruppe mit insgesamt 5 oder 6 Ringatomen bilden, von welchen eines das erwähnte Stickstoffatom ist und 0 oder 1 ein weiteres von Stickstoff und/oder Sauerstoff und/oder Schwefel gebildetes Heteroatom ist, wobei diese alicyclische Aminogruppe unsubstituiert ist oder, falls ein weiteres Stickstoffatom als Heteroatom vorliegt, an diesem von Stickstoff gebildeten zusätzlichen Heteroatom zumindest einen der unten definierten Substituenten (b) aufweist, wobei

die Substituenten (a)

$C_1$-$C_3$-Alkylgruppen, $C_1$-$C_3$-Alkoxygruppen, Hydroxygruppen, Halogenatome, Nitrogruppen, Aminogruppen und von allphatischen Carbonsäuren abgeleitete $C_2$-$C_4$-Acylgruppen sind und

die Substituenten (b)

$C_1$-$C_3$-Alkylgruppen, $C_1$-$C_3$-Alkoxygruppen, Hydroxygruppen, Halogenatome, Nitrogruppen, Aminogruppen, von aliphatischen Carbonsäuren abgeleitete $C_2$-$C_4$-Acylaminogruppen, Benzylgruppen, substituierte Benzylgruppen mit zumindest einem der oben definierten Substituenten (a), Phenylgruppen und substituierte Phenylgruppen mit zumindest einem der oben definierten Substituenten (a) sind,

mit der Maßgabe, daß

(i) falls B für die erwähnte Gruppe der Formel (II) steht und n gleich ist O, $R^1$ nicht für ein Wasserstoffatom oder ein Halogenatom steht, und

(ii) falls (- - -) für eine Einfachbindung steht und $R^1$ und $R^2$ zusammen mit den diese Reste tragenden Kohlenstoffatomen einen $C_6$-$C_7$-Kohlenwasserstoffring bilden, n gleich ist 1, oder ein pharmazeutisch annehmbares Salz hievon ist.

26. Verfahren nach Anspruch 25, worin die cerebro-aktive Suhstanz die Formel (Ib)

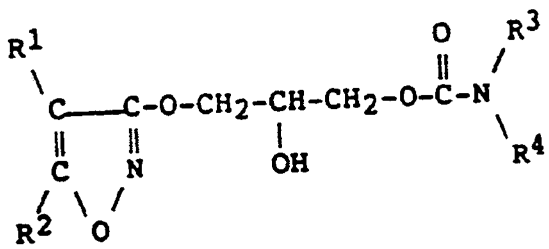

(Ib)

besitzt, in welcher

$R^1$ ein Wasserstoffatom, ein Halogenatom oder eine $C_1$-$C_4$-Alkylgruppe bedeutet,

$R^2$ eine Phenylgruppe oder eine Phenylgruppe mit zumindest einem der unten definierten Substituenten (c) darstellt und die

Substituenten (c)

$C_1$-$C_3$-Alkoxygruppen, Hydroxygruppen und Halogenatome sind, oder

$R^1$ und $R^2$ zusammen mit den diese Reste tragenden Kohlenstoffatomen einen an den Isoxazolring ankondensierten 6-gliedrigen Kohlenwasserstoffring bilden, welcher unsubstituiert ist oder zumindest ein Halogen als Substituenten aufweist, und

$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen oder

$R^3$, $R^4$ und das diese Reste tragende Stickstoffatom eine alicyclische Aminogruppe mit insgesamt 5 oder 6 Ringkohlenstoffatomen bilden, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

27. Verfahren nach Anspruch 25, worin die cerebro-aktive Substanz die Formel (Ib)

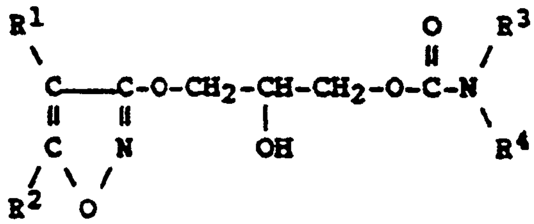

(Ib)

besitzt, in welcher
$R^1$ ein Wasserstoffatom, ein Chloratom oder eine $C_1$- oder $C_2$-Alkylgruppe bedeutet,
$R^2$ eine Phenylgruppe oder eine Phenylgruppe mit zumindest einem der unten definierten Substituenten (c′) darstellt oder
$R^1$ und $R^2$ zusammen mit den diese Reste tragenden Kohlenstoffatomen einen an den Isoxazolring ankondensierten Benzolring bilden, welcher unsubstituiert ist oder ein Halogenatom als Substituenten aufweist, wobei die

Substituenten (c′)
Methoxygruppen und Halogenatome sind,

$R^3$ und $R^4$ untereinander gleich oder voneinander verschieden sind und je für sich ein Wasserstoffatom, eine Methylgruppe oder eine Äthylgruppe darstellen oder
$R^3$, $R^4$ und das diese Reste tragende Stickstoffatom gemeinsam eine Morpholinogruppe, eine 1-Piperazinylgruppe, eine 4-Methyl-1-piperazinylgruppe, eine 1-Pyrrolidinylgruppe oder eine Piperidinogruppe bilden.

28. Verfahren nach Anspruch 25, worin die cerebro-aktive Substanz zumindest eine der Verbindungen
2-(2-Hydroxy-3-morpholinopropyl)-4-methyl-5-phenyl-3-isoxazolon,
3-(3-Carbamoyloxy-2-hydroxypropoxy)-5-(m-chlorphenyl)-isoxazol,
3-(2-Hydroxy-3-morpholinopropoxy)-5-phenylisoxazol,
3-(2-Hydroxy-3-morpholinopropoxy)-4-methyl-5-phenylisoxazol,
3-(3-Carbamoyloxy-2-hydroxypropoxy)-5-chlor-1,2-benzisoxazol
oder ein pharmazeutisch annehmbares Salz hievon ist.

29. Verwendung zumindest einer cerebro-aktiven Substanz zum Herstellen eines Heilmittels für die Behandlung einer cerebrovasculären Störung, worin die cerebro-aktive Substanz eine Verbindung der Formel (I)

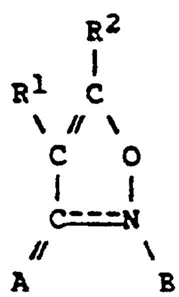

(I)

in welcher entweder

(a) die strichlierte Linie (---) eine Doppelbindung bedeutet,

A = für ein Sauerstoffatom steht und
B für eine Gruppe der Formel (II)

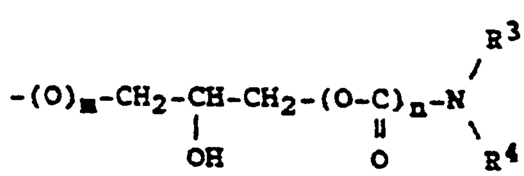

(II)

mit der Bedeutung von 0 für m und der Bedeutung von 0 oder 1 für n steht oder

(b) die strichlierte Linie (- - -) eine Doppelbindung bedeutet,

A = die erwähnte Gruppe der Formel (II) mit der Bedeutung von 1 für m und der Bedeutung von 0 oder 1 für n darstellt und

B nicht vorhanden ist und

$R^1$ ein Wasserstoffatom, ein Halogenatom, eine $C_1$-$C_4$-Alkylgruppe, eine $C_2$-$C_4$-Alkenylgruppe, eine $C_2$-$C_4$-Alkinylgruppe, eine Benzylgruppe, eine substituierte Benzylgruppe mit zumindest einem der unten definierten Substituenten (a), eine Phenylgruppe oder eine substituierte Phenylgrupne mit zumindest einem der unten definierten Substituenten (a) bedeutet,
$R^2$ ein Wasserstoffatom, ein Halogenatom, eine $C_1$-$C_4$-Alkylgruppe, eine Phenylgruppe, eine substituierte Phenylgruppe mit zumindest einem der unten definierten Substituenten (a) oder eine heterocyclische Gruppe mit 5 oder 6 Ringatomen, von welchen 1 bis 3 von Stickstoff- und/oder Sauerstoff- und/oder Schwefelatomen gebildeten Heteroatomen sind, darstellt, wobei die heterocyclische Gruppe unsubstituiert ist oder zumindest einen der unten definierten Substituenten (a) aufweist, oder
$R^1$ und $R^2$ zusammen mit den diese Reste tragenden Kohlenstoffatomen einen an den Isoxazolring ankondensierten und insesamt 5 bis 7 Ringkohlenstoffatome besitzenden Kohlenwasserstoffring bilden, welcher unsubstituiert ist oder zumindest einen der unten definierten Substituenten (b) aufweist,
$R^3$ und $R^4$ untereinander gleich oder voneinander verschieden sind und je für sich ein Wasserstoffatom, eine $C_1$-$C_4$-Alkylgruppe, eine Benzylgruppe, eine substituierte Benzylgruppe mit zumindest einem der unten definierten Substituenten (a), eine Phenylgruppe oder eine substituierte Phenylgruppe mit zumindest einem der unten definierten Substituenten (a) darstellen oder
$R^3$ und $R^4$ zusammen mit dem diese Reste tragenden Stickstoffatom eine alicyclische Aminogruppe mit insgesamt 5 oder 6 Ringatomen bilden, von welchen eines das erwähnte Stickstoffatom ist und 0 oder 1 ein weiteres von Stickstoff und/oder Sauerstoff und/oder Schwefel gebildetes Heteroatom ist, wobei diese alicyclische Aminogruppe unsubstituiert ist oder, falls ein weiteres Stickstoffatom als Heteroatom vorliegt, an diesem von Stickstoff gebildeten zusätzlichen Heteroatom zumindest einen der unten definierten Substituenten (b) aufweist, wobei

die Substituenten (a)
$C_1$-$C_3$-Alkylgruppen, $C_1$-$C_3$-Alkoxygruppen, Hydroxygruppen, Halogenatome, Nitrogruppen, Aminogruppen und von aliphatischen Carbonsäuren abgeleitete $C_2$-$C_4$-Acylgruppen sind und

die Substituenten (b)
$C_1$-$C_3$-Alkylgruppen, $C_1$-$C_3$-Alkoxygrupnen, Hydroxygruppen, Halogenatome, Nitrogruppen, Aminogruppen, von aliphatischen Carbonsäuren abgeleitete $C_2$-$C_4$-Acylaminogruppen, Benzylgrupnen, substituierte Benzylgruppen mit zumindest einem der oben definierten Substituenten (a), Phenylgruppen und substituierte Phenylgrunnen mit zumindest einem der oben definierten Substituenten (a) sind,

mit der Maßgabe, daß
(i) falls B für die erwähnte Gruppe der Formel (II) steht und n gleich ist O, $R^1$ nicht für ein Wasserstoffatom oder ein Halogenatom steht, und
(ii) falls (- - -) für eine Einfachbindung steht und $R^1$ und $R^2$ zusammen mit den diese Reste tragenden Kohlenstoffatomen einen $C_6$-$C_7$ -Kohlenwasserstoffring bilden, n gleich ist 1, oder ein pharmazeutisch annehmbares Salz hievon ist.

30. Verwendung nach Anspruch 29, worin die cerebro-aktive Substanz die Formel (Ib)

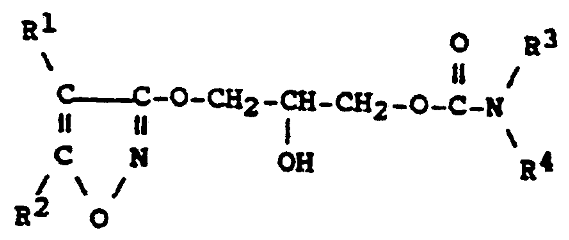

(Ib)

besitzt, in welcher
$R^1$ ein Wasserstoffatom, ein Halogenatom oder eine $C_1$-$C_4$-Alkylgruppe bedeutet,
$R^2$ eine Phenylgruppe oder eine Phenylgruppe mit zumindest einem der unten definierten Substituenten (c) darstellt und hiebei die

Substituenten (c)

$C_1$-$C_3$-Alkoxygruppen, Hydroxygruppen und Halogenatome sind oder

$R^1$ und $R^2$ zusammen mit den diese Reste tragenden Kohlenstoffatomen einen an den Isoxazolring ankondensierten 6-gliedrigen Kohlenwasserstoffring bilden, welcher unsubstituiert ist oder zumindest ein Halogen als Substituenten aufweist, und

$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen oder

$R^3$, $R^4$ und das diese Reste tragende Stickstoffatom zusammen eine alicyclische Aminogruppe mit insgesamt 5 oder 6 Ringatomen darstellen, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

31. Verwendung nach Anspruch 29, worin die cerebro-aktive Substanz die allgemeine Formel (Ib)

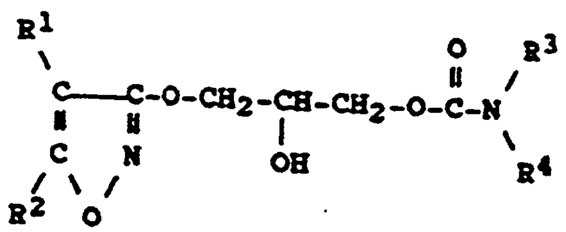

(Ib)

besitzt, in welcher

$R^1$ ein Wasserstoffatom, ein Chloratom der eine $C_1$- oder $C_2$-Alkylgruppe bedeutet,

$R^2$ eine Phenylgruppe oder eine Phenylgruppe mit zumindest einem der unten definierten Substituenten (c') darstellt oder

$R^1$ und $R^2$ zusammen mit den diese Reste tragenden Kohlenstoffatomen einen an den Isoxazolring ankondensierten Benzolring darstellen, welcher unsubstituiert ist oder ein Halogen als Substituenten aufweist und hiebei die

Substituenten (c')

Methoxygruppen und Halogenatome sind,

$R^3$ und $R^4$ untereinander gleich oder voneinander verschieden sind und je für sich ein Wasserstoffatom, eine Methylgruppe oder eine Äthylgruppe darstellen oder

$R^3$, $R^4$ und das diese Reste tragende Stickstoffatom gemeinsam eine Morpholinogruppe ; eine 1-Piperazinylgruppe, eine 4-Methyl-1-piperazinylgruppe, eine 1-Pyrrolidinylgruppe oder eine Piperidinogruppe bilden.

32. Verwendung nach Anspruch 29, worin die cerebro-aktive Substanz zumindest eine der Verbindungen

2-(2-Hydroxy-3-morpholinopropyl)-4-methyl-5-phenyl-3-isoxazolon,

3-(3-Carbamoyloxy-2-hydroxypropoxy)-5-(m-chlorphenyl)-isoxazol,

3-(2-Hydroxy-3-morpholinopropoxy)-5-phenylisoxazol,

3-(2-Hydroxy-3-morpholinopropoxy)-4-methyl-5-phenyl-isoxazol,

3-(3-Carbamoyloxy-2-hydroxypropoxy)-5-chlor-1,2-benzisoxazol

oder ein pharmazeutisch annehmbares Salz hievon ist.

33. Verwendung eines Wirkstoffes zum Herstellen eines Heilmittels zum Erzeugen einer zentral wirkenden Muskelrelaxationswirkung, worin der Wirkstoff eine in Anspruch 29 definierte Verbindung der Formel (I) oder ein pharmazeutisch annehmbares Salz hievon ist.

34. Verwendung nach Anspruch 33, worin der Wirkstoff die Formel (Ib)

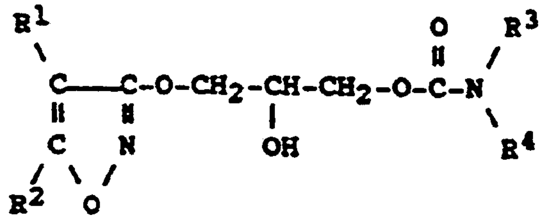

(Ib)

besitzt, in welcher
$R^1$ ein Wasserstoffatom, ein Halogenatom oder eine $C_1$-$C_4$-Alkylgruppe bedeutet,
$R^2$ eine Phenylgruppe oder eine substituierte Phenylgruppe mit zumindest einem der unten definierten Substituenten (c) darstellt und hiebei die

Substituenten (c)

$C_1$-$C_3$-Alkoxygruppen, Hydroxygruppen und Halogenatome sind oder
$R^1$ und $R^2$ zusammen mit den diese Reste tragenden Kohlenstoffatomen einen an den Isoxazolring ankondensierten 6-gliedrigen Kohlenwasserstoffring bilden, welcher unsubstituiert ist oder zumindest ein Halogen als Substituenten aufweist, und
$R^3$ und $R^4$ untereinander gleich oder voneinander verschieden sind und je für sich ein Wasserstoffatom, eine $C_1$-$C_4$-Alkylgruppe, eine Benzylgruppe oder eine Phenylgruppe darstellen oder
$R^3$, $R^4$ und das diese Reste tragende Stickstoffatom zusammen eine alicyclische Aminogruppe mit insgesamt 5 oder 6 Ringatomen darstellen, von welchen eines das erwähnte Stickstoffatom und 0 oder 1 ein von einem weiteren Stickstoffatom gebildetes Heteroatom ist, wobei die alicyclische Aminogruppe unsubstituiert ist oder, falls ein von einem weiteren Stickstoffatom gebildetes Heteroatom vorliegt, an diesem vom zusätzlichen Stickstoffatom gebildeten Heteroatom zumindest ein aus den unten definierten Substituenten (d) ausgewählter Substituent vorliegt und die

Substituenten (d)

$C_1$-$C_3$-Alkylgruppen, Benzylgruppen und Phenylgruppen sind.

35. Verwendung nach Anspruch 33, worin der Wirkstoff die Formel (Ib)

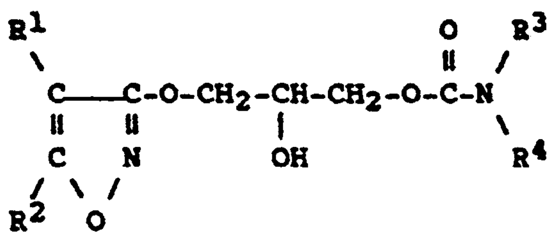

(Ib)

besitzt, in welcher
$R^1$ ein Wasserstoffatom, ein Chloratom oder eine $C_1$- oder $C_2$-Alkylgruppe bedeutet,
$R^2$ eine Phenylgruppe oder eine Phenylgruppe mit zumindest einem der unten definierten Substituenten (c′) darstellt oder
$R^1$ und $R^2$ zusammen mit den diese Reste tragenden Kohlenstoffatomen einen an den Isoxazolring ankondensierten Benzolring bilden, welcher unsubstituiert ist oder ein Halogen als Substituenten aufweist, und hiebei die

Substituenten (c′)

Methoxygruppen und Halogenatome sind,
$R^3$ und $R^4$ untereinander gleich oder voneinander verschieden sind und je für sich ein Wasserstoffatom, eine Methylgruppe oder eine Äthylgruppe darstellen oder
$R^3$, $R^4$ und das diese Reste tragende Stickstoffatom gemeinsam eine Morpholinogruppe, eine 1-Piperazinylgruppe, eine 4-Methyl-1-piperazinylgruppe, eine 1-Pyrrolidinylgruppe oder eine Piperidinogruppe bilden.

36. Verwendung nach Anspruch 33, worin der Wirkstoff zumindest eine der Verbindungen
2-(2-Hydroxy-3-morpholinopropyl)-4-methyl-5-phenyl-3-isoxazolon,
3-(3-Carbamoyloxy-2-hydroxypropoxy)-5-(m-chlorphenyl)-isoxazol,
3-(2-Hydroxy-3-morpholinopropoxy)-5-phenylisoxazol,
3-(2-Hydroxy-3-morpholinopropoxy)-4-methyl-5-phenyl-isoxazol,
3-(3-Carbamoyloxy-2-hydroxypropoxy)-5-chlor-1,2-benzisoxazol

oder ein pharmazeutisch annehmbares Salz hievon ist.

## Revendications

### Revendications pour les Etats contractants suivants : AT, BE, CH, DE, FR, GB, IT, LI, LU, NL, SE

1. Composés de formule (I) :

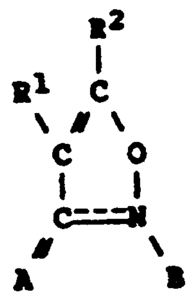

(I)

dans laquelle : soit

(a) le pointillé (- - -) représente une simple liaison ;

A = représente un atome d'oxygène ; et

B représente un groupe de formule (II) :

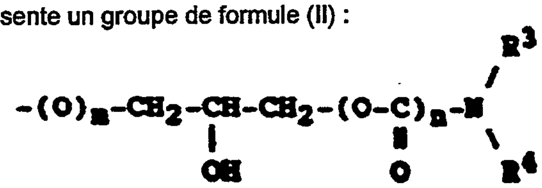

(II)

dans laquelle m est 0 et n est 0 ou 1 ;

(b) le pointillé (- - -) représente une double liaison ;

A = représente ledit groupe de formule (II) dans laquelle m est 1 et n est 0 ou 1 ; et

B est absent ; et

$R^1$ représente un atome d'hydrogène, un atome d'halogène, un groupe alkyle en $C_1$-$C_4$, un groupe alcynyle en $C_2$-$C_4$, un groupe alcynyle en $C_2$-$C_4$, un groupe benzyle, un groupe benzyle substitué ayant au moins un des substituants (a) définis ci-après, un groupe phényle ou un groupe phényle substitué ayant au moins un des substituants (a) définis ci-après ;

$R^2$ représente un atome d'hydrogène, un atome d'halogène, un groupe alkyle en $C_1$-$C_4$, un groupe phényle, un groupe phényle substitué ayant au moins un des substituants (a) définis ci-après ou un groupe hétérocyclique ayant 5 ou 6 atomes formant le cycle, dont 1 à 3 sont des hétéroatomes d'azote et/ou d'oxygène et/ou de soufre, ledit groupe hétérocyclique étant non substitué ou ayant au moins un des substituants (a) définis ci-après ; ou

$R^1$ et $R^2$, avec les atomes de carbone auxquels ils sont fixés, représentent un cycle hydrocarboné condensé au cycle isoxazole et ayant au total de 5 à 7 atomes de carbone formant le cycle, ledit cycle hydrocarboné étant non substitué ou ayant au moins un des substituants (b) définis ci-après;

$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis ci-après, un groupe phényle ou un groupe phényle substitué ayant au moins un des substituants (a) définis ci-après ; ou

$R^3$ et $R^4$ et l'atome d'azote auquel ils sont fixés représentent un groupe amino alicyclique ayant au total 5 ou 6 atomes formant le cycle, dont un est ledit atome d'azote et 0 ou 1 est un hétéroatome additionnel d'azote et/ou d'oxygène et/ou de soufre, ledit groupe amino alicyclique étant non substitué ou, lorsqu'il y a un hétéroatome d'azote additionnel, ayant au moins un des substituants (b) définis ci-après sur cet hétéroatome d'azote additionnel ;

substituants (a) :
groupes alkyle en $C_1$-$C_3$, groupes alcoxy en $C_1$-$C_3$, groupes hydroxy, atomes d'halogène, groupes nitro, groupes amino et groupes acyle carboxyliques aliphatiques en $C_2$-$C_4$ ;
substituants (b) :
groupes alkyle en $C_1$-$C_3$, groupes alcoxy en $C_1$-$C_3$, groupes hydroxy, atomes d'halogène, groupe nitro, groupes amino, groupes acylamino carboxyliques aliphatiques en $C_2$-$C_4$, groupes benzyle, groupes benzyle substitués ayant au moins un des substituants (a) définis ci-dessus, groupes phényles et groupes phényle substitués ayant au moins un des substituants (a) définis ci-dessus ;
sous réserve que :
(i) lorsque B représente ledit groupe de formule (II) et n est 0, alors $R^1$ ne représente pas un atome d'hydrogène ou d'halogène ; et
(ii) lorsque A représente ledit groupe de formule (II) et n est 0, $R^1$ et $R^2$ ensemble représentent un cycle benzène condensé au cycle isoxazole et $R^4$ représente un groupe alkyle, alors $R^3$ ne représente pas un atome d'hydrogène ;
(iii) lorsque (- - -) représente une simple liaison et $R^1$ et $R^2$, avec les atomes de carbone auxquels ils sont fixés, forment un cycle hydrocarboné en $C_6$-$C_7$, alors n est 1 ;
et leurs sels pharmaceutiquement acceptables, mais
à l'exclusion
du composé 5-chloro-2-(3-pipéridino-2-hydroxy)propylbenzisoxazoline-3-one.

2. Composés selon la revendication 1 représentés par la formule (Ia) :

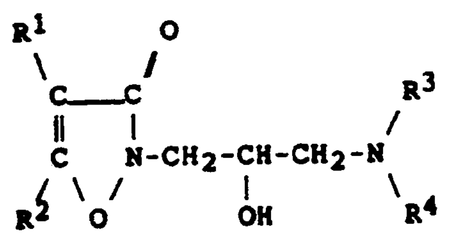

(Ia)

dans laquelle :
$R^1$ représente un groupe alkyle en $C_1$-$C_4$, un groupe alcényle en $C_2$-$C_4$, un groupe alcynyle en $C_2$-$C_4$, un groupe benzyle, un groupe benzyle substitué, un groupe phényle ou un groupe phényle substitué, les substituants étant choisis parmi les substituants (a) définis dans la revendication 1 ;
$R^2$ représente un atome d'hydrogène, un groupe alkyle en $C_1$-$C_4$, un groupe phényle, un groupe phényle substitué ou un groupe hétérocyclique comme défini dans la revendication 1 ;
$R^3$ et $R^4$ sont semblables ou différents et représentent chacun un atome d'hydrogène, un groupe alkyle en $C_1$-$C_4$, un groupe benzyle, un groupe benzyle substitué, un groupe phényle ou un groupe phényle substitué, ou $R^3$ et $R^4$ peuvent ensemble, avec l'atome d'azote auquel ils sont fixés, former un groupe amino alicyclique comme défini dans la revendication 1 ;
et leurs sels pharmaceutiquement acceptables.

3. Composés selon la revendication 1 représentés par la formule (Ib) :

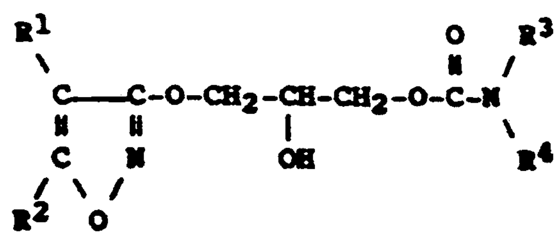

(Ib)

dans laquelle :
$R^1$ représente un atome d'hydrogène, un atome d'halogène, un groupe alkyle en $C_1$-$C_4$, un groupe alcényle en $C_2$-$C_4$, un groupe alcynyle en $C_2$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ;
$R^2$ représente un atome d'hydrogène, un groupe alkyle en $C_1$-$C_4$, un groupe phényle, un groupe phényle substitué ayant au moins un des substituants (a) définis dans la revendication 1 ou un groupe hétérocyclique comme défini dans la revendication 1 ; ou

$R^1$ et $R^2$, avec les atomes de carbone auxquels ils sont fixés, représentent un cycle hydrocarboné condensé au cycle isoxazole et comme défini dans la revendication 1 ;
$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ; ou
$R^3$ et $R^4$ et l'atome d'azote auquel ils sont fixés représentent ensemble un groupe amino alicyclique comme défini dans la revendication 1 ;
et leurs sels pharmaceutiquement acceptables.

4. Composés selon la revendication 1 représentés par la formule (Ic) :

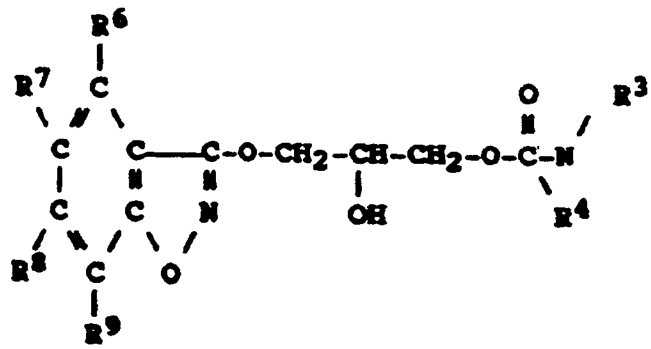

(Ic)

dans laquelle :
$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis dans la revendication 1, un groupe phényle ou un groupe phényle substitués ayant au moins un des substituants (a) définis dans la revendication 1 ; ou
$R^3$, $R^4$ et l'atome d'azote auquel ils sont fixés représentent ensemble un groupe amino alicyclique comme défini dans la revendication 1 ; et un de $R^6$, $R^7$, $R^8$ et $R^9$ représente un atome d'hydrogène, un atome d'halogène, un groupe alkyle en $C_1$-$C_4$ ou un groupe alkoxy en $C_1$-$C_4$ et tous les autres représentent des atomes d'hydrogène;
et leurs sels pharmaceutiquement acceptables.

5. Composés selon la revendication 1 représentés par la formule (Id) :

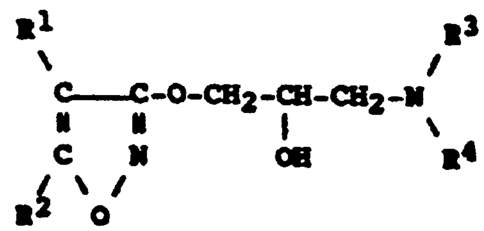

(Id)

dans laquelle :
$R^1$ représente un atome d'hydrogène, un atome d'halogène, un groupe alkyle en $C_1$-$C_4$, un groupe alcényle en $C_2$-$C_4$, un groupe alcynyle en $C_2$-$C_4$, un groupe benzyle, un groupe benzyle substitué ayant au moins un des substituants (a) définis dans la revendication 1, un groupe phényle substitué ayant au moins un des substituants (a) définis dans la revendication 1 ;
$R^2$ représente un atome d'hydrogène, un groupe alkyle en $C_1$-$C_4$, un groupe phényle, un groupe phényle substitué ayant au moins un des substituants (a) définis dans la revendication 1 ou un groupe hétérocyclique comme défini dans la revendication 1 ; ou
$R^1$ et $R^2$, ensemble avec les atomes de carbone auxquels ils sont fixés, représentent un cycle hydrocarbonés condensé au cycle isoxazole et comme défini dans la revendication 1 ;
$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ; ou
$R^3$, $R^4$ et l'atome d'azote auquel ils sont fixés représentent ensemble un groupe amino alicyclique comme défini dans la revendication 1 ; sous réserve que, lorsque $R^1$ et $R^2$ ensemble représentent un cycle benzène condensé

au cycle izoxazole et $R^4$ représente un groupe alkyle, alors $R^3$ ne représente pas un atome d'hydrogène ; et leurs sels pharmaceutiquement acceptables.

6. Composés selon la revendication 1 représentés par la formule (Ie) :

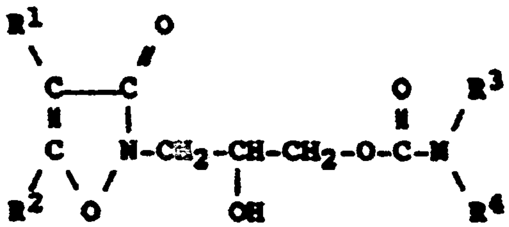 (Ie)

dans laquelle :

$R^1$ représente un atome d'hydrogène, un atome d'halogène, un groupe alkyle en $C_1$-$C_4$, un groupe alcényle en $C_2$-$C_4$, un groupe alcynyle en $C_2$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ;

$R^2$ représente un atome d'hydrogène, un groupe alkyle en $C_1$-$C_4$, un groupe phényle, un groupe phényle substitué ayant au moins un des substituants (a) définis dans la revendication 1 ou un groupe hétérocyclique comme défini dans la revendication 1 ; ou

$R^1$ et $R^2$, ensemble avec les atomes de carbone auxquels ils sont fixés, représentent un cycle hydrocarboné condenssé au cycle isoxazole et comme défini dans la revendication 1 ;

$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ; ou

$R^3$, $R^4$ et l'atome d'azote auquel ils sont fixés représentent ensemble un groupe amino alicyclique comme défini dans la revendication 1 ;

et leurs sels pharmaceutiquement acceptables.

7. Composés selon la revendication 1 représentés par la formule (If) :

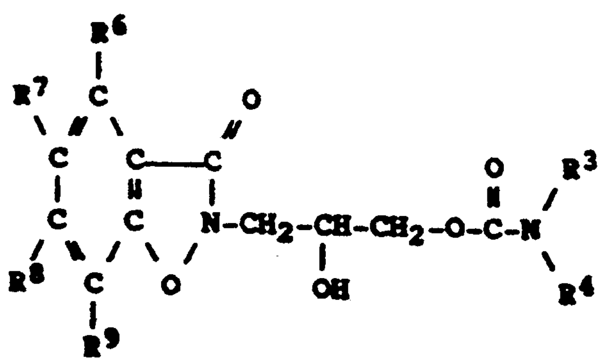 (If)

dans laquelle :

$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ; ou

$R^3$, $R^4$ et l'atome d'azote auquel ils sont fixés représentent ensemble un groupe amino alicyclique comme défini dans la revendication 1 ; et un de $R^6$, $R^7$, $R^8$ et $R^9$ représente un atome d'hydrogène, un atome d'halogène, un groupe alkyle en $C_1$-$C_4$ ou un groupe alcoxy en $C_1$-$C_4$, et tous les autres représentent des atomes d'hydrogène; et leurs sels pharmaceutiquement acceptables.

8. Composés selon la revendication 1, dans lesquels $R^1$ représente un atome d'hydrogène, un atome d'halogène ou un groupe alkyle en $C_1$-$C_4$.

9. Composés selon la revendication 1, dans lesquels $R^2$ représente un groupe phényle ou un groupe phényle substitué ayant au moins un des substituants (c) définis ci-après :

substituants (c) :

groupes alcoxy en $C_1$-$C_3$, groupes hydroxy et atomes d'halogène.

10. Composés selon la revendication 1, dans lesquels $R^1$ et $R^2$, avec les atomes de carbone auxquels ils sont fixés, représentent un cycle hydrocarboné à 6 chaînons condensé au cycle izoxazole et étant non substitué ou ayant au moins un atome d'halogène.

11. Composés selon la revendication 1, dans lesquels $R^3$ et $R^4$ sont semblables ou différents et représentent chacun un atome d'hydrogène, un groupe alkyle en $C_1$-$C_4$, un groupe benzyle ou un groupe phényle.

12. Composés selon la revendication 1, dans lesquels $R^3$, $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome d'azote additionnel, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :

substituants (d)

groupes alkyle en $C_1$-$C_3$, groupes benzyle et groupes phényle.

13. Composés selon la revendication 1, dans lesquels $R^1$ représente un atome d'hydrogène, un atome de chlore ou un groupe alkyle en $C_1$ ou $C_2$.

14. Composés selon la revendication 1, dans lesquels $R^2$ représente un groupe phényle ou un groupe phényle substitué ayant au moins un des substituants (c') définis ci-après :

substituants (c')

groupes méthoxy et atomes d'halogène.

15. Composés selon la revendication 1, dans lesquels $R^1$ et $R^2$, ensemble avec les atomes de carbone auxquels ils sont fixés, représentent un cycle benzène condensé au cycle isoxazole et étant non substitué ou ayant un substituant choisi parmi les atomes d'halogène.

16. Composés selon la revendication 1, dans lesquels $R^3$ et $R^4$ sont semblables ou différents et représentent chacun un atome d'hydrogène, un groupe méthyle ou un groupe éthyle.

17. Composés selon la revendication 1, dans lesquels $R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

18. Composés selon la revendication 1, dans lesquels :

$R^1$ représente un atome d'hydrogène, un atome d'halogène ou un groupe alkyle en $C_1$-$C_4$ ;

$R^2$ représente un groupe phényle ou un groupe phényle substitué ayant au moins un des substituants (c) définis ci-après :

substituants (c) :

groupes alcoxy en $C_1$-$C_3$, groupes hydroxy et atomes d'halogène ;

$R^3$ et $R^4$ sont semblables ou différents et représentent chacun un atome d'hydrogène, un groupe alkyle en $C_1$-$C_4$, un groupe benzyle ou un groupe phényle ; ou

$R^3$, $R^4$ et l'atome d'azote auquel ils sont fixés représentent ensemble un groupe amino alicyclique ayant au total 5 ou 6 atomes formant le cycle dont un est ledit atome d'azote et 0 ou 1 est un hétéroatome additionnel d'azote, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :

substituants (d) :

groupes alkyle en $C_1$-$C_3$, groupes benzyle et groupes phényle.

19. Composés selon la revendication 1, dans lesquels :

$R^1$ et $R^2$, ensemble avec les atomes de carbone auxquels ils sont fixés, représentent un cycle hydrocarboné à 6 chaînons condensé au cycle isoxazole et étant non substitué ou ayant au moins un atome d'halogène ;

$R^3$ et $R^4$ sont semblables ou différents et représentent chacun un atome d'hydrogène, un groupe alkyle en $C_1$-$C_4$, un groupe benzyle ou un groupe phényle ; ou

$R^3$, $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome d'azote additionnel, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :

substituants (d) :

groupes alkyle en $C_1$-$C_3$, groupes benzyle et groupes phényle.

20. Composés selon la revendication 1, dans lesquels :

$R^1$ représente un atome d'hydrogène, un atome de chlore ou un groupe alkyle en $C_1$ ou $C_2$ ;

$R^2$ représente un groupe phényle ou un groupe phényle substitué ayant au moins un des substituants (c') définis ci-après :

substituants (c')
groupes méthoxy et atomes d'halogène ;
$R^3$ et $R^4$ sont semblables ou différents et représentent chacun un atome d'hydrogène, un groupe méthyle ou un groupe éthyle ;
$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

21. Composés selon la revendication 1, dans lesquels :
$R^1$ et $R^2$, ensemble avec les atomes de carbone auxquels ils sont fixés, représentent un cycle benzène condensé au cycle izoxazole et étant non substitué ou ayant un atome d'halogène ;
$R^3$ et $R^4$ sont semblables ou différents et représentent chacun un atome d'hydrogène, un groupe méthyle ou un groupe éthyle ; ou
$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

22. Composés selon la revendication 3 représentés par la formule (Ib) :

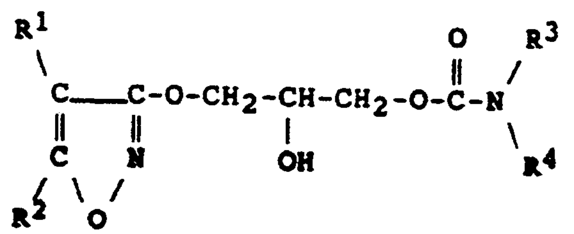

(Ib)

dans laquelle :
$R^1$ représente un atome d'hydrogène, un atome d'halogène ou un groupe alkyle en $C_1$-$C_4$ ;
$R^2$ représente un groupe phényle ou un groupe phényle substitué ayant au moins un des substituants (c) définis ci-après :
substituants (c) :
groupes alcoxy en $C_1$-$C_3$, groupes hydroxy et atomes d'halogène ; ou
$R^1$ et $R^2$, avec les atomes de carbone auxquels ils sont fixés, représentent un cycle hydrocarboné à 6 chaînons condensé au cycle isoxazole et étant non substitué ou ayant au moins un atome d'halogène ; et
$R^3$ et $R^4$ sont semblables ou différents et représentent chacun un atome d'hydrogène, un groupe alkyle en $C_1$-$C_4$, un groupe benzyle ou un groupe phényle ; ou
$R^3$, $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome d'azote additionnel, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :
substituants (d) :
groupes alkyle en $C_1$-$C_3$, groupes benzyle et groupes phényle.

23. Composés selon la revendication 22 représentés par la formule (Ib) :

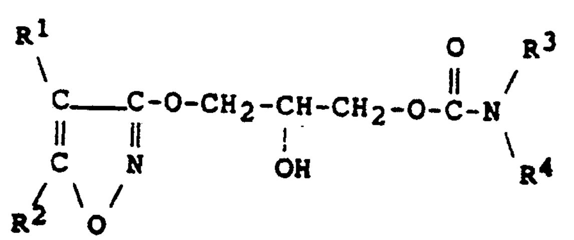

(Ib)

dans laquelle :
$R^1$ représente un atome d'hydrogène, un atome de chlore ou un groupe alkyle en $C_1$ ou $C_2$ ;
$R^2$ représente un groupe phényle ou un groupe phényle substitué ayant au moins un des substituants (c') définis ci-après ; ou
$R^1$ et $R^2$, ensemble avec les atomes de carbone auxquels ils sont fixés, représentent un cycle benzène condensé au cycle isoxazole et étant non substitué ou ayant un atome d'halogène ;

substituants (c')
groupes méthoxy et atomes d'halogène ; et
$R^3$ et $R^4$ sont semblables ou différents et représentent chacun un atome d'hydrogène, des groupes méthyle et des groupes éthyle ; ou
$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pypéridino.

24. 2-(2-hydroxy-3-morpholinopropyl)-4-méthyl-5-phényl-3-isoxazolone et ses sels pharmaceutiquement acceptables.

25. 3-(3- carbamoyloxy-2-hydroxypropoxy)-5-(m-chlorophényl) isoxazole et ses sels pharmaceutiquement acceptables.

26. 3-(2-hydroxy-3-morpholinopropoxy)-5-phénylisoxazole et ses sels pharmaceutiquement acceptables.

27. 3-(2-hydroxy-3-morpholinopropoxy)-4-méthyl-5-phényl-isoxazole et ses sels pharmaceutiquement acceptables.

28. 3-(3-carbamoyloxy-2-hydroxypropoxy)-5-chloro-1,2-benzisoxazole et ses sels pharmaceutiquement acceptables.

29. Utilisation, pour la préparation d'un médicament pour le traitement d'un trouble cérébro-vasculaire, d'au moins un médicament cérébro-actif, le médicament cérébro-actif étant un composé de formule (I) :

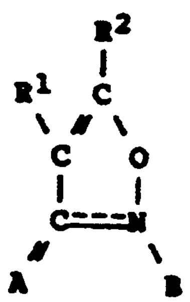

(I)

dans laquelle : soit
(a) le pointillé (---) représente une simple liaison ;

A = représente un atome d'oxygène ; et
B représente un groupe de formule (II) :

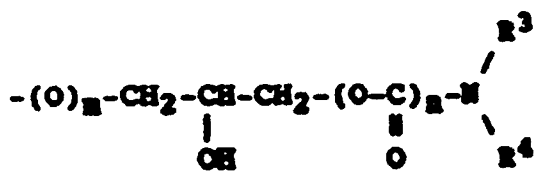

(II)

dans laquelle m est 0 et n est 0 ou 1 ; soit
(b) le pointillé (---) représente une double liaison ;

A = représente ledit groupe de formule (II) dans laquelle m est 1 et n est 0 ou 1 ; et
(B) est absent ; et

$R^1$ représente un atome d'hydrogène, un atome d'halogène, un groupe alkyle en $C_1$-$C_4$, un groupe alcényle en $C_2$-$C_4$, un groupe alcynyle en $C_2$-$C_4$, un groupe benzyle, un groupe benzyle substitué ayant au moins un des substituants (a) définis ci-après, un groupe phényle ou un groupe phényle substitué ayant au moins un des substituants (a) définis ci-après ;
$R^2$ représente un atome d'hydrogène, un atome d'halogène, un groupe alkyle en $C_1$-$C_4$, un groupe phényle, un groupe phényle substitué ayant au moins un des substituants (a) définis ci-après ou un groupe hétérocyclique ayant 5 ou 6 atomes formant le cycle, dont 1 à 3 sont des hétéroatomes d'azote et/ou d'oxygène et/ou de soufre, ledit groupe hétérocyclique étant non substitué ou ayant au moins un des substituants (a) définis ci-après ; ou
$R^1$ et $R^2$, avec les atomes de carbone auxquels ils sont fixés, représentent un cycle hydrocarboné condensé au cycle isoxazole et ayant au total de 5 à 7 atomes de carbone formant le cycle, ledit

cycle hydrocarboné étant non substitué ou ayant au moins un des substituants (b) définis ci-après; $R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis ci-après, un groupe phényle ou un groupe phényle substitué ayant au moins un des substituants (a) définis ci-après ; ou

$R^3$ et $R^4$ et l'atome d'azote auquel ils sont fixés représentent un groupe amino alicyclique ayant au total 5 ou 6 atomes formant le cycle, dont un est ledit atome d'azote et 0 ou 1 est un hétéroatome additionnel d'azote et/ou d'oxygène et/ou de soufre, ledit groupe amino alicyclique étant non substitué, ou, lorsqu'il y a un hétéroatome d'azote additionnel, ayant au moins un des substituants (b) définis ci-après sur cet hétéroatome d'azote additionnel ;

substituants (a) :

groupes alkyle en $C_1$-$C_3$, groupes alcoxy en $C_1$-$C_3$, groupes hydroxy, atomes d'halogène, groupes nitro, groupes amino et groupes acyle carboxyliques aliphatiques en $C_2$-$C_4$ ;

substituants (b) :

groupes alkyle en $C_1$-$C_3$, groupes alcoxy en $C_1$-$C_3$, groupes hydroxy, atomes d'halogène, groupes nitro, groupes amino, groupes acylamino carboxyliques aliphatiques en $C_2$-$C_4$, groupes benzyle, groupes benzyle substitués ayant au moins un des substituants (a) définis ci-dessus, groupes phényle et groupes phényle substitués ayant au moins un des substituants (a) définis ci-dessus ;

sous réserve que :

lorsque B représente ledit groupe de formule (II) et n est 0, alors $R^1$ ne représente pas un atome d'hydrogène ou d'halogène ;

et leurs sels pharmaceutiquement acceptables.

30. Utilisation selon la revendication 29, ledit médicament cérébro-actif étant représenté par la formule (Ib):

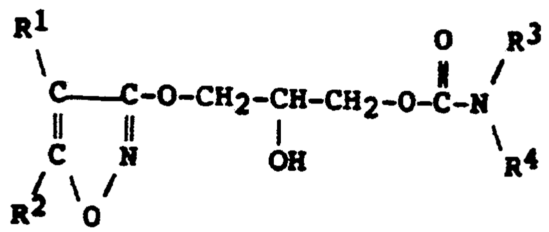

(Ib)

dans laquelle :

$R^1$ représente un atome d'hydrogène, un atome d'halogène ou un groupe alkyle en $C_1$-$C_4$ ;

$R^2$ représente un groupe phényle ou un groupe phényle ayant au moins un des substituants (c) définis ci-après:

substituants (c) :

groupes alcoxy en $C_1$-$C_3$, groupes hydroxy et atomes d'halogène ; ou

$R^1$ et $R^2$, ensemble avec les atomes de carbone auxquels ils sont fixés, représentent un cycle hydrocarboné à 6 chaînons condensé au cycle isoxazole et étant non substitué ou ayant au moins un atome d'halogène ; et $R^3$ et $R^4$ sont semblables ou différents et représentent chacun un atome d'hydrogène, un groupe alkyle en $C_1$-$C_4$, un groupe benzyle ou un groupe phényle ; ou

$R^3$, $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome d'azote additionnel, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :

substituants (d) :

groupes alkyle en $C_1$-$C_3$, groupes benzyle et groupes phényle.

31. Utilisation selon la revendication 29, dans laquelle le médicament cérébro-actif est représenté par la formule (Ib) :

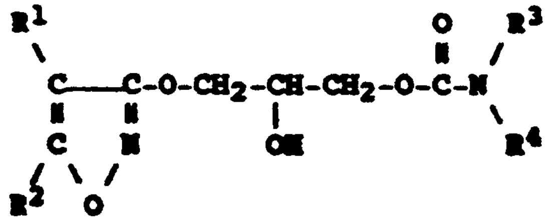

(Ib)

dans laquelle :

$R^1$ représente un atome d'hydrogène, un atome de chlore ou un groupe alkyle en $C_1$ ou $C_2$ ;

$R^2$ représente un groupe phényle ou un groupe phényle ayant au moins un des substituants (c') définis ci-après; ou

$R^1$ et $R^2$, ensemble avec les atomes de carbone auxquels ils sont fixés, représentent un cycle benzène condensés au cycle izoxazole et étant non substitué ou ayant un atome d'halogène ; et

substituants (c')

groupes méthoxy et atomes d'halogène ;

$R^3$ et $R^4$ sont semblables ou différents et représentent chacun un atome d'hydrogène, un groupe méthyle ou un groupe éthyle ; ou

$R^3$, $R^4$ et l'atome d'azote auquel ils sont fixés représentent ensemble un groupe morpholino, un groupe 1-pipérazilnyle, un groupe 4-méthyl-1-pipérazinyle, un groupe 1-pyrrolidinyle ou un groupe pipéridino.

32. Utilisation selon la revendication 29, ledit médicament cérébro-actif étant au moins un des composés suivants :

2-(2-hydroxy-3-morpholinopropyl)-4-méthyl-5-phényl-3-isoxazolone ;

3-(3-carbamoyloxy-2-hydroxypropoxy)-5-(m-chlorophényl)isoxazole ;

3-(2-hydroxy-3-morpholinopropoxy)-5-phénylisoxasole ;

3-(2-hydroxy-3-morpholinopropoxy)-4-méthyl-5-phénylisoxazole ;

3-(3-carbamoyloxy-2-hydroxypropoxy)-5-chloro-1,2-benzisoxazole ;

et leurs sels pharmaceutiquement acceptables.

33. Utilisation pour la préparation d'un médicament pour exercer une activité myorelaxante à effet central d'un composé actif, le composé actif étant un composé de formule (I) ou un de ses sels pharmaceutiquement acceptables, selon la revendication 29.

34. Utilisation selon la revendication 33, ledit composé actif étant représenté par la formule (Ib) :

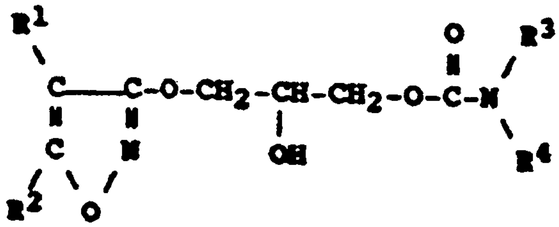

(Ib)

dans laquelle :

$R^1$ représente un atome d'hydrogène, un atome d'halogène ou un groupe alkyle en $C_1$-$C_4$ ;

$R^2$ représente un groupe phényle ou un groupe phényle ayant au moins un des substituants (c) définis ci-après:

substituants (c) :

groupes alcoxy en $C_1$-$C_3$, groupes hydroxy et atomes d'halogène ; ou

$R^1$ et $R^2$, ensemble avec les atomes de carbone auxquels ils sont fixés, représentent un cycle hydrocarboné à 6 chaînons condensé au cycle isoxazole et étant non substitué ou ayant au moins un atome d'halogène ; et

$R^3$ et $R^4$ sont semblables ou différents et représentent chacun un atome d'hydrogène, un groupe alkyle en $C_1$-$C_4$, un groupe benzyle ou un groupe phényle ; ou

$R^3$, $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome d'azote additionnel, ledit groupe amino alicyclique étant non substitié ou, lorsqu'il y a un hétéroatome d'azote additionnel, ayant sur cet hétéroatome d'azote additionnel au moins un substituant choisi parmi les substituants (d) définis ci-après :

substituants (d) :

groupes alkyle en $C_1$-$C_3$, groupes benzyle et groupes phényle.

35. Utilisation selon la revendication 33, ledit composé actif étant représentés par la formule (Ib) :

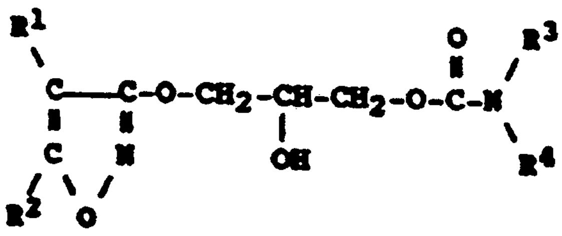

(Ib)

dans laquelle :

$R^1$ représente un atome d'hydrogène, un atome de chlore ou un groupe alkyle en $C_1$ ou $C_2$ ;

$R^2$ représente un groupe phényle ou un groupe phényle ayant au moins un des substituants (c') définis ci-après; ou

$R^1$ et $R^2$, ensemble avec les atomes de carbone auxquels ils sont fixés, représentent un cycle benzène condensé au cycle isoxazole et étant non substitié ou ayant un atome d'halogène ; et

<u>substituants (c')</u>

groupes méthoxy et atomes d'halogène ;

$R^3$ et $R^4$ sont semblables ou différents et représentent chacun un atome d'hydrogène, un groupe méthyle ou un groupe éthyle ; ou

$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

36. Utilisation selon la revendication 33, ledit composé actif étant au moins un des composés suivants :

2-(2-hydroxy-3-morpholinopropyl)-4-méthyl-5-phényl-3-isoxazolone ;
3-(3-carbamoyloxy-2-hydroxypropoxy)-5-(m-chlorophényl) isoxazole ;
3-(2-hydroxy-3-morpholinopropoxy)-5-phénylisoxazole ;
3-(2-hydroxy-3-morpholinopropoxy)-4-méthyl-5-phénylisoxazole ;
3-(3-carbamoyloxy-2-hydroxypropoxy)-5-chloro-1,2-benzisoxazole ;
ou un de leurs sels pharmaceutiquement acceptables.

37. Composition pharmaceutique pour le traitement des troubles cérébro-vasculaires ou pour l'utilisation comme myorelaxant à action centrale, qui comprend un composé actif en mélange avec un véhicule ou diluant pharmaceutiquement acceptables, le composé actif étant un composé de formule (I) ou un de ses sels pharmaceutiquement acceptables, comme défini dans la revendication 29.

38. Composition selon la revendication 37, dans laquelle ledit composé actif est représenté par la formule (Ib) :

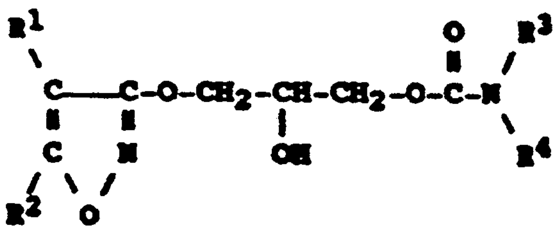

(Ib)

dans laquelle :

$R^1$ représente un atome d'hydrogène, un atome d'halogène ou un groupe alkyle en $C_1$-$C_4$ ;

$R^2$ représente un groupe phényle ou un groupe phényle substitué ayant au moins un des substituants (c) définis ci-après :

<u>substituants (c)</u> :

groupes alcoxy en $C_1$-$C_3$, groupes hydroxy et atomes d'halogène ; ou

$R^1$ et $R^2$, ensemble avec les atomes de carbone auxquels ils sont fixés, représentent un cycle hydrocarboné à 6 chaînons condensé au cycle isoxazole et étant non substitué ou ayant au moins un atome d'halogène ; et

$R^3$ et $R^4$ sont semblables ou différents et représentent chacun un atome d'hydrogène, un groupe alkyle en $C_1$-$C_4$, un groupe benzyle ou un groupe phényle ; ou

$R^3$, $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome d'azote additionnel, ledit groupe amino alicyclique étant non substitué ou, lorsqu'il y a un hétéroatome d'azote additionnel,

ayant sur cet hétéroatome d'azote additionnel au moins un substituant choisi parmi les substituants (d) définis ci-après :

substituants (d) :

groupes alkyle en $C_1$-$C_3$, groupes benzyle et groupes phényle.

39. Composition selon la revendication 37, dans laquelle ledit composé actif est représenté par la formule (Ib) :

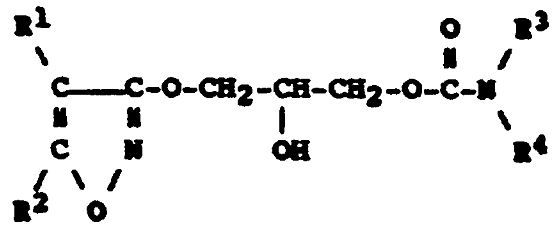

(Ib)

dans laquelle :

$R^1$ représente un atome d'hydrogène, un atome de chlore ou un groupe alkyle en $C_1$ ou $C_2$ ;

$R^2$ représente un groupe phényle ou un groupe phényle ayant au moins un des substituants (c') définis ci-après; ou

$R^1$ et $R^2$, ensemble avec les atomes de carbone auxquels ils sont fixés, représentent un cycle benzène condensé au cycle izoxazole et étant non substitué ou ayant un atome d'halogène ; et

substituants (c')

groupes méthoxy et atomes d'halogène ;

$R^3$ et $R^4$ sont semblables ou différents et représentent chacun un atome d'hydrogène, un groupe méthyle ou un groupe éthyle ; ou

$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

40. Composition selon la revendication 37, dans laquelle ledit composé actif est au moins un des composés :

2-(2-hydroxy-3-morpholinopropyl)-4-méthyl-5-phényl-3-isoxazolone ;

3-(3-carbamoyloxy-2-hydropropoxy)-5-(m-chlorophényl)isoxazole ;

3-(2-hydroxy-3-morpholinopropoxy)-5-phénylisoxazole ;

3-(2-hydroxy-3-morpholinopropoxy)-4-méthyl-5-phénylisoxazole ;

3-(3-carbamoyloxy-2-hydroxypropoxy)-5-chloro-1,2-benzisoxazole ;

et leurs sels pharmaceutiquement acceptables.

41. Procédé pour préparer un composé selon l'une quelconque des revendications 1 à 28, qui comprend les étapes de :

(i) réaction d'un composé de formule (III) :

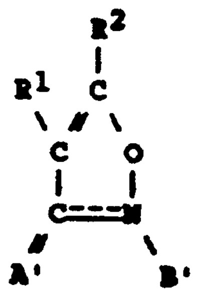

(III)

[dans laquelle : soit

(a) le pointillé (- - -) représente une simple liaison ;

A' = représente un atome d'oxygène ; et

B' représente un groupe de formule (IV) :

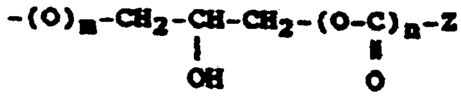 (IV)

dans laquelle m est 0 et n est 0 ou 1 ; soit
(b) le pointillé (- - -) représente une double liaison ;

A' = représente ledit groupe de formule (IV) dans laquelle m est 1 et n est 0 ou 1 ; et
B' est absent ;

Z représente un groupe labile ;
et $R^1$ et $R^2$ sont comme définis dans la revendication 1] avec une amine de formule (V) :

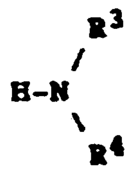 (V)

(dans laquelle $R^3$ et $R^4$ sont comme définis dans la revendication 1) ; et, le cas échéant, la salification du produit obtenu.

**Revendications pour les Etats contractants suivants : ES, GR**

1. Procédé pour préparer un composé de formule (I) :

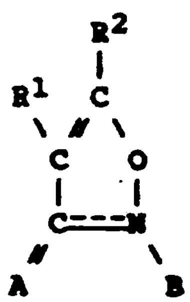 (I)

dans laquelle : soit
(a) le pointillé (- - -) représente une simple liaison ;

A = représente un atome d'oxygène ; et
B représente un groupe de formule (II) :

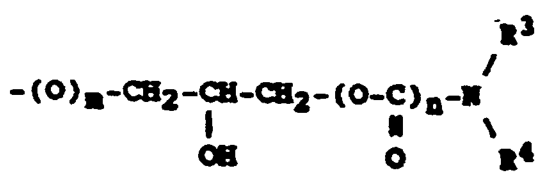 (II)

dans laquelle m est 0 et n est 0 ou 1, soit
(b) le pointillé (- - -) représente une double liaison ;

A = représente ledit groupe de formule (II) dans laquelle m est 1 et n est 0 ou 1 ; et
B est absent ; et

$R^1$ représente un atome d'hydrogène, un atome d'halogène, un groupe alkyle en $C_1$-$C_4$, un groupe alcényle en $C_2$-$C_4$, un groupe alcynyle en $C_2$-$C_4$, un groupe benzyle, un groupe benzyle substitué ayant au moins un des substituants (a) définis ci-après, un groupe phényle ou un groupe phényle

substitué ayant au moins un des substituants (a) définis ci-après ;

$R^2$ représente un atome d'hydrogène, un atome d'halogène, un groupe alkyle en $C_1$-$C_4$, un groupe phényle, un groupe phényle substitués ayant au moins un des substituants (a) définis ci-après ou un groupe hétérocyclique ayant 5 ou 6 atomes formant le cycle, dont 1 à 3 sont des hétéroatomes d'azote et/ou d'oxygène et/ou de soufre, ledit groupe hétérocyclique étant non substitué ou ayant au moins un des substituants (a) définis ci-après ; ou

$R^1$ et $R^2$, avec les atomes de carbone auxquels ils sont fixés, représentent un cycle hydrocarboné condensé au cycle isoxazole et ayant au total de 5 à 7 atomes de carbone formant le cycle, ledit cycle hydrocarboné étant non substitué ou ayant au moins un des substituants (b) définis ci-après;

$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis ci-après, un groupe phényle ou un groupe phényle substitué ayant au moins un des substituants (a) définis ci-après ; ou

$R^3$ et $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome additionnel d'azote et/ou d'oxygène et/ou de soufre, ledit groupe amino alicyclique étant non substitué ou, lorsqu'il y a un hétéroatome d'azote additionnel, ayant au moins un des substituants (b) définis ci-après sur cet hétéroatome d'azote additionnel ;

substituants (a) :

groupes alkyle en $C_1$-$C_3$, groupes alcoxy en $C_1$-$C_3$, groupes hydroxy, atomes d'halogène, groupes nitro, groupes amino et groupes acyle carboxyliques aliphatiques en $C_2$-$C_4$ ;

substituants (b) :

groupes alkyle en $C_1$-$C_3$, groupes alcoxy en $C_1$-$C_3$, groupes hydroxy, atomes d'halogène, groupes nitro, groupes amino, groupes acylamino carboxyliques aliphatiques en $C_2$-$C_4$, groupes benzyle, groupes benzyle substitués ayant au moins un des substituants (a) définis ci-dessus, groupes phényle et groupes phényle substitués ayant au moins un des substituants (a) définis ci-dessus ;

sous réserve que :

(i) lorsque B représente ledit groupe de formule (II) et n est 0, alors $R^1$ ne représente pas un atome d'hydrogène ou d'halogène ; et

(ii) lorsque A représente ledit groupe de formule (II) et n est 0, $R^1$ et $R^2$ ensemble représentent un cycle benzène condensé au cycle isoxazole et $R^4$ représente un groupe alkyle, alors $R^3$ ne représente pas un atome d'hydrogène ;

(iii) lorsque (- - -) représente une simple liaison et $R^1$ et $R^2$, avec les atomes de carbone auxquels ils sont fixés, forment un cycle hydrocarboné en $C_6$-$C_7$, alors n est 1 ;

ou un de ses sels pharmaceutiquement acceptables ; mais

à l'exclusion

du composé 5-chloro-2-(3-pipéridino-2-hydroxy)propylbenzisoxazoline-3-one, lequel procédé comprend les étapes de :

(i) réaction d'un composé de formule (III) :

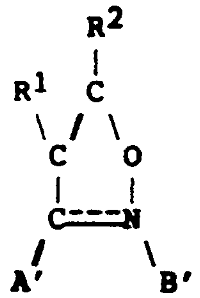

(III)

[dans laquelle : soit

(a) le pointillé (- - -) représente une simple liaison ;

A' = représente un atome d'oxygène ; et

B' représente un groupe de formule (IV) :

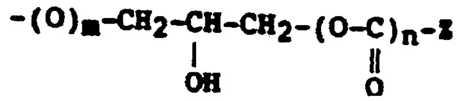

(IV)

dans laquelle m est 0 et n est 0 ou 1 ; soit
(b) le pointillé (- - -) représente une double liaison ;

A' = représente ledit groupe de formule (IV) dans laquelle m est 1 et n est 0 ou 1 ; et
B' est absent ;

Z représente un groupe labile ;
et $R^1$ et $R^2$ sont comme définis ci-dessus] avec une amine de formule (V) :

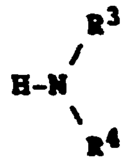

(V)

(dans laquelle $R^3$ et $R^4$ sont comme définis ci-dessus) ;
et, le cas échéant, la salification du produit obtenu.

2. Procédé selon la revendication 1, dans lequel les composés réagissants et les conditions de réaction sont choisis de façon à préparer un composé de formule (Ia) :

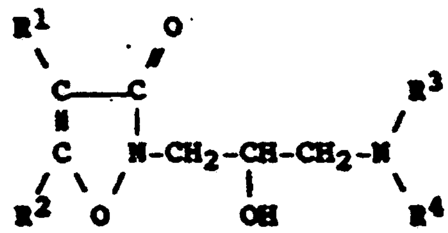

(Ia)

dans laquelle :
$R^1$ représente un groupe alkyle en $C_1$-$C_4$, un groupe alcényle en $C_2$-$C_4$, un groupe alcynyle en $C_2$-$C_4$, un groupe benzyle, un groupe benzyle substitué, un groupe phényle ou un groupe phényle substitué, les substituants étant choisis parmi les substituants (a) définis dans la revendication 1 ;
$R^2$ représente un atome d'hydrogène, un groupe alkyle en $C_1$-$C_4$, un groupe phényle, un groupe phényle susbstitué ou un groupe hétérocyclique comme défini dans la revendication 1 ;
$R^3$ et $R^4$ sont semblables ou différents et représentent chacun un atome d'hydrogène, un groupe alkyle en $C_1$-$C_4$, un groupe benzyle, un groupe benzyle substitué, un groupe phényle ou un groupe phényle substitué, ou $R^3$ et $R^4$ peuvent ensemble, avec l'atome d'azote auquel ils sont fixés, former un groupe amino alicyclique comme défini dans la revendication 1 ;
ou un de ses sels pharmaceutiquement acceptables.

3. Procédé selon la revendication 1, dans lequel les composés réagissants et les conditions de réaction sont choisis de façon à préparer un composé de formule (Ib) :

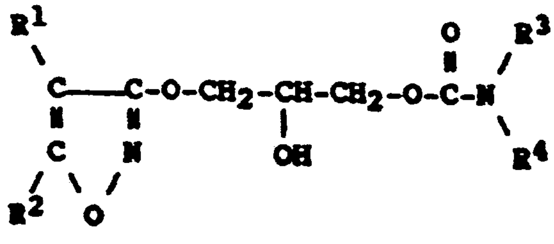

(Ib)

dans laquelle :
$R^1$ représente un atome d'hydrogène, un atome d'halogène, un groupe alkyle en $C_1$-$C_4$, un groupe alcényle en

$C_2$-$C_4$, un groupe alcynyle en $C_2$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ;

$R^2$ représente un atome d'hydrogène, un groupe alkyle en $C_1$-$C_4$, un groupe phényle, un groupe phényle substitué ayant au moins un des substituants (a) définis dans la revendication 1 ou un groupe hétérocyclique comme défini dans la revendication 1 ; ou

$R^1$ et $R^2$, avec les atomes de carbone auxquels ils sont fixés, représentent un cycle hydrocarboné condensé au cycle isoxazole et comme défini dans la revendication 1 ;

$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ; ou

$R^3$ et $R^4$ et l'atome d'azote auquel ils sont fixés représentent ensemble un groupe amino alicyclique comme défini dans la revendication 1 ;

ou un de ses sels pharmaceutiquement acceptables.

4. Procédé selon la revendication 1, dans lequel les composés réagissants et les conditions de réaction sont choisis de façon à préparer un composé de formule (Ic) :

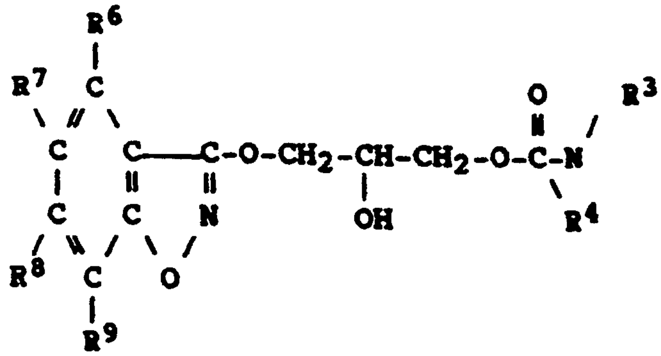

(Ic)

dans laquelle :

$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ; ou

$R^3$, $R^4$ et l'atome d'azote auquel ils sont fixés représentent ensemble un groupe amino alicyclique comme défini dans la revendication 1 ; et un de $R^6$, $R^7$, $R^8$ et $R^9$ représente un atome d'hydrogène, un atome d'halogène, un groupe alkyle en $C_1$-$C_4$ ou un groupe alcoxy en $C_1$-$C_4$ et tous les autres représentent des atomes d'hydrogène; ou un de ses sels pharmaceutiquement acceptables.

5. Procédé selon la revendication 1, dans lequel les composé réagissants et les conditions de réaction sont choisis de façon à préparer un composés de formule (Id) :

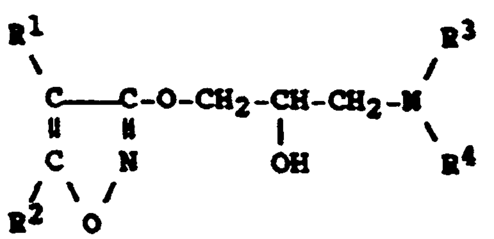

(Id)

dans laquelle :

$R^1$ représente un atome d'hydrogène, un atome d'halogène, un groupe alkyle en $C_1$-$C_4$, un groupe alcényle en $C_2$-$C_4$, un groupe alcynyle en $C_2$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ;

$R^2$ représente un atome d'hydrogène, un groupe alkyle en $C_1$-$C_4$, un groupe phényle, un groupe phényle substitué ayant au moins un des substituants (a) définis dans la revendication 1 ou un groupe hétérocyclique comme défini dans la revendication 1 ; ou

$R^1$ et $R^2$, ensemble avec les atomes de carbone auxquels ils sont fixés, représentent un cycle hydrocarboné condensé au cycle isoxazole et comme défini dans la revendication 1 ;

$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ; ou

$R^3$, $R^4$ et l'atome d'azote auquel ils sont fixés représentent ensemble un groupe amino alicyclique comme défini dans la revendication 1 ; sous réserve que ; lorsque $R^1$ et $R^2$ ensemble représentent un cycle benzène condensé au cycle isoxazole et $R^4$ représente un groupe alkyle, alors $R^3$ ne représente pas un atome d'hydrogène ;

ou un de ses sels pharmaceutiquement acceptables.

6. Procédé selon la revendication 1, dans lequel les composés réagissants et les conditions de réaction sont choisis de façon à préparer un composé de formule (Ie) :

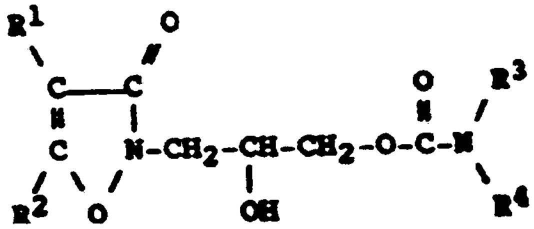

(Ie)

dans laquelle :

$R^1$ représente un atome d'hydrogène, un atome d'halogène, un groupe alkyle en $C_1$-$C_4$, un groupe alcényle en $C_2$-$C_4$, un groupe alcynyle en $C_2$-$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ;

$R^2$ représente un atome d'hydrogène, un groupe alkyle en $C_1$-$C_4$, un groupe phényle, un groupe phényle substitué ayant au moins un des substituants (a) définis dans la revendication 1 ou un groupe hétérocyclique comme défini dans la revendication 1 ; ou

$R^1$ et $R^2$, ensemble avec les atomes de carbone auxquels ils sont fixés, représentent un cycle hydrocarboné condensé au cycle isoxazole et comme défini dans la revendication 1 ;

$R^3$ et $R^4$ sont semblables ou différents et représentent chacun un atome d'hydrogène, un groupe alkyle en $C_1$-$C_4$, un groupe benzyle, un groupe benzyle substitués ayant au moins un des substituants (a) définis dans la revendication 1, un groupe phényle ou un groupe phényle substitué ayant au moins un des substituants (a) définis dans la revendication 1 ; ou

$R^3$, $R^4$ et l'atome d'azote auquel ils sont fixés représentent ensemble un groupe amino alicyclique comme défini dans la revendication 1 ;

ou un de ses sels pharmaceutiquement acceptables.

7. Procédé selon la revendication 1, dans lequel les composés réagissants et les conditions de réaction sont choisis de façon à préparer un composé de formule (If) :

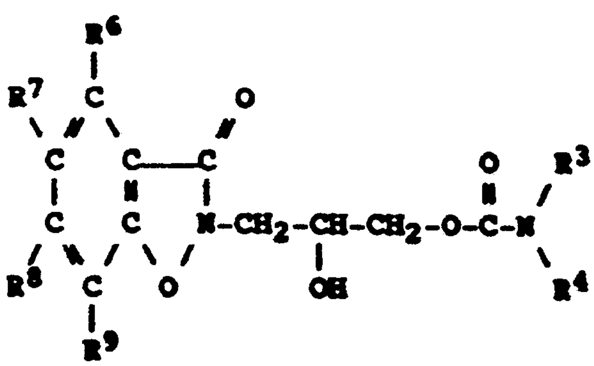

(If)

dans laquelle :

$R^3$ et $R^4$ sont semblables ou différents et représentent chacun un atome d'hydrogène, un groupe alkyle en $C_1$-

$C_4$, un groupe benzyle, un groupe benzyle substitué ayant au moins un des substituants (a) définis dans la revendication 1, un groupe phényle ou un groupe phényle substitué ayant au moins un des substituants (a) définis dans la revendication 1 ; ou

$R^3$, $R^4$ et l'atome d'azote auquel ils sont fixés représentent ensemble un groupe amino alicyclique comme défini dans la revendication 1 ; et un de $R^6$, $R^7$, $R^8$ et $R^9$ représente un atome d'hydrogène, un atome d'halogène, un groupe alkyle en $C_1$-$C_4$ ou un groupe alcoxy en $C_1$-$C_4$, et tous les autres représentent des atomes d'hydrogène; ou un de ses sels pharmaceutiquement acceptables.

8. Procédé selon la revendication 1, dans lequel les composés réagissants et les conditions de réaction sont choisis de façon à préparer un composé de formule (I) ou un de ses sels, où $R^1$ représente un atome d'hydrogène, un atome d'halogène ou un groupe alkyle en $C_1$-$C_4$.

9. Procédé selon la revendication 1, dans lequel les composés réagissants et les conditions de réaction sont choisis de façon à préparer un composé de formule (I) ou un de ses sels, où $R^2$ représente un groupe phényle ou un groupe phényle substitués ayant au moins un des substituants (c) définis ci-après :

substituants (c) :

groupes alcoxy en $C_1$-$C_3$, groupes hydroxy et atomes d'halogène.

10. Procédé selon la revendication 1, dans lequel les composés réagissants et les conditions de réaction sont choisis de façon à préparer un composé de formule (I) ou un de ses sels, où $R^1$ et $R^2$, avec les atomes de carbone auxquels ils sont fixés, représentent un cycle hydrocarboné à 6 chaînons condensé au cycle isoxazole et étant non substitués ou ayant au moins un atome d'halogène.

11. Procédé selon la revendication 1, dans lequel les composés réagissants et les conditions de réaction sont choisis de façon à préparer un composé de formule (I) ou un de ses sels, où $R^3$ et $R^4$ sont semblables ou différents et représentent chacun un atome d'hydrogène, un groupe alkyle en $C_1$-$C_4$, un groupe benzyle ou un groupe phényle.

12. Procédé selon la revendication 1, dans lequel les composés réagissants et les conditions de réaction sont choisis de façon à préparer un composé de formule (I) ou un de ses sels, où $R^3$, $R^4$ et l'atome d'azote auquel ils sont fixés représentent ensemble un groupe amino alicyclique ayant au total 5 ou 6 atomes formant le cycle, dont un est ledit atome d'azote et 0 et 1 est un hétéroatome d'azote additionnel, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :

substituants (d) :

groupes alkyle en $C_1$-$C_3$, groupes benzyle et groupes phényle.

13. Procédé selon la revendication 1, dans lequel les composés réagissants et les conditions de réaction sont choisis de façon à préparer un composé de formule (I) ou un de ses sels, où $R^1$ représente un atome d'hydrogène, un atome de chlore ou un groupe alkyle en $C_1$ ou $C_2$.

14. Procédé selon la revendication 1, dans lequel les composés réagissants et les conditions de réaction sont choisis de façon à préparer un composé de formule (I) ou un de ses sels, où $R^2$ représente un groupe phényle ou un groupe phényle substitué ayant au moins un des substituants (c') définis ci-après :

substituants (c')

groupes méthoxy et atomes d'halogène.

15. Procédé selon la revendication 1, dans lequel les composés réagissants et les conditions de réaction sont choisis de façon à préparer un composé de formule (I) ou un de ses sels, où $R^1$ et $R^2$, ensemble avec les atomes de carbone auxquels ils sont fixés, représentent un cycle benzène condensé au cycle isoxazole et étant non substitués ou avant un substituant choisi parmi les atomes d'halogène.

16. Procédé selon la revendication 1, dans lequel les composés réagissants et les conditions de réaction sont choisis de façon à préparer un composé de formule (I) ou un de ses sels, où $R^3$ et $R^4$ sont semblables ou différents et représentent chacun un atome d'hydrogène, un groupe méthyle ou un groupe éthyle.

17. Procédé selon la revendication 1, dans lequel les composés réagissants et les conditions de réaction sont choisis de façon à préparer un composé de formule (I) ou un de ses sels, où $R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

18. Procédé selon la revendication 1, dans lequel les composés réagissants et les conditions de réaction sont choisis de façon à préparer un composé de formule (I) ou un de ses sels, où :

$R^1$ représente un atome d'hydrogène, un atome d'halogène ou un groupe alkyle en $C_1$-$C_4$ ;

$R^2$ représente un groupe phényle ou un groupe phényle substitué ayant au moins un des substituants (c) définis ci-après :

substituants (c) :

groupes alcoxy en $C_1$-$C_3$, groupes hydroxy et atomes d'halogène ;

$R^3$ et $R^4$ sont semblables ou différents et représentent chacun un atome d'hydrogène, un groupe alkyle en $C_1$-

$C_4$, un groupe benzyle ou un groupe phényle ; ou

$R^3$, $R^4$ et l'atome d'azote auquel ils sont fixés représentent ensemble un groupe amino alicyclique ayant au total 5 ou 6 atomes formant le cycle dont un est ledit atome d'azote et 0 ou 1 est un hétéroatome additionnel d'azote, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :

substituants (d) :

groupes alkyle en $C_1$-$C_3$, groupes benzyle et groupes phényle.

19. Procédé selon la revendication 1, dans lequel les composés réagissants et les conditions de réaction sont choisis de façon à préparer un composé de formule (I) ou un de ses sels, où :

$R^1$ et $R^2$, ensemble avec les atomes de carbone auxquels ils sont fixés, représentent un cycle hydrocarboné à 6 chaînons condensé au cycle isoxazole et étant non substitué ou ayant au moins un atome d'halogène ;

$R^3$ et $R^4$ sont semblables ou différents et représentent chacun un atome d'hydrogène, un groupe alkyle en $C_1$-$C_4$, un groupe benzyle ou un groupe phényle ; ou

$R^3$, $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome d'azote additionnel, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :

substituants (d) :

groupes alkyle en $C_1$-$C_3$, groupes benzyle et groupes phényle.

20. Procédé selon la revendication 1, dans lequel les composés réagissants et les conditions de réaction sont choisis de façon à préparer un composé de formule (I) ou un de ses sels, où :

$R^1$ représente un atome d'hydrogène, un atome de chlore ou un groupe alkyle en $C_1$ ou $C_2$ ;

$R^2$ représente un groupe phényle ou un groupe phényle substitué ayant au moins un des substituants (c') définis ci-après :

substituants (c')

groupes méthoxy et atomes d'halogène ;

$R^3$ et $R^4$ sont semblables ou différents et représentent chacun un atome d'hydrogène, un groupe méthyle ou un groupe éthyle ; ou

$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

21. Procédé selon la revendication 1, dans lequel les composés réagissants et les conditions de réaction sont choisis de façon à préparer un composé de formule (I) ou un de ses sels, où :

$R^1$ et $R^2$, ensemble avec les atomes de carbone auxquels ils sont fixés, représentent un cycle benzène condensé au cycle isoxazole et étant non substitué ou ayant un atome d'halogène ;

$R^3$ et $R^4$ sont semblables ou différents et représentent chacun un atome d'hydrogène, un groupe méthyle ou un groupe éthyle ; ou

$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

22. Procédé selon la revendication 3, dans lequel les composés réagissants et les conditions de réaction sont choisis pour préparer un composé de formule (Ib) :

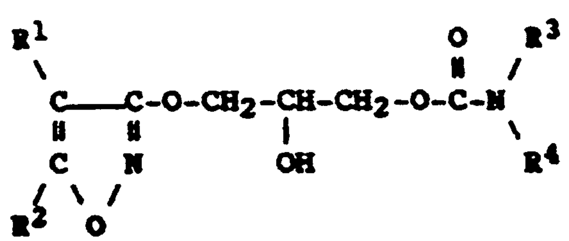

(Ib)

dans laquelle :

$R^1$ représente un atome d'hydrogène, un atome d'halogène ou un groupe alkyle en $C_1$-$C_4$ ;

$R^2$ représente un groupe phényle ou un groupe phényle substitué ayant au moins un des substituants (c) définis ci-après :

substituants (c) :

groupes alcoxy en $C_1$-$C_3$, groupes hydroxy et atomes d'halogène ; ou

$R^1$ et $R^2$, avec les atomes de carbone auxquels ils sont fixés, représentent un cycle hydrocarboné à 6 chaînons condensé au cycle isoxazole et étant non substitué ou ayant au moins un atome d'halogène ; et

$R^3$ et $R^4$ sont semblables ou différents et représentent chacun un atome d'hydrogène, un groupe alkyle en $C_1$-$C_4$, un groupe benzyle ou un groupe phényle ; ou

$R^3$, $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome d'azote additionnel, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :

substituants (d) :

groupes alkyle en $C_1$-$C_3$, groupes benzyle et groupes phényle, ou un sel pharmaceutiquement acceptable de celui-ci.

23. Procédé selon la revendication 22, dans lequel on choisit les composés réagissants et les conditions de réaction pour préparer un composé de formule (Ib) :

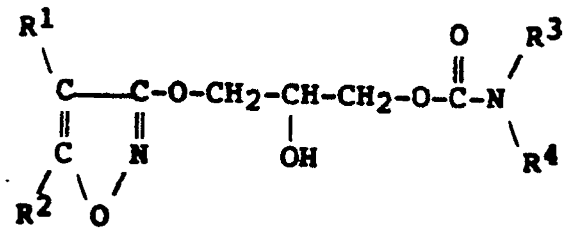

(Ib)

dans laquelle :

$R^1$ représente un atome d'hydrogène, un atome de chlore ou un groupe alkyle en $C_1$ ou $C_2$ ;

$R^2$ représente un groupe phényle ou un groupe phényle substitué ayant au moins un des substituants (c') définis ci-après ; ou

$R^1$ et $R^2$, ensemble avec les atomes de carbone auxquels ils sont fixés, représentent un cycle benzène condensés au cycle isoxazole et étant non substitué ou ayant un atome d'halogène ;

substituants (c')

groupes méthoxy et atomes d'halogène ; et

$R^3$ et $R^4$ sont semblables ou différents et représentent chacun un atome d'hydrogène, des groupes méthyle et des groupes éthyle ; ou

$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino,

ou un sel pharmaceutiquement acceptable de celui-ci.

24. Procédé selon la revendication 1 dans lequel on choisit les composés réagissants et les conditions de réaction pour préparer :

la 2-(2-hydroxy-3-morpholinopropyl)-4-méthyl-5-phényl-3-isoxazolone ;

le 3-(3-carbamoyloxy-2-hydroxypropoxy)-5-(m-chlorophényl)isoxazole ;

le 3-(2-hydroxy-3-morpholinopropoxy)-5-phénylisoxazole ;

le 3-(2-hydroxy-3-morpholinopropoxy)-4-méthyl-5-phénylisoxazole ;

le 3-(3-carbamoyloxy-2-hydropropoxy)-5-chloro-1,2-benzisoxazole ;

ou un de leurs sels pharmaceutiquement acceptables.

25. Procédé de préparation d'une composition pharmaceutique pour le traitement d'un trouble cérébro-vasculaire chez un mammifère atteint ou susceptible de troubles cérébro-vasculaires ou pour établir une activité myorelaxante à action centrale, qui comprend le mélange d'au moins un médicament cérébro-actif avec un véhicule ou diluant pharmaceutiquement acceptables, dans lequel le médicament cérébro-actif est un composé de formule (I) :

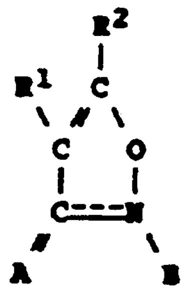

(I)

dans laquelle : soit

(a) le pointillé (- - -) représente une simple liaison ;

A = représente un atome d'oxygène ; et

B représente un groupe de formule (II) :

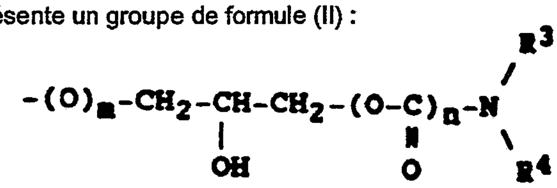

(II)

dans laquelle m est 0 et n est 0 ou 1 ; soit

(b) le pointillé (- - -) représente une double liaison ;

A = représente ledit groupe de formule (II) dans laquelle m est 1 et n est 0 ou 1 ; et

B est absent ; et

$R^1$ représente un atome d'hydrogène, un atome d'halogène, un groupe alkyle en $C_1$-$C_4$, un groupe alcényle en $C_2$-$C_4$, un groupe alcynyle en $C_2$-$C_4$, un groupe benzyle, un groupe benzyle substitué ayant au moins un des substituants (a) définis ci-après, un groupe phényle ou un groupe phényle substitués ayant au moins un des substituants (a) définis ci-après ;

$R^2$ représente un atome d'hydrogène, un atome d'halogène, un groupe alkyle en $C_1$-$C_4$, un groupe phényle, un groupe phényle substitué ayant au moins un des substituants (a) définis ci-après ou un groupe hétérocyclique ayant 5 ou 6 atomes formant le cycle, dont 1 à 3 sont des hétéroatomes d'azote et/ou d'oxygène et/ou de soufre, ledit groupe hétérocyclique étant non substitués ou ayant au moins un des substituants (a) définis ci-après ; ou

$R^1$ et $R^2$, avec les atomes de carbone auxquels ils sont fixés, représentent un cycle hydrocarboné condensé au cycle isoxazole et ayant au total de 5 à 7 atomes de carbone formant le cycle, ledit cycle hydrocarboné étant non substitusé ou ayant au moins un des substituants (b) définis ci-après;

$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis ci-après, un groupe phényle ou un groupe phényle substitué ayant au moins un des substituants (a) définis ci-après ; ou

$R^3$ et $R^4$ et l'atome d'azote auquel ils sont fixés représentent un groupe amino alicyclique ayant au total 5 ou 6 atomes formant le cycle, dont un est ledit atome d'azote et 0 ou 1 est un hétéroatome additionnel d'azote et/ou d'oxygène et/ou de soufre, ledit groupe amino alicyclique étant non substitués, ou, lorsqu'il y a un hétéroatome d'azote additionnel, ayant au moins un des substituants (b)définis ci-après sur cet hétéroatome d'azote additionnel ;

substituants (a) :

groupe alkyle en $C_1$-$C_3$, groupes alcoxy en $C_1$-$C_3$, groupes hydroxy, atomes d'halogène, groupes nitro, groupes amino et groupes acyle carboxyliques aliphatiques en $C_2$-$C_4$ ;

substituants (b) :

groupes alkyle en $C_1$-$C_3$, groupes alcoxy en $C_1$-$C_3$, groupes hydroxy, atomes d'halogène, groupes nitro, groupes amino, groupes acylamino carboxyliques aliphatiques en $C_2$-$C_4$, groupes benzyle, groupes benzyle substitués ayant au moins un des substituants (a) définis ci-dessus, groupes phényle et groupes phényle substitués ayant au moins un des substituants (a) définis ci-dessus ;

sous réserve que :

(i) lorsque B représente ledit groupe de formule (II) et n est 0, alors $R^1$ ne représente pas un atome d'hydrogène ou d'halogène ;
(ii) lorsque (- - -) représente une simple liaison, $R^1$ et $R^2$, avec les atomes de carbone auxquels ils sont fixés, forment un cycle hydrocarbonés en $C_6$-$C_7$, alors n = 1 ;
et leurs sels pharmaceutiquement acceptables.

26. Procédé selon la revendication 25 dans lequel ledit médicament cérébro-actif est représenté par la formule (Ib) :

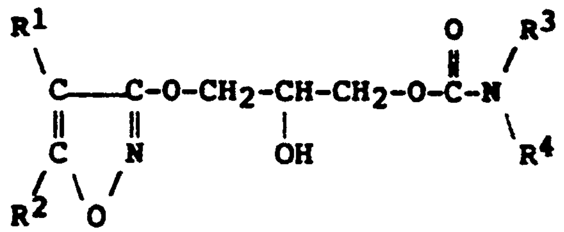

(Ib)

dans laquelle :
$R^1$ représente un atome d'hydrogène, un atome d'halogène ou un groupe alkyle en $C_1$-$C_4$ ;
$R^2$ représente un groupe phényle ou un groupe phényle ayant au moins un des substituants (c) définis ci-après:
substituants (c) :
groupes alcoxy en $C_1$-$C_3$, groupes hydroxy et atomes d'halogène ; ou
$R^1$ et $R^2$, ensemble avec les atomes de carbone auxquels ils sont fixés, représentent un cycle hydrocarboné à 6 chaînons condensé au cycle isoxazole et étant non substitué ou ayant au moins un atome d'halogène ; et
$R^3$ et $R^4$ sont semblables ou différents et représentent chacun un atome d'hydrogène, un groupe alkyle en $C_1$-$C_4$, un groupe benzyle ou un groupe phényle ; ou
$R^3$, $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome d'azote additionnel, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :
substituants (d) :
groupes alkyle en $C_1$-$C_3$, groupes benzyle et groupes phényle.

27. Procédé selon la revendication 25 dans lequel ledit médicament cérébro-actif est représenté par la formule (Ib) :

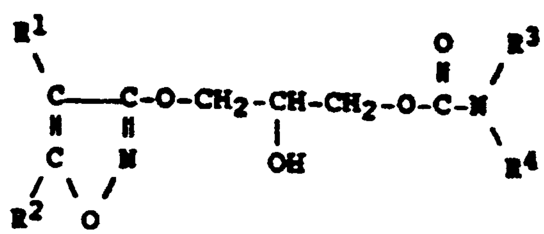

(Ib)

dans laquelle :
$R^1$ représente un atome d'hydrogène, un atome de chlore ou un groupe alkyle en $C_1$ ou $C_2$ ;
$R^2$ représente un groupe phényle ou un groupe phényle ayant au moins un des substituants (c') définis ci-après; ou
$R^1$ et $R^2$, ensemble avec les atomes de carbone auxquels ils sont fixés, représentent un cycle benzène condensés au cycle isoxazole et étant non substitué ou ayant un atome d'halogène ; et
substituant (c')
groupes méthoxy et atomes d'halogène ;
$R^3$ et $R^4$ sont semblables ou différents et représentent chacun un atome d'hydrogène, un groupe méthyle ou un groupe éthyle ; ou
$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

28. Procédé selon la revendication 25, dans lequel ledit médicament cérébro-actif est au moins un des

composés suivants :
2-(2-hydroxy-3-morpholinopropyl)-4-méthyl-5-phényl-3-isoxazolone ;
3-(3-carbamoyloxy-2-hydroxypropoxy)-5-(m-chlorophényl)isoxazole ;
3-(2-hydroxy-3-morpholinopropoxy)-5-phénylisoxazole ;
3-(2-hydroxy-3-morpholinopropoxy)-4-méthyl-5-phénylisoxazole ;
3-(3-carbamoyloxy-2-hydroxypropoxy)-5-chloro-1,2-benzisoxazole ;
et leurs sels pharmaceutiquement acceptables.

29. Utilisation pour la préparation d'un médicament pour le traitement d'un trouble cérébro-vasculaire d'au moins un médicament cérébro-actif, dans laquelle le médicament cérébro-actif est un composé de formule (I):

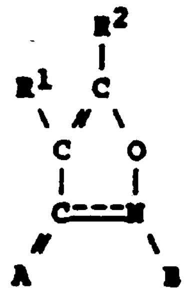

(I)

dans laquelle : soit

(a) le pointillé (- - -) représente une simple liaison ;

A = représente une simple liaison ;
B représente un groupe de formule (II) :

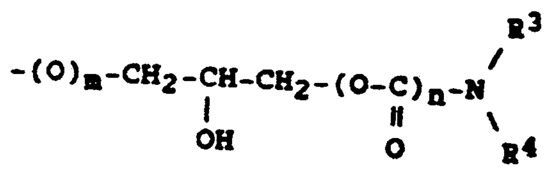

(II)

dans laquelle m est 0 et n est 0 ou 1 ; soit

(b) le pointillé (- - -) représente une double liaison ;

A = représente ledit groupe de formule (II) dans laquelle m est 1 et n est 0 ou 1 ; et
B est absent ; et

$R^1$ représente un atome d'hydrogène, un atome d'halogène, un groupe alkyle en $C_1$-$C_4$, un groupe alcényle en $C_2$-$C_4$, un groupe alcynyle en $C_2$-$C_4$, un groupe benzyle, un groupe benzyle substitué ayant au moins un des substituants (a) définis ci-après, un groupe phényle ou un groupe phényle substitué ayant au moins un des substituants (a) définis ci-après ;

$R^2$ représente un atome d'hydrogène, un atome d'halogène, un groupe alkyle en $C_1$-$C_4$, un groupe phényle, un groupe phényle substitué ayant au moins un des substituants (a) définis ci-après ou un groupe hétérocyclique ayant 5 ou 6 atomes formant le cycle, dont 1 à 3 sont des hétéroatomes d'azote et/ou d'oxygène et/ou de soufre, ledit groupe hétérocyclique étant non substitué ou ayant au moins un des substituants (a) définis ci-après ; ou

$R^1$ et $R^2$, avec les atomes de carbone auxquels ils sont fixés, représentent un cycle hydrocarboné condensé au cycle isoxazole et ayant au total de 5 à 7 atomes de carbone formant le cycle, ledit cycle hydrocarboné étant non substitué ou ayant au moins un des substituants (b) définis ci-après;

$R^3$ et $R^4$ sont semblables ou différents et représentent chacun un atome d'hydrogène, un groupe alkyle en $C_1$-$C_4$, un groupe benzyle, un groupe benzyle substitué ayant au moins un des substituants (a) définis ci-après, un groupe phényle ou un groupe phényle substitué ayant au moins un des substituants (a) définis ci-après ; ou

$R^3$ et $R^4$ et l'atome d'azote auquel ils sont fixés représentent un groupe amino alicyclique ayant au total 5 ou 6 atomes formant le cycle, dont un est ledit atome d'azote et 0 ou 1 est un hétéroatome additionnel d'azote et/ou d'oxygène et/ou de soufre, ledit groupe amino alicyclique étant substitué

ou, lorsqu'il y a un hétéroatome d'azote additionnel, ayant au moins un des substituants (b) définis ci-après sur cet hétéroatome d'azote additionnel ;

substituants (a) :

groupes alkyle en $C_1$-$C_3$, groupes alcoxy en $C_1$-$C_3$, groupes hydroxy, atomes d'halogène, groupes nitro, groupes amino et groupes acyle carboxyliques aliphatiques en $C_2$-$C_4$ ;

substituants (b) :

groupes alkyle en $C_1$-$C_3$, groupes alcoxy en $C_1$-$C_3$, groupes hydroxy, atomes d'halogène, groupes nitro, groupes amino, groupes acylamino carboxyliques aliphatiques en $C_2$-$C_4$, groupes benzyle, groupes benzyle substitués ayant au moins un des substituants (a) définis ci-dessus, groupes phényle et groupes phényles substitués ayant au moins un des substituants (a) définis ci-dessus ;

sous réserve que :

(i) lorque B représente ledit groupe de formule (II) et n est 0, alors $R^1$ ne représente pas un atome d'hydrogène ou d'halogène ;

(ii) lorsque (- - -) représente une simple liaison et $R^1$ et $R^2$, avec les atomes de carbone auxquels ils sont fixés, forment un cycle hydrocarboné en $C_6$-$C_7$, alors n est 1 ;

et leurs sels pharmaceutiquement acceptables.

30. Utilisation selon la revendication 31, dans laquelle ledit médicament cérébro-actif est représentés par la formule (Ib) :

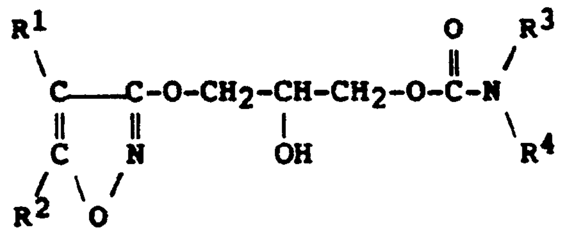

(Ib)

dans laquelle :

$R^1$ représente un atome d'hydrogène, un atome d'halogène ou un groupe alkyle en $C_1$-$C_4$ ;

$R^2$ représente un groupe phényle ou un groupe phényle ayant au moins un des substituants (c) définis ci-après:

substituants (c) :

groupes alcoxy $C_1$-$C_3$, groupes hydroxy et atomes d'halogène ; ou

$R^1$ et $R^2$, ensemble avec les atomes de carbone auxquels ils sont fixés, représentent un cycle hydrocarboné à 6 chaînons condensé au cycle izoxazole et étant non substitués ou ayant au moins un atome d'halogène ; et

$R^3$ et $R^4$ sont semblables ou différents et représentent chacun un atome d'hydrogène, un groupe alkyle en $C_1$-$C_4$, un groupe benzyle ou un groupe phényle ; ou

$R^3$, $R^4$ et l'atome d'azote auquel ils sont fixés représentent ensemble un groupe amino alicyclique ayant au tatal 5 ou 6 atomes formant le cycle, dont un est ledit atome d'azote et 0 ou 1 est un hétéroatome d'azote additionnel, ledit groupe amino alicyclique étant non substitué ou, lorqu'il y a un hétéroatome d'azote additionnel, ayant sur cet hétéroatome d'azote additionnel au moins un substituant choisi parmi les substituants (d) définis ci-après :

substituants (d) :

groupes alkyle en $C_1$-$C_3$, groupes benzyle et groupes phényle.

31. Utilisation selon la revendication 29, dans laquelle ledit médicament cérébro-actif est représenté par la formule (Ib) :

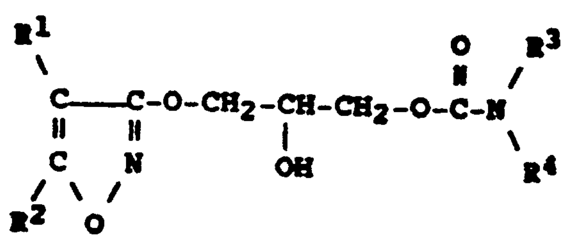

(Ib)

dans laquelle :

$R^1$ représente un atome d'hydrogène, un atome de chlore ou un groupe alkyle en $C_1$-$C_2$ ;
$R^2$ représente un groupe phényle ou un groupe phényle ayant au moins un des substituants (c') définis ci-après; ou
$R^1$ et $R^2$, ensemble avec les atomes de carbone auxquels ils sont fixés, représentent un cycle benzène condensé au cycle isoxazole et étant non substitués ou ayant un atome d'halogène ; et
substituant (c')
groupes méthoxy et atomes d'halogène ;
$R^3$ et $R^4$ sont semblables ou différents et représentent chacun un atome d'hydrogène, un groupe méthyle ou un groupe éthyle ; ou
$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

32. Utilisation selon la revendication 29, dans laquelle ledit médicament cérébro-actif est au moins un des composés suivants :
2-(2-hydroxy-3-morpholinopropyl)-4-méthyl-5-phényl-3-isoxazolone ;
3-(3-carbamoyloxy-2-hydroxypropoxy)-5-(m-chlorophényl)isoxazole ;
3-(2-hydroxy-3-morpholinopropoxy)-5-phénylisoxazole ;
3-(2-hydroxy-3-morpholinopropoxy)-4-méthyl-5-phénylisoxazole ;
3-(3-carbamoyloxy-2-hydroxypropoxy)-5-chloro-1,2-benzisoxazole ;
et leurs sels pharmaceutiquement acceptables.

33. Utilisation pour la préparation d'un médicament pour exercer une activité myorelaxante à activité centrale d'un composé actif dans laquelle le composé actif est un composé de formule (I) ou un sel pharmaceutiquement acceptable de celui-ci, selon la revendication 29.

34. Utilisation selon la revendication 33, dans laquelle ledit composé actif est représenté par la formule (Ib) :

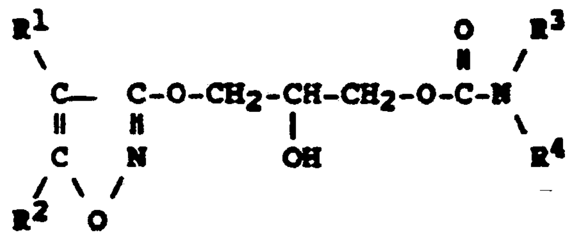
(Ib)

dans laquelle :
$R^1$ représente un atome d'hydrogène, un atome d'halogène ou un groupe alkyle en $C_1$-$C_4$ ;
$R^2$ représente un groupe phényle ou un groupe phényle ayant au moins un des substituants (c) définis ci-après:
substituants (c) :
groupes alcoxy en $C_1$-$C_3$, groupes hydroxy et atomes d'halogène ; ou
$R^1$ et $R^2$, ensemble avec les atomes de carbone auxquels ils sont fixés, représentent un cycle hydrocarboné à 6 chaînons condensé au cycle isoxazole et étant non substitué ou ayant au moins un atome d'halogène ; et
$R^3$ et $R^4$ sont semblables ou différents et représentent chacun un atome d'hydrogène, un groupe alkyle en $C_1$-$C_4$, un groupe benzyle ou un groupe phényle ; ou
$R^3$, $R^4$ et l'atome d'azote auquel ils sont fixés représentent ensemble un groupe amino alicyclique ayant au total 5 ou 6 atomes formant le cycle, dont un est ledit atome d'azote et 0 ou 1 est un hétéroatome d'azote additionnel, ledit groupe amino alicyclique étant non substitué ou, lorsqu'il y a un hétéroatome d'azote additionnel, ayant sur cet hétéroatome d'azote additionnel au moins un substituant choisi parmi les substituants (d) définis ci-après :
substituants (d) :
groupes alkyle en $C_1$-$C_3$, groupes benzyle et groupes phényle.

35. Utilisation selon la revendication 33, dans laquelle ledit composé actif est représenté par la formule (Ib) :

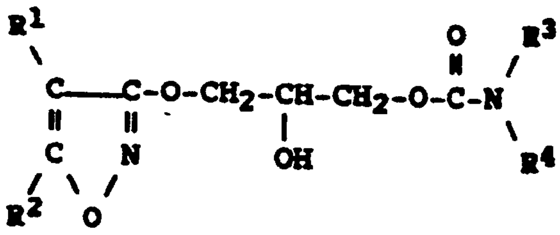

(Ib)

dans laquelle :

$R^1$ représente un atome d'hydrogène, un atome de chlore ou un groupe alkyle en $C_1$ ou $C_2$ ;

$R^2$ représente un groupe phényle ou un groupe phényle ayant au moins un des substituants (c') définis ci-après; ou

$R^1$ et $R^2$, ensemble avec les atomes de carbone auxquels ils sont fixés, représentent un cycle benzène condensé au cyxle isoxazole et étant non substitué ou ayant un atome d'halogène ; et

substituants (c') :

groupes méthoxy et atomes d'halogène ;

$R^3$ et $R^4$ sont semblables ou différents et représentent chacun un atome d'hydrogène, un groupe méthyle ou un groupe éthyle ; ou

$R^3$, $R^4$ et l'atome d'azote auquel ils sont fixés représentent ensemble un groupe morpholino, un groupe 1-pipérazinyle, un groupe 4-méthyl-1-pipérazinyle, un groupe 1-pyrrolidinyle ou un groupe pipéridino.

36. Utilisation selon la revendication 33, dans laquelle ledit composé actif est au moins un des composés suivants :

2-(2-hydroxy-3-morpholinopropyl)-4-méthyl-5-phényl-3-isoxazolone ;

3-(3-carbamoyloxy-2-hydroxypropoxy)-5-(m-chlorophényl)isoxazole ;

3-(2-hydroxy-3-morpholinopropoxy)-5-phénylisoxazole ;

3-(2-hydroxy-3-morpholinopropoxy)-4-méthyl-5-phénylisoxazole ;

3-(3-carbamoyloxy-2-hydroxypropoxy)-5-chloro-1,2-benzisoxazole ;

ou un de leurs sels pharmaceutiquement acceptables.